# NOUVEAU FASCICULE DE BREVET EUROPEEN

(11) **EP 1 506 225 B2**
(45) Date de publication et mention de la décision concernant l'opposition: **17.03.2010**
(45) Mention de la délivrance du brevet: 12.07.2006
(21) Numéro de dépôt: 03752815.5
(22) Date de dépôt: 15.05.2003
(51) Int. Cl.: C07K 14/18

(54) **NOUVELLES COMPOSITIONS PEPTIDIQUES ET LEUR UTILISATION NOTAMMENT DANS LA PREPARATION DE COMPOSITIONS PHARMACEUTIQUES ACTIVES CONTRE LE VIRUS DE L'HEPATITE C**
NEUE PEPTIDZUSAMMENSETZUNGEN UND DEREN VERWENDUNG, INSBESONDERE ZUR HERSTELLUNG VON PHARMAZEUTISCHEN ZUSAMMENSETZUNGEN MIT WIRKUNG GEGEN DAS HEPATITIS-C-VIRUS
NOVEL PEPTIDE COMPOSITIONS AND THE USE THEREOF, IN PARTICULAR, IN THE PREPARATION OF ACTIVE PHARMACEUTICAL COMPOSITIONS AGAINST THE HEPATITIS C VIRUS

(30) Priorité: 17.05.2002 FR 0206111
(43) Date de publication de la demande: 16.02.2005
(73) Titulaire: TRANSGENE S.A., 67000 Strasbourg (FR); INSTITUT NATIONAL DE LA SANTE ET DE LA RECHERCHE MEDICALE (INSERM), 75654 Paris Cédex 13 (FR)
(72) Inventeur: FOURNILLIER, Anne, F-69100 Villeurbanne (FR); HIMOUDI, Nourredine, F-69009 Lyon (FR); INCHAUSPE, Geneviève, F-69003 Lyon (FR)
(74) Mandataire: Texier, Christian
(86) Numéro de dépôt international: PCT/FR2003/001478
(87) Numéro de publication internationale: WO 2003/097677

(56) Documents cités:
- EP-A- 0 450 931
- EP-A- 0 468 527
- WO-A-01/21189
- WO-A-01/90197
- WO-A-01/92311
- WO-A-93/00365
- WO-A-99/58658
- WO-A-99/67285
- US-A- 5 736 321

## Description

La présente invention a pour objet de nouvelles compositions peptidiques et leur utilisation notamment dans la préparation de compositions pharmaceutiques actives contre le virus de l'hépatite C.

La présente invention a pour objet une composition peptidique utile notamment dans la vaccination prophylactique et thérapeutique dirigée contre le virus de l'hépatite C.

L'hépatite C est la cause principale des hépatites acquises par transfusion. L'hépatite C peut également être transmise par d'autres voies percutanées, par exemple par injection de drogues par voie intraveineuse. Le risque de contamination des professionnels de la santé n'est par ailleurs pas négligeable.

L'hépatite C se distingue des autres formes de maladies du foie associées à des virus, telles que les hépatites A, B ou D. Les infections par le virus de l'hépatite C (VHC ou HCV) sont majoritairement chroniques avec pour résultante des maladies du foie, telles que hépatite, cirrhose et carcinome dans un grand nombre de cas (5 à 20%).

Bien que le risque de transmission du virus par transfusion ait diminué du fait de la mise en place de tests de criblage dans les années 1990, la fréquence des hépatites C reste élevée. A titre d'exemple, une étude récente indique qu'il y aurait encore aujourd'hui 10 000 à 15 000 nouveaux cas d'infection par an en France (S. Deuffic et al., Hepatology 1999; 29: 1596-1601). Actuellement, environ 170 millions de personnes à travers le monde sont infectés de manière chronique par le VHC. Les populations à risque élevé sont principalement le personnel hospitalier et les utilisateurs de drogues intraveineuses, mais il existe des donneurs de sang asymptomatiques qui n'appartiennent pas à ces groupes à risque élevé et chez lesquels des anticorps anti-VHC circulants ont été retrouvés. Pour ces derniers, la voie de l'infection n'a encore pas été identifiée.

Le VHC a été le premier virus hépatotrope isolé au moyen des techniques de biologie moléculaire. Les séquences du génome viral ont été clonées avant que la particule virale n'ait été visualisée.

Le VHC appartient à un nouveau groupe de la famille des *Flaviviridae,* les hepacivirus. C'est un virus à ARN simple brin positif, de 9,5 kb, qui se réplique par une copie d'ARN complémentaire et dont le produit de la traduction est un précurseur d'une polyprotéine unique d'environ 3 000 acides aminés. L'extrémité 5' du génome du VHC correspond à une région non traduite adjacente aux gènes qui codent pour les protéines structurales, la protéine core de la nucléocapside, les deux glycoprotéines d'enveloppe, E1 et E2, et une petite protéine appelée p7. La région non traduite 5' et le gène core sont relativement bien conservés dans les différents génotypes. Les protéines d'enveloppe E1 et E2 sont codées par des régions plus variables d'un isolat à un autre. La protéine p7 est une protéine extrêmement hydrophobe dont la fonction n'est pas connue. L'extrémité 3' du génome du VHC contient les gènes qui codent pour les protéines non structurales (NS2, NS3, NS4, NS5) et pour une région 3' non codante possédant un domaine bien conservé (Major ME, Feinstone SM, Hepatology, juin 1997, 25(6): 1527-1538).

La thérapie pour le traitement de l'hépatite C vers laquelle on s'oriente actuellement est une bithérapie utilisant l'interféron pégylé et la ribavirine (Manns MP et al., The Lancet, 22 septembre 2001, Vol. 358, 958-965). Alors que cette thérapie est particulièrement efficace dans le cas des patients infectés par des souches virales appartenant aux génotypes 2 et 3, elle n'a qu'un effet limité sur les génotypes 1a, 1b et 4 (Manns MP, *supra*).

Il est donc nécessaire de mettre au point une composition vaccinale ciblant en priorité ces génotypes mauvais répondeurs.

Plusieurs études montrent aujourd'hui que le contrôle d'une infection due au VHC, soit naturellement (« résolution spontanée »), soit après traitement (« résolution thérapeutique ») est associé à l'induction ou la potentialisation de réponses immunes à médiation cellulaire faisant intervenir les lymphocytes T-CD4⁺ et T-CD8⁺ (CERNY A et al., J. Clin. Invest., 95 : 521-530 (1995)).

Les vaccins basés sur l'utilisation de peptides ont généralement pour but d'induire des réponses immunes médiées par les lymphocytes T-CD4⁺ et/ou T-CD8⁺.

Les molécules du complexe majeur d'histocompatibilité (CMH) sont dites de classe I ou de classe II. Les molécules de classe I sont exprimées sur la quasi totalité des cellules nucléées et peuvent être la cible de lymphocytes T cytotoxiques (CTL) CD8⁺. Les CTL reconnaissent les peptides ou épitopes qui sont présentés en association avec les molécules du CMH de classe I.

Par exemple, la molécule de classe I HLA-A2.1 indique que le site de liaison du peptide est créé par l'assemblage des domaines α₁ et α₂ de la chaîne lourde de classe I (Bjorkman et al., Nature, 329 : 506 (1987)).

Certains auteurs ont conclu au pouvoir immunogène de préparations peptidiques en se basant sur leurs bons scores de fixation sur des molécules HLA, comme dans la demande de brevet WO01/21189.

Une telle déduction n'est pas évidente et peut conduire :
- soit à la sélection de peptides ne présentant pas de pouvoir immunogène, bien qu'ayant un score de fixation élevé, comme la Demanderesse l'a démontré dans l'exemple 1 de la présente demande avec le peptide FLAT,
- soit à l'élimination de peptides qui sont en fait bien immunogéniques, comme montré avec le peptide CIN dans Brinster C. et al. (Hepatology, Vol.34, N°6, 2001, 1206-1217). En effet, bien que le peptide CIN ait un score de fixation moyen, voire faible (335), il est néanmoins capable d'induire une réponse forte médiée par des lymphocytes T cytotoxiques.

La Demanderesse a maintenant trouvé de façon inattendue qu'une nouvelle composition peptidique contenant au moins deux peptides choisis parmi les peptides A à D présentail un fort pouvoir immunogène et avait un effet sur la capacité des cellules provenant de patients infectés par des souches virales de génotype 1 a, 1 b et 4 à induire des réponses immunitaires spécifiques. Ces patients présentent de façon préférée, mais non limitative, un HLA de type HLA-A2.J

Ainsi, la présente invention a pour objet les compositions peptidiques comprenant au moins deux composés choisis parmi :
- un peptide A ayant au moins la séquence en acides aminés SEQ ID N°1 suivante :
   X₁AX₂QGYKVX₃VLNPSVX₄ATLX₅FGX₆YMSKAX₇GX₈,
   dans laquelle X₁ est Y ou H, X₂ est A, G ou T, X₃ est R ou L, X₄ est A ou T, X₅ est G ou S, X₆ est A, T ou V, X₇ est H ou Y et X₈ est I, T, M ou V, et ayant au plus les 46 acides aminés tels que décrits dans la séquence SEQ ID N°2 suivante :
   SX₉X₁₀VPX₁₁X₁₂X₁AX₂QGYKVX₃VLNPSVX₄ATLX₅FGX₆YMSKAX₇GX₈X₁₃PX₁₄ X₁₅X₁₆X₁₇GV,
   dans laquelle X₁ à X₈ sont tels que définis ci-dessus, et X₉ est T ou N, X₁₀ est K ou R, X₁₁ est A ou V, X₁₂ est A ou E, X₁₃ est E ou D, X₁₄ est N ou S, X₁₅ est I, L ou V, X₁₆ est R ou S et X₁₇ est T ou S,
- un peptide B ayant au moins la séquence en acide aminé SEQ ID N°45 suivante :
   GX₁₈X₁₉X₂₀X₂₁X₂₂X₂₃TSLTGRDX₂₄NX₂₅X₂₆X₂₇GEX₂₈QX₂₉X₃₀STAX₃₁X₃₂X₃₃FLX₃₄ X₃₅X₃₆X₃₇NGX₃₈X₃₉WTVX₄₀
   dans laquelle X₁₈ est L ou V, X₁₉ est L ou F, X₂₀ est G ou S, X₂₁ est C ou T, X₂₂ est I ou V, X₂₃ est I ou V, X₂₄ est K, R ou T, X₂₅ est Q ou E, X₂₆ est V ou N, X₂₇ est D, E ou C, X₂₈ est V ou A, X₂₉ est V, I, F, ou M, X₃₀ est L, ou V, X₃₁ est T, K ou A, X₃₂ est Q ou H, X₃₃ est S ou T, X₃₄ est A ou G, X₃₅ est T ou S, X₃₆ est C ou A, X₃₇ est V, I ou T, X₃₈ est V ou A, X₃₉ est C ou M et X₄₀ est Y ou F, et ayant au plus les 63 acides aminés tels que décrits dans la séquence SEQ ID N°46 suivante :
   AX₄₁ITX₄₂YX₄₃X₄₄QTRGX₁₈RX₁₉X₂₀X₂₁X₂₂X₂₃TSLTGRDX₂₄NX₂₅X₂₆X₂₇GEX₂₈QX₂₉ X₃₀STAX₃₁X₃₂X₃₃FLX₃₄X₃₅X₃₆X₃₇GX₃₈X₃₉WTVX₄₀HGAGX₄₅X₄₆X₄₇
   dans laquelle X₁₈ à X₄₀ sont tels que définis dans la revendication ci-dessus et X₄₁ est P, S ou H, X₄₂ est A ou T, X₄₃ est S. A, T ou C, X₄₄ est Q ou R, X₄₅ est S, T ou A, X₄₆ est K ou R et X₄₇ est T ou I,
- un peptide C ayant au moins la séquence en acides aminés SEQ ID N°127 suivante :
   SX₄₇M X₄₈FTX₄₉X₅₀X₅₁TSPLX₅₂X₅₃X₅₅TLX₅₆FNIX₅₇GGWAX₅₈QX₅₉
   dans laquelle X₄₇ est L ou P, X₄₈ est A ou S, X₄₉ est A ou S, X₅₀ est A ou S, X₅₁ est I ou V, X₅₂ est T, S ou A, X₅₃ est T ou I, X₅₄ est Q, S ou G, X₅₅ est N, Q, H, S, Y ou T, X₅₆ est L ou M,X₅₇ est L ou W, X₅₈ est A ou S et X₅₉ est L, P ou I, et ayant au plus les 57 acides aminés tels que décrits dans la séquence SEQ ID N°128 suivante :
   NFIX₆₀GX₆₁QYLAX₆₂LSTLPGNX₆₃AX₆₄X₆₅SX₄₇MX₄₈FTX₄₉X₅₀X₅₁TSPLX₅₂X₅₃X₅₄ X₅₅TLX₅₆FNIX₅₇GGWVAX₅₈QX₅₉X₆₆X₆₇X₆₈
   dans laquelle X₄₇ à X₅₉ sont tels que définis ci-dessus et X₆₀ est T ou S, X₆₁ est I, T ou V, X₆₂ est G ou A, X₆₃ est P ou L, X₆₄ est I ou M, X₆₅ est A, V ou R, X₆₆ est A ou R, X₆₇ est P, A ou D et X₆₈ est P, A ou S,
- un peptide D ayant au moins la séquence en acides aminés SEQ ID N°174 suivante :
   X₆₉KX₇₀ARX₇₁IVX₇₂PX₇₃LGX₇₄RVCEKX₇₅ALX₇₆X₇₇VX₇₈X₇₉X₈₀X₈₁
   dans laquelle X₆₉ est R ou Q, X₇₀ est P ou A, X₇₁ est L ou F, X₇₂ est F ou Y, X₇₃ est D
   ou E, X₇₄ est V ou S, X₇₅ est M ou R, X₇₆ est Y ou H, X₇₇ est D ou N, X₇₈ est V ou I, X₇₉ est S, T ou K, X₈₀ est T, K, I ou N et X₈₁ est L ou T, et ayant au plus les 44 acides aminés tels que décrits dans la séquence SEQ ID N°175 suivante :
   KGGX₆₉KX₇₀ARX₇₁IVX₇₂PX₇₃LGX₇₄RVCEKX₇₅ALX₇₆X₇₇VX₇₈X₇₉X₈₀X₈₃X₈₂X₈₃X₈₄ VMGX₈₅X₈₆YX₈₇X₈₈Q
   dans laquelle X₆₉ à X₈₁ sont tels que définis ci-dessus et X₈₂ est P ou A, X₈₃ est Q, L. H, R, K ou P, X₈₄ est A, T, V ou P, X₈₅ est P, S ou A, X₈₆ est S ou A, X₈₇ est G ou R et X₈₈ est F ou C,
- un épitope B' ayant la séquence SEQ ID N°213 suivante :
   GX₁₈X₁₉X₂₀X₂₁X₂₂X₂₃TSL
   dans laquelle X₁₈ est L ou V, X₁₉ est L ou F, X₂₀ est G ou S, X₂₁ est C ou T, X₂₂ est J ou V et X₂₃ est I ou V, et
- un épitope C' ayant la séquence SEQ ID N°221 suivante :
   SPLX₅₂X₅₃X₅₄X₅₅TL.
   dans laquelle X₅₂ est T, S ou A, X₅₃ est T ou I, X₅₄ est Q, S ou G et X₅₅ est N, Q, H, S, Y ou T.
ainsi que les compositions pharmaceutiques les contenant et leur utilisation, notamment en tant que vaccin, pour la préparation d'un médicament destiné à l'inhibition ou la prévention d'une infection provoquée par le virus de l'hépatite C, et en tant que composition diagnostique.

L'invention a notamment pour objet une composition peptidique telle que définie ci-dessus, caractérisée en ce qu'elle comprend au moins deux peptides choisis parmi les peptides A, B, C, D.

Elle a également pour objet les peptides particuliers A, B. C, D, les séquences nucléotidiques codant pour lesdits peptides et les microorganismes ou cellules hôtes cotransformés par ces vecteurs.

Elle a enfin pour objet les anticorps dirigés contre les compositions de l'invention et un procédé de détection etlou de quantification du virus de l'hépatite C dans un échantillon biologique en utilisant lesdits anticorps.

Les compositions peptidiques de l'invention, présentant un fort pouvoir immunogène contre les génotypes 1 a, 1b et 4 du virus de l'hépatitc C, contiennent donc
au moins deux peptides choisis parmi les peptides A à D tels que définis ci-dessus.

Le peptide A ayant la séquence en acides aminés SEQ ID NO:1 est inclus dans la protéine non structurale 3 (NS3) entre les positions 1244 et 1274 de la pnlyprotéine codée par le virus VHC.

Le peptide B ayant la séquence en acides aminés SEQ ID NO:45 est également inclus dans la protéine NS3 entre les positions 1038 et 1082 de la polyprotéine virale.

Le peptide C ayant la séquence en acide aminés SEQ ID NO: 127 quant à lui, est inclus dans la protéine non structurale NS4 entre les positions 1789 et 1821 de ladite polyprotéine virale.

S'agissant du peptide D ayant la séquence en acide aminés SEQ ID NO:174, il est inclus dans la protéine non structurale NS5b entre les positions 2573 et 2601 de ladite polyprotéine virale.

Bien entendu, on entend par peptide, le peptide en tant que tel, ainsi que ses homologues ayant au moins 60%, de préférence au moins 70%, de préférence encore au moins 80%, mieux au moins 90% et mieux encore 95% d'homologie avec le peptide d'intérêt.

Les peptides A à D ont été obtenus à partir de la souche HCV-JA (Kato L., et al., (1990), Proc. Natl. Acad. Sci. USA, 87(24). 9524-9528) autrement appelée souche Shimotono. Ils contiennent des épitopes connus mais, comme indiqué précédemment, ces épitopes n'ont pas forcément un score de fixation élevé.

Le score de fixation est obtenu par prédiction, à l'aide de logiciels, de la capacité de séquences épitopiques connues ou potentielles (séquences peptidiques) à se fixer sur la molécule HLA d'intérêt. Ce score peut être compris dans une gamme allant de valeurs négatives à la valeur d'environ 1000 et on entend par score de fixation élevé, un score de fixation supérieur ou égal à 600.

En revanche, de façon inattendue, l'association des peptides A à D de l'invention permet d'induire des lymphocytes T cytotoxiques spécifiques capables d'une vigueur et d'une efficacité supérieure à celle obtenue avec chacun des peptides utilisés séparément et/ou chacun des épitopes individuels contenus dans ces peptides utilisés seuls ou en association ainsi que de stimuler une production supérieure d'interferon γ.

De plus, ces peptides sont capables d'induire des lymphocytes T spécifiques ayant une réactivité croisée.

Selon un mode de réalisation de l'invention, les compositions de l'invention contiennent deux peptides selon les combinaisons suivantes : peptides A et B, peptides A et C, peptides A et D, peptides B et C, peptides B et D et peptides C et D.

Les compositions préférées contiennent les peptides A et B, A et C, B et D, et C et D, les compositions contenant les peptides A et B, C et D, et B et D étant davantage préférées.

Selon un autre mode de réalisation, les compositions de l'invention contiennent trois peptides selon les combinaisons suivantes : peptides A, B et D, peptides A, C et D, et peptides B, C et D, le.s compositions comprenant les peptides A, B et C, A, C et D, et B, C et 1) étant particulièrement préférées.

Selon encore un autre mode de réalisation, les compositions de l'invention contiennent les quatre peptides A, B, C, D.

Le peptide A est situé entre les positions 1237 et 1282 de la polyprotéine virale.

Selon encore un autre mode de réalisation, le peptide A est choisi parmi les peptides suivants :
- les peptides ayant au moins la séquence SEQ ID N°3, qui correspond à la séquence SEQ ID N°1 dans laquelle X₁ est Y, X₂ est A, X₃ est R, X₄ est A, X₅ est G, X₆ est A, X₇ est H et X₈ est J, et. au plus la séquence SRQ ID N°19 qui correspond à la séquence SEQ ID N°2 dans laquelle X₁ à X₈ sont tels que définis pour la séquence SEQ ID N°3 et X₉ est T, X₁₀ est K, X₁₁ est A, X₁₂ est A,X₁₃ est E, X₁₄ est N, X₁₅ est I, X₁₆ est R et X₁₇ est T,
- les peptides ayant au moins la séquence SEQ ID N°4 qui correspond à la séquence SEQ ID N°1 dans laquelle X₁ est Y, X₂ est A, X₃ est L, X₄ est A, X₅ est G, X₆ est A, X₇ est H et X₈ est I, et au plus une séquence choisie parmi les séquences suivantes :
   i) la séquence SEQ ID N°20 qui correspond à la séquence SEQ ID N°2 dans laquelle X₁ à X₈ sont tels que définis pour la séquence SEQ ID N°4 et X₉ est T, X₁₀ est K, X₁₁ est A, X₁₂ est A, X₁₃ est D, X₁₄ est N, X₁₅ est I, X₁₆ est R et X₁₇ est T,
   ii) la séquence SEQ ID N°24 qui correspond à la séquence SEQ ID N°2 dans laquelle X₁ à X₈ sont tels que définis pour la séquence SEQ ID N°4 et X₉ est T, X₁₀ est K, X₁₁ est A, X₁₂ est A, X₁₃ est F, X₁₄ est N, X₁₅ est 1, X₁₆ est R et X₁₇ est T,
   iii) la séquence SEQ ID N°32 qui correspond à la séquence SEQ ID N°2 dans laquelle X₁ à X₈ sont tels que définis pour la séquence SEQ ID N°4 et X₉ est T, X₁₀ est R, X₁₁ est A, X₁₂ est A, X₁₃ est D, X₁₄ est N, X₁₅ est L, X₁₆ est R et X₁₇ est T, et
   iv) la séquence SEQ ID N°34 qui correspond à la séquence SEQ ID N°2 dans laquelle X₁ à X₈ sont tels que définis pour la séquence SEQ ID N°4 et X₉ est T, X₁₀ est K, X₁₁ est A, X₁₂ est A, X₁₃ est D, X₁₄ est N, X₁₅ est V, X₁₆ est R et X₁₇ est T,
- les peptides ayant au moins la séquence SEQ ID N°5 qui correspond à la séquence SEQ ID N°1 dans laquelle X₁ est Y, X₂ est A, X₃ est L, X₄ est A, X₅ est G, X₄ est A, X₇ est H et X₈ est V, et au plus une séquence choisie parmi les séquences suivantes :
   i) la séquence SEQ ID N°21 qui correspond à la séquence SEQ ID N°2 dans laquelle X₁ à X₈ sont tels que définis pour la séquence SEQ ID N°5 et X₉ est T, X₁₀ est K, X₁₁ est A, X₁₂ est A, X₁₃ est D, X₁₄ est N, X₁₅ est I, X₁₆ est R et X₁₇ est T,
   ii) la séquence SEQ ID N°28 qui correspond à la séquence SEQ 10 N°2 dans laquelle X₁ à X₈ sont tels que définis pour la séquence SEQ ID N°5 et X₉ est N, X₁₀ est K, X₁₁ est V, X₁₂ est E, X₁₃ est D, X₁₄ est N, X₁₅ est I, X₁₆ est R et X₁₇ est T, et
   iii) la séquence SEQ ID N°36 qui correspond à la séquence SEQ ID N°2 dans laquelle X₁ à X₈ sont tels que définis pour la séquence SEQ ID N°5 et X₉ est T, X₁₀ est K, X₁₁ est A, X₁₂ est A, X₁₃ est D, X₁₄ est N, X₁₅ est J, X₁₆ est S et X₁₇ est T,
- les peptides ayant au moins la séquence SEQ ID N°6 qui correspond à la séquence SEQ ID N°1 dans laquelle X₁ est Y, X₂ est A, X₃ est L, X₁ est A, X₅ est G, X₆ est A, X₇ est H et X₈ est T, et au plus une séquence choisie parmi les séquences suivantes :
   i) la séquence SEQ ID N°22 qui correspond à la séquence SEQ ID N°2 dans laquelle X₁ à X₈ sont tels que définis pour la séquence SEQ ID N°6 et X₉ est T, X₁₀ est K, X₁₁ est A, X₁₂ est A, X₁₃ est D, X₁₄ est N, X₁₅ est I, X₁₆ est R et X₁₇ est T,
   ii) la séquence SEQ ID N°27 qui correspond à la séquence SEQ ID N°2 dans laquelle X₁ à X₈ sont tels que définis pour la séquence SEQ ID N°6 et X₉ est T, X₁₀ est K, X₁₁ est A, X₁₂ est A, X₁₃ est L, X₁₄ est N, X₁₅ est I, X₁₆ est R et X₁₇ est T, et
   iii) la séquence SEQ ID N°41 qui correspond à la séquence SEQ ID N°2 dans laquelle X₁ à X₈ sont tels que définis pour la séquence SEQ ID N°6 et X₉ est T, X₁₀ est R, X₁₁ est A, X₁₂ est A, X₁₃ est D, X₁₄ est N, X₁₅ est I, X₁₆ est R et X₁₇ est T,
- les peptides ayant au moins la séquence SEQ ID N°7 qui correspond à la séquence SEQ ID N°1 dans laquelle X₁ est Y, X₂ est A, X₃ est L, X₄ est A, X₅ est G, X₆ est A, X₇ est Y et X₈ est T, et au plus une séquence choisie parmi les séquences suivantes :
   i) la séquence SEQ ID N°23 qui correspond à la séquence SEQ ID N°2 dans laquelle X₁ à X₈ sont tels que définis pour la séquence SEQ ID N°7 et X₉ est T, X₁₀ est K, X₁₁ est A, X₁₂ est A, X₁₃ est D, X₁₄ est N, X₁₅ est I, X₁₆ est R et X₁₇ est T, et
   ii) la séquence SEQ ID N°37 qui correspond à la séquence SEQ ID N°2 dans laquelle X₁ à X₈ sont tels que définis pour la séquence SEQ ID N°7 et X₉ est T, X₁₀ est K, X₁₁ est A, X₁₂ est A, X₁₃ est D, X₁₄ est N, X₁₅ est V, X₁₆ est R et X₁₇ est T,
- les peptides ayant au moins la séquence SEQ ID N°8 qui correspond à la séquence SEQ ID N°1 dans laquelle X₁ est Y, X₂ est A, X₃ est L, X₄ est A, X₅ est S, X₆ est A, X₇ est H et X₈ est T, et au plus la séquence SEQ ID N°25 qui correspond à la séquence SEQ ID N°2 dans laquelle X₁ à X₈ sont tels que définis pour la séquence SEQ ID N°8 et X₉ est T, X₁₀ est K, X₁₁ est A, X₁₂ est A, X₁₃ est D, X₁₄ est N, X₁₅ est I, X₁₆ est R et X₁₇ est T,
- les peptides ayant au moins la séquence SEQ ID N°9 qui correspond à la séquence SEQ ID N°1 dans laquelle X₁ est Y, X₂ est A, X₃ est L, X₄ est A, X₅ est G, X₆ est A, X₇ est Y et X₈ est I, et au plus la séquence SEQ ID N°26 qui correspond à la séquence SEQ ID N°2 dans laquelle X₁ à X₈ sont tels que définis pour la séquence SEQ ID N°9 et X₉ est T, X₁₀ est K, X₁₁ est A, X₁₂ est A, X₁₃ est D, X₁₄ est N, X₁₅ est V, X₁₆ est R et X₁₇ est T,
- les peptides ayant au moins la séquence SEQ ID N°10 qui correspond à la séquence SEQ ID N°1 dans laquelle X₁ est Y, X₂ est A, X₃ est L, X₄ est T, X₅ est G, X₆ est A, X₇ est H et X₈ est V, et au plus la séquence SEQ ID N°29 qui correspond à la séquence SEQ ID N°2 dans laquelle X₁ à X₈ sont tels que définis pour la séquence SEQ ID N°10 et X₉ est T, X₁₀ est K, X₁₁ est A, X₁₂ est A, X₁₃ est D, X₁₄ est N, X₁₅ est I, X₁₆ est R et X₁₇ est T,
- les peptides ayant au moins la séquence SEQ ID N°11 qui correspond à la séquence SEQ ID N°1 dans laquelle X₁ est Y, X₂ est G, X₃ est L, X₄ est A, X₅ est G, X₆ est A, X₇ est H et X₈ est I, et au plus la séquence SEQ ID N°30 qui correspond à la séquence SEQ ID N°2 dans laquelle X₁ à X₈ sont tels que définis pour la séquence SEQ ID N°11 et X₉ est T, X₁₀ est K, X₁₁ est A, X₁₂ est A, X₁₃ est D, X₁₄ est N, X₁₅ est I, X₁₆ est R et X₁₇ est T,
- les peptides ayant au moins la séquence SEQ ID N°12 qui correspond à la séquence SEQ ID N°1 dans laquelle X₁ est Y, X₂ est A, X₃ est L, X₄ est A, X₅ est S. X₆ est A, X₇ est H et X₈ est I, et au plus la séquence SEQ ID N°31 qui correspond à la séquence SEQ ID N°2 dans laquelle X₁ à X₈ sont tels que définis pour la séquence SEQ ID N°12 et X₉ est T, X₁₀ est K, X₁₁ est A, X₁₂ est A, X₁₃ est D, X₁₄ est N, X₁₅ est 1, X₁₆ est R et X₁₇ est T,
- les peptides ayant au moins la séquence SEQ ID N°13 qui correspond à la séquence SEQ ID N°1 dans laquelle X₁ est Y, X₂ est T, X₃ est L, X₄ est A, X₅ est G, X₆ est A, X₇ est H et X₈ est T, et au plus la séquence SEQ ID N°33 qui correspond à la séquence SEQ ID N°2 dans laquelle X₁ à X₈ sont tels que définis pour la séquence SEQ ID N°13 et X₉ est T, X₁₀ est K, X₁₁ est A, X₁₂ est A, X₁₃ est D, X₁₄ est N, X₁₅ est I, X₁₆ est R et X₁₇ est T,
- les peptides ayant au moins la séquence SEQ ID N°14 qui correspond à la séquence SEQ ID N°1 dans laquelle X₁ est Y, X₂ est A, X₃ est L, X₄ est A, X₅ est S, X₆ est A, X₇ est H et X₈ est V, et au plus une séquence choisie parmi les séquences suivantes :
   i) la séquence SEQ ID N°35 qui correspond à la séquence SEQ ID N°2 dans laquelle X₁ à X₈ sont tels que définis pour la séquence SEQ ID N°14 et X₉ est T, X₁₀ est K, X₁₁ est A, X₁₂ est A, X₁₃ est D, X₁₄ est S, X₁₅ est I, X₁₆ est R et X₁₇ est T, et
   ii) la séquence SEQ ID N°39 qui correspond à la séquence SEQ ID N°2 dans laquelle X₁ à X₈ sont tels que définis pour la séquence SEQ ID N°14 et X₉ est T, X₁₀ est K, X₁₁ est A, X₁₂ est A, X₁₃ est D, X₁₄ est N, X₁₅ est 1, X₁₆ est R et X₁₇ est T,
- les peptides ayant au moins la séquence SEQ ID N°15 qui correspond à la séquence SEQ ID N°1 dans laquelle X₁ est Y, X₂ est T, X₃ est L, X₄ est A, X₅ est S, X₆ est A, X₇ est Y et X₈ est M, et au plus la séquence SEQ ID N°38 qui correspond à la séquence SEQ ID N°2 dans laquelle X₁ à X₈ sont tels que définis pour la séquence SEQ ID N°15 et X₉ est T, X₁₀ est K, X₁₁, est A, X₁₂ est A, X₁₃ est D, X₁₄ est N, X₁₅ est L, X₁₆ esl R et X₁₇ est T,
- les peptides ayant au moins la séquence SEQ ID N°16 qui correspond à la séquence SEQ ID N°1 dans laquelle X₁ est Y, X₂ est A, X₃ est L, X₄ est A, X₅ est S, X₆ est A, X₇ est Y et X₈ est V, et au plus une séquence choisie parmi les séquences suivantes :
   i) la séquence SEQ ID N°40 qui correspond à la séquence SEQ ID N°2 dans laquelle X₁ à X₈ sont tels que définis pour la séquence SEQ ID N°16 et X₉ est T, X₁₀ est K, X₁₁ est A, X₁₂ est A, X₁₃ est D, X₁₄ est N, X₁₅ est I, X₁₆ est R et X₁₇ est T, et
   ii) la séquence SEQ ID N°42 qui correspond à la séquence SEQ ID N°2 dans laquelle X₁ à X₈ sont tels que définis pour la séquence SEQ ID N°16 et X₉ est T, X₁₀ est R, X₁₁ est A, X₁₂ est A, X₁₃ est D, X₁₄ est N, X₁₅ est I, X₁₆ est R et X₁₇ est T,
- les peptides ayant au moins la séquence SEQ ID N°17 qui correspond à la séquence SEQ ID N°1 dans laquelle X₁ est Y, X₂ est A, X₃ est L, X₄ est A, X₅ est G, X₆ est T, X₇ est Y et X₈ est T, et au plus la séquence SEQ ID N°43 qui correspond à la séquence SEQ ID N°2 dans laquelle X₁ à X₈ sont tels que définis pour la séquence SEQ ID N°17 et X₉ est T, X₁₀ est R, X₁₁ est A, X₁₂ est A, X₁₃ est D, X₁₄ est N, X₁₅ est J, X₁₆ est R et X₁₇ est T, et
- les peptides ayant au moins la séquence SEQ ID N°18 qui correspond à la séquence SEQ ID N°1 dans laquelle X₁ est H, X₂ est A, X₃ est L, X₄ est A, X₅ est G, X₆ est V, X₇ est Y et X₈ est J, et au plus la séquence SEQ ID N°44 qui correspond à la séquence SEQ ID N°2 dans laquelle X₁ à X₈ sont tels que définis pour la séquence SEQ ID N°18 et X₉ est T, X₁₀ est K, X₁₁ est A, X₁₂ est A, X₁₃ est D, X₁₄ est N, X₁₅ est I, X₁₆ est R et X₁₇ est S.

De préférence, le peptide A est choisi parmi les peptides de séquences SEQ ID N°3 à 18, le peptide de séquence SEQ ID N°3 étant particulièrement préféré.

Le peptide B est situé entre les positions 1027 et 1089 de la polyprotéine virale.

Selon encore un autre mode de réalisation le peptide B est choisi parmi les peptides suivants :
- les peptides ayant au moins la séquence SEQ ID N°47 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est D, X₂₈ est V, X₂₉ est V, X₃₀ est L, X₃, est T, X₃₂ est Q, X₃₃ est S, X₃₄ est A, X₃₅ est T, X₃₆ est C, X₃₇ est V, X₃₈ est V, X₃₉ est C et X₄₀ est Y, et au plus la séquence SEQ 1D N°81 qui correspond à la séquence SEQ ID N°46 dans laquelle X₁₈ à X₄₀ sont tels que définis pour la séquence SEQ ID N°47 et X₄₁ est P, X₄₂ est X₄₃ est S, X₄₄ est Q, X₁₅ est S, X₄₆ est K et X₄₇ est T,
- les peptides ayant au moins la séquence SEQ ID N°48 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est I, X₃₀ est V, X₃₁ est A, X₃₂ est Q, X₃₃ est T, X₃₄ est A, X₃₅ est T, X₃₆ est C, X₃₇ est I, X₃₈ est V, X₃₉ est C et X₄₀ est Y, et au plus la séquence SEQ ID N°82 qui correspond à la séquence SEQ ID N°46 dans laquelle X₁₈ àX₄₀ sont tels que définis pour la séquence SEQ ID N°48 et X₄₁ est P, X₄₂ est A, X₄₃ est A, X₄₄ est Q, X₄₅ est T, X₄₆ est R et X₄₇ est T,
- les peptides ayant au moins la séquence SEQ ID N°49 qui correspond à la séquence SEQ ID N°45 dans laquelle X₁₈ est L, X₁₉ est L, X₂₀ est G, X₂₁ est C, X₂₂ est T, X₂₃ est I, X₂₄ est K, X₂₅ est Q, X₂₆ est V, X₂₇ est E. X₂₈ est V, X₂₉ est I, X₃₀ est V, X₃₁ est T, X₃₂ est Q, X₃₃ est T, X₃₄ est A, X₃₅ est T, X₃₆ est C, X₃₇ est I, X₃₈ est V, X₃₉ est C et X₁₀ est Y, et au plus la séquence SEQ ID N°83 qui correspond à la séquence SEQ ID N°46 dans laquelle X₁₈ à X₄₀ sont tels que définis pour la séquence SEQ ID N°49 et X₄₁ est P, X₄₂ est A, X₄₃ est T, X₄₄ est Q, X₄₅ est T, X₄₆ est R et X₄₇ est T,
- les peptides ayant au moins la séquence SEQ ID N°50 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆, est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est Y, et au plus une séquence choisie parmi les séquences suivantes:
   i) la séquence SEQ ID N°84 qui correspond à la séquence SEQ ID N°46 dans laquelle X₁₈ à X₄₀ sont tels que définis pour la séquence SEQ ID N°50 et X₄₁ est P, X₄₂ est A, X₄₃ est S, X₄₄ est Q, X₄₅ est S, X₄₆ est K et X₄₇ est T,
   ii) la séquence SEQ ID N°90 qui correspond à la séquence SEQ ID N°46 dans laquelle X₁₈ à X₄₀ sont tels que définis pour la séquence SEQ ID N°50 et X₄₁ est P, X₄₂ est A, X₄₃ est S, X₄₄ est R, X₄₅ est S, X₄₆ est K et X₄₇ est T,
   iii) la séquence SEQ ID N°105 qui correspond à la séquence SEQ ID N°46 dans laquelle X₁₈ à X₄₀ sont tels que définis pour la séquence SEQ ID N°50 et X₄₁ est P, X₄₂ est A, X₄₃ est C, X₄₄ est Q, X₄₅ est S, X₄₆ est K et X₄₇ est T,
   iv) la séquence SEQ ID N°107 qui correspond à la séquence SEQ ID N°46 dans laquelle X₁₈ à X₄₀ sont tels que définis pour la séquence SEQ ID N°50 et X₄₁ est P, X₄₂ est A, X₄₃ est S, X₄₄ est Q, X₄₅ est T, X₄₆ est K et X₄₇ est T, et
   v) la séquence SEQ ID N°116 qui correspond à la séquence SEQ ID N°46 dans laquelle X₁₈ à X₄₀ sont tels que définis pour la séquence SEQ ID N°50 et X₄₁ est P, X₄₂ est T, X₄₃ est S, X₄₄ est Q, X₄₅ est T, X₄₆ est K et X₄₇ est T,
- les peptides ayant au moins la séquence SEQ ID N°51 qui correspond à la séquence SEQ ID N°45 dans laquelle X₁₈ est L, X₁₉ est L, X₂₀ est G, X₂₁ est G, X₂₂ est I, X₂₃ est I, X₂₄ est R, X₂₅ est Q, X₂₆ est V, X₂₇ est E, X₂₈ est V, X₂₉ est V, X₃₀ est V, X₃₁ est.T, X₃₂ est Q, X₃₃ est S, X₃₄ est A, X₃₅ est T, X₃₆ est C, X₃₇ est V, X₃₈ est V, X₃₉ est C et X₄₀ est Y, et au plus une séquence choisie parmi les séquences suivantes:
   i) la séquence SEQ ID N°85 qui correspond à la séquence SEQ ID N°46 dans laquelle X₁₈ à X₄₀ sont tels que définis pour la séquence SEQ ID N°51 et X₄₁ est P, X₄₂ est A, X₄₃ est S, X₄₄ est Q, X₄₅ est S, X₄₆ est K et X₄₇, est T, et
   ii) la séquence SEQ ID N°124 qui correspond à la séquence SEQ ID N°46 dans laquelle X₁₈ à X₄₀ sont tels que définis pour la séquence SEQ ID N°51 et X₄₁ est P, X₄₂ est A. X₄₃ est S, X₄₄ est Q, X₄₅ est A, X₄₆ est K et X₄₇ est T,
- les peptides ayant au moins la séquence SEQ ID N°52 qui correspond à la séquence SEQ ID N°45 dans laquelle X₁₈ est L, X₁₉ est L, X₂₀ est G, X₂₁ est C, X₂₂ est 1, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I, X₃₈ est V, X₃₉ est C et X₄₀ est Y, et au plus une séquence choisie parmi les séquences suivantes:
   i) la séquence SEQ ID N°86 qui correspond à la séquence SEQ ID N°46 dans laquelle X₁₈ à X₄₀ sont tels que définis pour la séquence SEQ ID N°52 et X₄₁ est P, X₄₂ est A, X₄₃ est S, X₄₄ est Q, X₄₅ est S, X₄₆ est K et X₄₇ est T, et
   ii) la séquence SEQ ID N°120 qui correspond à la séquence SEQ ID N°46 dans laquelle X₁₈ à X₄₀ sont tels que définis pour la séquence SEQ ID N°52 et X₄₁ est P, X₄₂ est A, X₄₃ est A. X₄₄ est Q, X₄₅ est S, X₄₆ est K et X₄₇ est T,
- les peptides ayant au moins la séquence SEQ ID N°53 qui correspond à la séquence SEQ ID N°45 dans laquelle X₁₈ est L, X₁₉ est F, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est Y, et au plus la séquence SEQ ID N°87 qui correspond à la séquence SEQ ID N°46 dans laquelle X₁₈ à X₄₀ sont tels que définis pour la séquence SEQ ID N°53 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°54 qui correspond à la séquence SEQ ID N°45 dans laquelle X₁₈ est V, X₁₉ est L, X₂₀ est G, X₂₁ est C, X₂₂ est V, X₂₃ est I. X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I; X₃₈ est V, X₃₉ est C et X₄₀ est Y, et au plus une séquence choisie parmi les séquences suivantes :
   i) la séquence SEQ ID N°88 qui correspond à la séquence SEQ ID N°46 dans laquelle X₁₈ à X₄₀ sont tels que définis pour la séquence SEQ ID N°54 et X₄₁ est P, X₄₂ est A, X₄₃ est S, X₄₄ est Q, X₄₅ est S, X₄₆ est K et X₄₇ est T, et
   ii) la séquence SEQ ID N°117 qui correspond à la séquence SEQ ID N°46 dans laquelle X₁₈ à X₄₀ sont tels que définis pour la séquence SEQ ID N°54 et X₄₁ est S, X₄₂ est A, X₄₃ est S, X₄₄ est Q, X₄₅ est S, X₄₆ est K et X₄₇ est T,
- les peptides ayant au moins la séquence SEQ ID N°55 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est A, X₃₉ est C et X₄₀ est F, et au plus la séquence SEQ ID N°89 qui correspond à la séquence SEQ ID N°46 dans laquelle X₁₈ à X₄₀ sont tels que définis pour la séquence SEQ ID N°55 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°56 qui correspond à la séquence SEQ ID N°45 dans laquelle X₁₈ est L, X₁₉ est L, X₂₀ est G, X₂₁ est C, X₂₂ est J, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I, X₃₈ est V, X₃₉ est C et X₄₀ est F, et au plus une séquence choisie parmi les séquences suivantes :
   i) la séquence SEQ ID N°91 qui correspond à la séquence SEQ ID N°46 dans laquelle X₁₈ à X₄₀ sont tels que définis pour la séquence SEQ ID N°56 et X₄₁ est P, X₄₂ est A, X₄₃ est S, X₄₄ est Q, X₄₅ est A, X₄₆ est K et X₄₇ est T, et
   ii) la séquence SEQ ID N°92 qui correspond à la séquence SEQ ID N°46 dans laquelle X₁₈ à X₄₀ sont tels que définis pour la séquence SEQ ID N°56 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°57 qui correspond à la séquence SEQ ID N°45 dans laquelle X₁₈ est V, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I, X₃₈ est V, X₃₉ est C et X₄₀ est Y, et au plus la séquence SEQ ID N°93 qui correspond à la séquence SEQ ID N°46 dans laquelle X₁₈ à X₄₀ sont tels que définis pour la séquence SEQ ID N°57 et X₄₁ est P, X₄₂ est A. X₄₃ est S. X₄₄ est Q, X₄₅ est S, X₄₆ est K et X₄₇ est T,
- les peptides ayant au moins la séquence SEQ ID N°58 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est F., X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est F, et au plus une séquence choisie parmi les séquences suivantes :
   i) la séquence SEQ ID N°94 qui correspond à la séquence SFQ ID N°46 dans laquelle X₁₈ à X₄₀ sont tels que définis pour la séquence SEQ ID N°58 et X₄₁ est P, X₄₂ est A, X₄₃ est A, X₄₄ est Q, X₄₅ est S, X₄₆ est K et X₄₇ est T, et
   ii) la séquence SEQ ID N°110 qui correspond à la séquence SEQ ID N°46 dans laquelle X₁₈ à X₄₀ sont tels que définis pour la séquence SEQ ID N°58 et X₁₁ est P, X₄₂ est A, X₄₃ est S, X₄₄ est Q, X₄₅ est T, X₄₆ est K et X₄₇ est T,
- les peptides ayant au moins la séquence SEQ ID N°59 qui correspond à la séquence SEQ ID N°45 dans laquelle X₁₈ est L, X₁₉ est L, X₂₀ est G, X₂₁ est C, X₂₂ est I, X₂₃ est V, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est A, X₃₉ est C et X₄₀ est F, et au plus la séquence SEQ ID N°95 qui correspond à la séquence SEQ ID N°46 dans laquelle X₁₈ à X₄₀ sont tels que définis pour la séquence SEQ ID N°59 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°60 qui correspond à la séquence SEQ ID N°45 dans laquelle X₁₈ est V, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est F, et au plus une séquence choisie parmi les séquences suivantes :
   i) la séquence SEQ ID N°96 qui correspond à la séquence SEQ ID N°46 dans laquelle X₁₈ à X₄₀ sont tels que définis pour la séquence SEQ ID N°60 et X₄₁ est P, X₄₂ est A, X₄₃ est A, X₄₄ est Q, X₄₅ est S, X₄₆ est K et X₄₇ est T, et
   ii) la séquence SEQ ID N°115 qui correspond à la séquence SEQ ID N°46 dans laquelle X₁₈ à X₄₀ sont tels que définis pour la séquence SEQ ID N°60 et X₄₁ est P, X₄₂ est A, X₄₃ est S, X₄₄ est Q. X₄₅ est S, X₄₆ est K et X₄₇ est T,
- les peptides ayant au moins la séquence SEQ ID N°61 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est D, X₂₈ est V, X₂₉ est V, X₃₀ est L, X₃₁ est T, X₃₂ est Q, X₃₃ est S, X₃₄ est A, X₃₅ est T, X₃₆ est C, X₃₇ est I, X₃₈ est V, X₃₉ est C et X₄₀ est Y, et au plus la séquence SEQ ID N°97 qui correspond à la séquence SEQ ID N°46
dans laquelle X₁₈ à X₄₀ sont tels que définis pour la séquence SEQ ID N°61 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°62 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est A, X₃₉ est C et X₄₀ est Y, et au plus une séquence choisie parmi les séquences suivantes :
   i) la séquence SEQ ID N°98 qui correspond à la séquence SEQ ID N°46 dans laquelle X₁₈ à X₄₀ sont tels que définis pour la séquence SEQ ID N°62 et X₄₁ est P, X₄₂ est A. X₄₃ est S. X₄₄ est Q, X₄₅ est S, X₄₆ est K et X₄₇ est T, et
   ii) la séquence SEQ ID N°109 qui correspond à la séquence SEQ ID N°46 dans laquelle X₁₈ à X₄₀ sont tels que définis pour la séquence SEQ ID N°62 et X₄₁ est P, X₄₂ est A, X₄₃ est S, X₄₄ est Q, X₄₅ est T, X₄₆ est K et X₄₇ est T,
- les peptides ayant au moins la séquence SEQ ID N°63 qui correspond à la séquence SEQ ID N°45 dans laquelle X₁₈ est V. X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est H, X₃₃ est S, X₃₄ est A, X₃₅ est T, X₃₆ est C, X₃₇ est I, X₃₈ est V, X₃₉ est C et X₄₀ est Y, et au plus la séquence SEQ ID N°99 qui correspond à la séquence SEQ ID N°46 dans laquelle X₁₈ à X₄₀ sont tels que définis pour la séquence SEQ ID N°63 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°64 qui correspond à la séquence SEQ ID N°45 dans laquelle X₁₈ est L, X₁₉ est L, X₂₀ est G, X₂₁ est C, X₂₂ est I, X₂₃ est l, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I, X₃₈ est A, X₃₉ est C et X₄₀ est Y, et au plus la séquence SEQ ID N°100 qui correspond à la séquence SEQ ID N°46 dans laquelle X₁₈ à X₄₀ sont tels que définis pour la séquence SEQ ID N°64 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°65 qui correspond à la séquence SEQ ID N°45 dans laquelle X₁₈ est L. X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est S, X₃₆ est C, X₃₇ est V, X₃₈ est V, X₃₉ est C et X₄₀ est Y, et au plus la séquence SEQ ID N°101 qui correspond à la séquence SEQ ID N°46 dans laquelle X₁₈ à X₄₀ sont tels que définis pour la séquence SEQ ID N°65 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°66 qui correspond à la séquence SEQ ID N°45 dans laquelle X₁₈ est L, X₁₉ est L. X₂₀ est G, X₂₁ est C, X₂₂ est I, X₂₃ est I, X₂₄ est K, X₂₅ est Q, X₂₆ est V, X₂₇ est F, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est T, X₃₈ est V, X₃₉ est C et X₄₀ est Y, et au plus la séquence SEQ ID N°102 qui correspond à la séquence SEQ ID N°46 dans laquelle X₁₈ à X₄₀ sont tels que définis pour la séquence SEQ ID N°66 et X₄₁ est P, X₄₂ est A, X₄₃ est S, X₄₄ est Q. X₄₅ est S, X₄₆ est K et X₄₇ est T,
- les peptides ayant au moins la séquence SEQ ID N°67 qui correspond à la séquence SEQ ID N°45 dans laquelle X₁₈ est V, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est Y, et au plus la séquence SEQ ID N°103 qui correspond à la séquence SEQ ID N°46 dans laquelle X₁₈ à X₄₀ sont tels que définis pour la séquence SEQ ID N°67 et X₄₁ est P, X₄₂ est A, X₄₃ est S. X₄₄ est Q, X₄₅ est S, X₄₆ est K et X₄₇ est T,
- les peptides ayant au moins la séquence SEQ ID N°68 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est K, X₃₂ est Q, X₃₃ est S, X₃₄ est A, X₃₅ est T, X₃₆ est C, X₃₇ est V, X₃₈ est V, X₃₉ est C et X₄₀ est Y, et au plus la séquence SEQ ID N°104 qui correspond à la séquence SEQ ID N°46 dans laquelle X₁₈ à X₄₀ sont tels que définis pour la séquence SEQ ID N°68 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°69 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est K, X₃₂ est Q, X₃₃ est S, X₃₄ est A, X₃₅ est T, X₃₆ est C, X₃₇ est V, X₃₈ est A, X₃₉ est C et X₄₀ est Y, et au plus la séquence SEQ ID N°106 qui correspond à la séquence SEQ ID N°46 dans laquelle X₁₈ à X₄₀ sont tels que définis pour la séquence SEQ ID N°69 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°70 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est T, X₃₄ est A, X₃₅ est T, X₃₆ est C, X₃₇ est V, X₃₈ est V, X₃₉ est C et X₄₀ est F, et au plus la séquence SEQ ID N°108 qui correspond à la séquence SEQ ID N°46 dans laquelle X₁₈ à X₄₀ sont tels que définis pour la séquence SEQ ID N°70 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°71 qui correspond à la séquence SEQ ID N°45 dans laquelle X₁₈ est L, X₁₉ est L, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est E, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est Y, et au plus la séquence SEQ ID N°111 qui correspond à la séquence SEQ ID N°46 dans laquelle X₁₈ à X₄₀ sont tels que définis pour la séquence SEQ ID N°71 et X₄₁ est P, X₄₂ est A, X₄₃ est S, X₄₄ est Q, X₄₅ est S, X₄₆ est K et X₄₇ est I,
- les peptides ayant au moins la séquence SEQ ID N°72 qui correspond à la séquence SEQ ID N°45 dans laquelle X₁₈ est L, X₁₉ est F, X₂₀ est G, X₂₁ est C, X₂₂ est I, X₂₃ est V, X₂₄ est K, X₂₅ est Q, X₂₆ est V, X₂₇ est E, X₂₈ est A, X₂₉ est V, X₃₀ est V, X₃, est T, X₃₂ est Q, X₃₃ est S, X₃₄ est A, X₃₅ est T, X₃₆ est C, X₃₇ est V, X₃₈ est V, X₃₉ est C et X₄₀ est Y, et au plus la séquence SEQ ID N°112 qui correspond à la séquence SEQ ID N°46 dans laquelle X₁₈ à X₄₀ sont tels que définis pour la séquence SEQ ID N°72 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°73 qui correspond à la séquence SEQ ID N°45 dans laquelle X₁₈ est L, X₁₉ est F, X₂₀ est G, X₂₁ est C, X₂₂ est I, X₂₃ est I, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S; X₃₄ est A, X₃₅ est T, X₃₆ est C, X₃₇ est 1, X₃₈ est V, X₃₉ est C et X₄₀ est Y, et au plus la séquence SEQ ID N°113 qui correspond à la séquence SFQ ID N°46
dans laquelle X₁₈ à X₄₀ sont tels que définis pour la séquence SEQ ID N°73 et X₄₁ est P, X₄₂ est A, X₄₃; est S, X₄₄ est Q, X₄₅ est S, X₄₆ est K et X₄₇ est T,
- les peptides ayant au moins la séquence SEQ ID N°74 qui correspond à la séquence SEQ ID N°45 dans laquelle X₁₈ est L, X₁₉ est F, X₂₀ est G, X₂₁ est C, X₂₂ est I, X₂₃ est V, X₂₄ est K, X₂₅ est Q, X₂₆, est V, X₂₇ est E, X₂₈ est A, X₂₉ est V, X₃₀ est V, X₃₁ est T. X₃₂ est Q, X₃₃ est S, X₃₄ est A, X₃₅ est T. X₃₆ est C. X₃₇ est V, X₃₈ est A, X₃₉ est C et X₄₀ est Y, et au plus la séquence SEQ ID N°114 qui correspond à la séquence SEQ ID N°46 dans laquelle X₁₈ à X₄₀ sont tels que définis pour la séquence SEQ ID N°74 et X₄₁ est P, X₄₂ est A, X₄₃ est S, X₄₄ est Q, X₄₅ est S, X₄₆ est K et X₄₇ est T,
- les peptides ayant au moins la séquence SEQ ID N°75 qui correspond à la séquence SEQ ID N°45 dans laquelle X₁₈ est L, X₁₉ est L, X₂₀ est G, X₂₁ est C, X₂₂ est I, X₂₃ est V, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est F, et au plus une séquence choisie parmi les séquences suivantes :
   i) la séquence SEQ ID N°118 qui correspond à la séquence SEQ ID N°46 dans laquelle X₁₈ à X₄₀ sont tels que définis pour la séquence SEQ ID N°75 et X₄₁ est S, X₄₂ est A, X₄₃ est S, X₄₄ est Q, X₄₅ est S, X₄₆ est K et X₄₇ est T, et
   ii) la séquence SEQ ID N°119 qui correspond à la séquence SEQ ID N°46 dans laquelle X₁₈ à X₄₀ sont tels que définis pour la séquence SEQ ID N°75 et X₄₁ est P, X₄₂ est A, X₄₃ est A, X₄₄ est Q, X₄₅ est S, X₄₆ est K et X₄₇ est T,
- les peptides ayant au moins la séquence SEQ ID N°76 qui correspond à la séquence SEQ ID N°45 dans laquelle X₁₈ est L, X₁₉ est F, X₂₀ est G, X₂₁, est C, X₂₂ est I, X₂₃ est I, X₂₄ est R, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I, X₃₈ est V, X₃₉ est C et X₄₀ est Y, et au plus la séquence SEQ ID N°121 qui correspond à la séquence SEQ ID N°46
dans laquelle X₁₈ à X₄₀ sont tels que définis pour la séquence SEQ ID N°76 et X₄₁ est P. X₄₂ est A, X₄₃ est A, X₄₄ est Q, X₄₅ est S, X₄₆ est K et X₄₇ est T,
- les peptides ayant au moins la séquence SEQ ID N°77 qui correspond à la séquence SEQ ID N°45 dans laquelle X₁₈ est L, X₁₉ est F, X₂₀ est G, X₂₁ est C, X₂₂ est I, X₂₃ est 1, X₂₄ est K, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I, X₃₈ est V, X₃₉ est C et X₄₀ est Y, et au plus la séquence SEQ ID N° 122 qui correspond à la séquence SEQ ID N°46
dans laquelle X₁₈ à X₄₀ sont tels que définis pour la séquence SEQ ID N°77 et X₄₁ est P, X₄₂ est A, X₄₃ est A, X₄₄ est Q, X₄₅ est S, X₄₆ est K et X₄₇ est T,
- les peptides ayant au moins la séquence SEQ ID N°78 qui correspond à la séquence SEQ ID N°45 dans laquelle X₁₈ est L, X₁₉ est L, X₂₀ est G, X₂₁ est C, X₂₂ est I, X₂₃ est I, X₂₄ est R, X₂₅ est Q, X₂₆ est V, X₂₇ est E, X₂₈ est V, X₂₉ est V, X₃₀ est V, X₃₁ est T, X₃₂ est Q, X₃₃ est S, X₃₄ est A, X₃₅ est T, X₃₆ est C, X₃₇ est I, X₃₈ est V, X₃₉ est C et X₄₀ est F, et au plus la séquence SEQ ID N° 123 qui correspond à la séquence SEQ ID N°46
dans laquelle X₁₈ à X₄₀ sont tels que définis pour la séquence SEQ ID N°78 et X₄₁ est P, X₄₂ est A, X₄₃ est S, X₄₄ est Q, X₄₅ est S, X₄₆ est K et X₄₇ est T, les peptides ayant au moins la séquence SEQ ID N°79 qui correspond à la séquence SEQ ID N°45 dans laquelle X₁₈ est L, X₁₉ est L, X₂₀ est G, X₂₁ est C, X₂₂ est I, X₂₃ est I, X₂₄ est R, X₂₅ est Q, X₂₆ est V, X₂₇ est E, X₂₈ est V, X₂₉ est M, X₃₀ est V, X₃₁ est T, X₃₂ est Q, X₃₃ est S, X₃₄ est A, X₃₅ est T, X₃₆ est C, X₃₇ est V, X₃₈ est V, X₃₉ est C et X₄₀ est Y, et au plus la séquence SEQ ID N°125 qui correspond à la séquence SEQ ID N°46 dans laquelle X₁₈ à X₄₀ sont tels que définis pour la séquence SEQ ID N°79 et X₄₁ est H, X₄₂ est A, X₄₃ est S, X₄₄ est Q, X₄₅ est S, X₄₆ est K et X₄₇ est T, et
- les peptides ayant au moins la séquence SEQ ID N°80 qui correspond à la séquence SEQ ID N°45 dans laquelle X₁₈ est L, X₁₉ est F, X₂₀ est S, X₂₁ est T, X₂₂ est I, X₂₃ est I, X₂₄ est T, X₂₅ est E, X₂₆ est N, X₂₇ est C, X₂₈ est V, X₂₉ est V, X₃₀ est L, X₃₁ est T, X₃₂ est Q, X₃₃ est S, X₃₄ est G, X₃₅ est T, X₃₆ est A, X₃₇ est V, X₃₈ est V, X₃₉ est M et X₄₀ est Y, et au plus la séquence SEQ ID N°126 qui correspond à la séquence SEQ ID N°46
dans laquelle X₁₈ à X₄₀ sont tels que définis pour la séquence SEQ ID N°80 et X₄₁ est P, X₄₂ est A, X₄₃ est S, X₄₄ est Q, X₄₅ est A, X₄₆ est K et X₄₇ est T.

De préférence, le peptide B est choisi parmi les peptides de séquences SEQ ID N°47 à 80, le peptide de séquence SEQ ID N°47 étant particulièrement préféré.

Le peptide C est situé entre les positions 1767 et 1823 de la polyprotéine virale.

Selon un autre mode de réalisation, le peptide C est choisi parmi les peptides suivants:
- les peptides ayant au moins la séquence SEQ ID N°129 qui correspond à la séquence SEQ ID N°127 dans laquelle X₄₇ est L, X₄₈ est A, X₄₉ est A, X₅₀ est S, X₅₁ est I, X₅₂ est T, X₅₃ est T, X₅₄ est Q, X₅₅ est N, X₅₆ est L, X₅₇ est I, X₅₈ est A et X₅₉ est L et au plus une séquence choisie parmi les séquences suivantes :
   i) la séquence SEQ ID N°147 qui correspond à la séquence SEQ ID N°128 dans laquelle X₄₇ à X₅₉ sont tels que définis pour la séquence SEQ ID N°129 et X₆₀ est T, X₆₁ est I, X₆₂ est G, X₆₃ est P, X₆₄ est I, X₆₅ est A, X₆₆ est A, X₆₇ est P et X₆₈ est P,
   ii) la séquence SEQ ID N°153 qui correspond à la séquence SEQ ID N°128 dans laquelle X₄₇ à X₅₉, sont tels que définis pour la séquence SEQ ID N°129 et X₆₀ est S, X₆₁ est I, X₆₂ est G, X₆₃ est P. X₆₄ est I, X₆₅ est A, X₆₆ est A, X₆₇ est P et X₆₈ est P,
   iii) la séquence SEQ ID N°162 qui correspond à la séquence SEQ ID N°128 dans laquelle X₄₇ à X₅₉ sont tels que définis pour la séquence SEQ ID N°129 et X₆₀ est S, X₆₁ est I, X₆₂ est A, X₆₃ est P, X₆₄ est I, X₆₅ est A, X₆₆ est A, X₆₇ est P et X₆₈ est A, et
   iv) la séquence SEQ ID N°167 qui correspond à la séquence SEQ ID N°128 dans laquelle X₄₇ à X₅₉ sont tels que définis pour la séquence SEQ ID N°129 et X₆₀ est S, X₆₁ est V, X₆₂ est G, X₆₃ est P, X₆₄ est I, X₆₅ est A, X₆₆ est A, X₆₇ est P et X₆₈ est P,
- les peptides ayant au moins la séquence SEQ ID N°130 qui correspond à la séquence SEQ ID N°127 dans laquelle X₄₇ est L, X₄₈ est A, X₄₉ est A, X₅₀ est A, X₅₁ est V, X₅₂ est T, X₅₃ est T, X₅₄ est S, X₅₅ est Q, X₅₆ est L, X₅₇ est L, X₅₈ est A et X₅₉ est L et au plus une séquence choisie parmi les séquences suivantes :
   i) la séquence SEQ ID N°148 qui correspond à la séquence SEQ ID N° 128 dans laquelle X₄₇ à X₅₉ sont tels que définis pour la séquence SEQ ID N°130 et X₆₀ est S, X₆₁ est I, X₆₂ est 6, X₆₃ est P, X₆₄ est I, X₆₅ est A, X₆₆ est A, X₆₇ est A et X₆₈ est P, et
   ii) la séquence SEQ ID N°150 qui correspond à la séquence SEQ ID N°128 dans laquelle X₄₇ à X₅₀ sont tels que définis pour la séquence SEQ ID N°130 et X₆₀ est S. X₆₁ est T, X₆₂ est G, X₆₃ est P, X₆₄ est I, X₆₅ est A., X₆₆ est A, X₆₇ est A et X₆₈ est P,
- les peptides ayant au moins la séquence SEQ ID N°131 qui correspond à la séquence SEQ ID N°127 dans laquelle X₄₇ est L, X₄₈ est A, X₄₉ est A, X₅₀ est A, X₅₁ est V, X₅₂ est T, X₅₃ est T, X₅₄ est G, X₅₅ est Q, X₅₆ est L, X₅₇ est L, X₅₈ est A et X₅₉ est L et au plus la séquence SEQ ID N°149 qui correspond à la séquence SEQ ID N°128 dans laquelle X₄₇ à X₅₉ sont tels que définis pour la séquence SEQ ID N°131 et X₆₀ est S, X₆₁ est I, X₆₂ est G, X₆₃ est P, X₆₄ est I, X₆₅ est A, X₆₆ est A, X₆₇ est A et X₆₈ est P
- les peptides ayant au moins la séquence SEQ ID N°132 qui correspond à la séquence SEQ ID N°127 dans laquelle X₄₇ est L, X₄₈ est A, X₄₉ est A, X₅₀ est S, X₅₁ est I, X₅₂ est T, X₅₃ est T, X₅₄ est Q, X₅₅ est H, X₅₆ est L, X₅₇ est L, X₅₈ est A et X₅₉ est L et au plus une séquence choisie parmi les séquences suivantes :
   i) la séquence SEQ ID N°151 qui correspond à la séquence SEQ ID N°128 dans laquelle X₄₇ à X₅₉ sont tels que définis pour la séquence SEQ ID N°132 et X₆₀ est S, X₆₁ est I, X₆₂ est G, X₆₃ est P, X₆₄ est I, X₆₅ est A, X₆₆ est A, X₆₇ est P et X₆₈ est P,
   ii) la séquence SEQ ID N°155 qui correspond à la séquence SEQ ID N°128 dans laquelle X₄₇ à X₅₉ sont tels que définis pour la séquence SEQ ID N°132 et X₆₀ est S, X₆₁ est I, X₆₂ est G, X₆₃ est P, X₆₄ est I, X₆₅ est V, X₆₆ est A, X₆₇ est P et X₆₈ est P,
   iii) la séquence SEQ ID N°168 qui correspond à la séquence SEQ ID N°128 dans laquelle X₄₇ à X₅₉ sont tels que définis pour la séquence SEQ ID N°132 et X₆₀ est S, X₆₁ est V, X₆₂ est G, X₆₃ est P, X₆₄ est I, X₆₅ est A, X₆₆ est A, X₆₇ est P et X₆₈ est P. et
   iv) la séquence SEQ ID N°171 qui correspond à la séquence SEQ ID N°128 dans laquelle X₄₇ à X₅₉ sont tels que définis pour la séquence SEQ ID N°132 et X₆₀ est S, X₆₁ est I, X₆₂ est G, X₆₃ est L, X₆₄ est I, X₆₅ est A, X₆₆ est A, X₆₇ est P et X₆₈ est P,
- les peptides ayant au moins la séquence SEQ ID N°133 qui correspond à la séquence SEQ ID N°127 dans laquelle X₄₇ est I., X₄₈ est A, X₄₉ est A, X₅₀ est S, X₅₁ est I, X₅₂ est T, X₅₃ est T, X₅₄ est Q, X₅₅ est S, X₅₆ est I., X₅₇ est L, X₅₈ est A et X₅₉ est L et au plus la séquence SEQ ID N°152 qui correspond à la séquence SEQ ID N°128 dans laquelle X₄₇ à X₅₉ sont tels que définis pour la séquence SEQ ID N°133 et X₆₀ est S, X₆₁ est I, X₆₂ est G, X₆₃ est P, X₆₄ est I, X₆₅ est A, X₆₆ est A, X₆₇ est P et X₆₈ est P,
- les peptides ayant au moins la séquence SEQ ID N°134 qui correspond à la séquence SEQ ID N°127 dans laquelle X₄₇ est L, X₄₈ est A, X₄₉ est A, X₅₀ est S, X₅₁ est I, X₅₂ est T, X₅₃ est T, X₅₄ est Q, X₅₅ est Y, X₅₆ est I., X₅₇ est L, X₅₈ est A et X₅₉ est L et au plus une séquence choisie parmi les séquences suivantes:
   i) la séquence SEQ ID N°154 qui correspond à la séquence SEQ ID N°128 dans laquelle X₄₇ à X₅₉ sont tels que définis pour la séquence SEQ ID N°134 et X₆₀ est S, X₆₁ est I, X₆₂ est G, X₆₃ est P, X₆₄ est I, X₆₅ est A, X₆₆ est A, X₆₇ est P et X₆₈ est P, et
   ii) la séquence SEQ ID N°169 qui correspond à la séquence SEQ ID N°128 dans laquelle X₄₇ à X₅₉ sont tels que définis pour la séquence SEQ ID N°134 et X₆₀ est S, X₆₁ est V, X₆₂ est G, X₆₃ est P, X₆₄ est I, X₆₅ est A, X₆₆ est A, X₆₇ est P et X₆₈ est P,
- les peptides ayant au moins la séquence SEQ ID N°135 qui correspond à la séquence SEQ ID N°127 dans laquelle X₄₇ est L, X₄₈ est A, X₄₉ est A, X₅₀ est S, X₅₁ est V, X₅₂ est T, X₅₃ est T, X₅₄ est Q, X₅₅ est N, X₅₆ est L, X₅₇ est L, X₅₈ est A et X₅₉ est L et au plus la séquence SEQ ID N°156 qui correspond à la séquence SEQ ID N°128 dans laquelle X₄₇ à X₅₉ sont tels que définis pour la séquence SHQ ID N°135 et X₆₀ est S, X₆₁ est L X₆₂ est G, X₆₃ est P, X₆₄ est I, X₆₅ est A, X₆₆ est A, X₆₇ est P et X₆₈ est P,
- les peptides ayant au moins la séquence SEQ ID N°136 qui correspond à la séquence SEQ ID N°127 dans laquelle X₄₇ est L, X₄₈ est A, X₄₉ est A, X₅₀ est S, X₅₁ est V, X₅₂ est T, X₅₃ est T, X₅₄ est Q, X₅₅ est S, X₅₆ est L, X₅₇ est L, X₅₈ est A et X₅₉ est L et au plus une séquence choisie parmi les séquences suivantes :
   i) la séquence SEQ ID N°157 qui correspond à la séquence SEQ ID N°128 dans laquelle X₄₇ à X₅₀ sont tels que définis pour la séquence SEQ ID N°136 et X₆₀ est S, X₆₁ est I, X₆₂ est G, X₆₃ est P, X₆₄ est I, X₆₅ est A, X₆₆ est A, X₆₇ est P et X₆₈ est P, et
   ii) la séquence SEQ ID N°170 qui correspond à la séquence SEQ ID N°128 dans laquelle X₄₇ à X₅₉ sont tels que définis pour la séquence SEQ ID N°136 et X₆₀ est S, X₆₁ est V, X₆₂ est G, X₆₃ est P, X₆₄ est I, X₆₅ est A, X₆₆ est A, X₆₇ est P et X₆₈ est P,
- les peptides ayant au moins la séquence SEQ ID N°137 qui correspond à la séquence SEQ ID N°127 dans laquelle X₄₇ est L, X₄₈ est A, X₄₉ est A, X₅₀ est S. X₅₁ est I, X₅₂ est T, X₅₃ est T, X₅₄ est Q, X₅₅ est T, X₅₆ est M, X₅₇ est L, X₅₈ est A et X₅₉ est I, et au plus la séquence SEQ ID N°158 qui correspond à la séquence SEQ ID N°128 dans laquelle X₄₇ à X₅₉ sont tels que définis pour la séquence SEQ ID N°137 et X₆₀ est S, X₆₁ est I, X₆₂ est G, X₆₃ est P, X₆₄ est I, X₆₅ est A, X₆₆ est A, X₆₇ est P et X₆₈ est P,
- les peptides ayant au moins la séquence SEQ ID N°138 qui correspond à la séquence SEQ ID N° 127 dans laquelle X₄₇ est L, X₄₈ est A, X₄₉ est A, X₅₀ est S, X₅₁, est V, X₅₂ est T, X₅₃ est T, X₅₄ est Q, X₅₅ est Y, X₅₆ est L, X₅₇ est L, X₅₈ est A et X₅₉ est I et au plus la séquence SEQ ID N°159 qui correspond à la séquence SEQ ID N°128 dans laquelle X₄₇ à X₅₉ sont tels que définis pour la séquence SEQ ID N°138 et X₆₀ est S, X₆₁ est I. X₆₂ est G, X₆₃ est P, X₆₄ est I, X₆₅ est A, X₆₆ est A, X₆₇ est P et X₆₈ est P,
- les peptides ayant au moins la séquence SEQ ID N°139 qui correspond à la séquence SEQ ID N°127 dans laquelle X₄₇ est P, X₄₈ est A, X₄₉ est A, X₅₀ est S, X₅₁ est I, X₅₂ est T, X₅₃ est T, X₅₄ est Q. X₅₅ est H, X₅₆ est L. X₅₇ est I, X₅₈ est A et X₅₉ est L et au plus la séquence SEQ ID N°160 qui correspond à la séquence SEQ ID N°128 dans laquelle X₄₇ à X₅₉ sont tels que définis pour la séquence SEQ ID N°139 et X₆₀ est S, X₆₁ est I, X₆₂ est G, X₆₃ est P, X₆₄ est I, X₆₅ est R, X₆₆ est A, X₆₇ est P et X₆₈ est P,
- les peptides ayant au moins la séquence SEQ ID N°140 qui correspond à la séquence SEQ ID N°127 dans laquelle X₄₇ est L, X₄₈ est A, X₄₉ est A, X₅₀ est S, X₅₁ est I, X₅₂ est T, X₅₃ est I, X₅₄ est Q, X₅₅ est H, X₅₆ est L, X₅₇ est L, X₅₈ est A et X₅₉ est P et au plus la séquence SEQ ID N°161 qui correspond à la séquence SEQ ID N°128 dans laquelle X₄₇ à X₅₉ sont tels que définis pour la séquence SEQ ID N° 140 et X₆₀ est S, X₆₁ est I, X₆₂ est G, X₆₃ est P, X₆₄ est I, X₆₅ est A, X₆₆ est A, X₆₇ est P et X₆₈ est P,
- les peptides ayant au moins la séquence SEQ ID N°141 qui correspond à la séquence SEQ ID N°127 dans laquelle X₄₇ est L, X₄₈ est A, X₄₉ est S, X₅₀ est S, X₅₁ est I, X₅₂ est T, X₅₃ est T, X₅₄ est Q, X₅₅ est S, X₅₆ est L, X₅₇ est L, X₅₈ est A et X₅₉ est L et au plus la séquence SEQ ID N°163 qui correspond à la séquence SEQ ID N°128 dans laquelle X₄₇ à X₅₉ sont tels que définis pour la séquence SEQ ID N°141 et X₆₀ est S, X₆₁ est I, X₆₂ est G, X₆₃ est P, X₆₄ est I, X₆₅ est A, X₆₆ est A, X₆₇ est P et X₆₈ est P,
- les peptides ayant au moins la séquence SEQ ID N°142 qui correspond à la séquence SEQ ID N°127 dans laquelle X₄₇ est L, X₄₈ est A, X₄₉ est A, X₅₀ est S, X₅₁ est I, X₅₂ est S, X₅₃ est T, X₅₄ est Q, X₅₅ est N, X₅₆ est L, X₅₇ est W, X₅₈ est A et X₅₉ est L et au plus la séquence SEQ ID N°164 qui correspond à la séquence SEQ ID N°128 dans laquelle X₄₇ à X₅₉ sont tels que définis pour la séquence SEQ ID N°142 et X₆₀ est S, X₆₁ est I, X₆₂ est G, X₆₃ est P, X₆₄ est I, X₆₅ est A, X₆₆ est A, X₆₇ est P et X₆₈ est P,
- les peptides ayant au moins la séquence SEQ ID N°143 qui correspond à la séquence SEQ ID N°127 dans laquelle X₄₇ est L, X₄₈ est A, X₄₉ est A, X₅₀ est S, X₅₁ est I, X₅₂ est T, X₅₃ est T, X₅₄ est Q, X₅₅ est N, X₅₆ est M, X₅₇ est L, X₅₈ est A et X₅₉ est L et au plus la séquence SEQ ID N°165 qui correspond à la séquence SEQ ID N°128 dans laquelle X₄₇ à X₅₉ sont tels que définis pour la séquence SEQ ID N°143 et X₆₀ est S, X₆₁ est I, X₆₂ est G, X₆₃ est P, X₆₄ est I, X₆₅ est A, X₆₆ est A, X₆₇ est P et X₆₈ est P,
- les peptides ayant au moins la séquence SEQ ID N°144 qui correspond à la séquence SEQ ID N° 127 dans laquelle X₄₇ est L, X₄₈ est A, X₄₉ est A, X₅₀ est S, X₅₁ est I, X₅₂ est T, X₅₃ est T, X₅₄ est Q, X₅₅ est H, X₅₆ est M, X₅₇ est L, X₅₈ est A et X₅₉ est L et au plus la séquence SEQ ID N°166 qui correspond à la séquence SEQ ID N°128 dans laquelle X₄₇ à X₅₉ sont tels que définis pour la séquence SEQ ID N°144 et X₆₀ est S, X₆₁ est I, X₆₂ est G, X₆₃ est P, X₆₄ est M, X₆₅ est A, X₆₆ est A, X₆₇ est P et X₆₈ est P,
- les peptides ayant au moins la séquence SEQ ID N°145 qui correspond à la séquence SEQ ID N° 127 dans laquelle X₄₇ est L, X₄₈ est A, X₄₉ est A, X₅₀ est S, X₅₁ est I, X₅₂ est A, X₅₃ est T, X₅₄ est Q, X₅₅ est Y, X₅₆ est I, X₅₇ est L, X₅₈ est A et X₅₉ est L et au plus la séquence SEQ ID N°172 qui correspond à la séquence SEQ ID N°128 dans laquelle X₄₇ à X₅₉ sont tels que définis pour la séquence SEQ ID N°145 et X₆₀ est S, X₆₁ est I, X₆₂ est G, X₆₃ est P, X₆₄ est I, X₆₅ est A, X₆₆ est A, X₆₇ est P et X₆₈ est P,
- les peptides ayant au moins la séquence SEQ ID N°146 qui correspond à la séquence SEQ ID N°127 dans laquelle X₄₇ est L, X₄₈ est S, X₄₉ est A, X₅₀ est A, X₅₁ est V, X₅₂ est T, X₅₃ est T, X₅₄ est Q, X₅₅ est Q, X₅₆ est L, X₅₇ est L, X₅₈ est S et X₅₉ est I et au plus la séquence SEQ ID N°173 qui correspond à la séquence SEQ ID N°128 dans laquelle X₄₇ à X₅₉ sont tels que définis pour la séquence SEQ ID N°146 et X₆₀ est S, X₆₁ est I, X₆₂ est G, X₆₃ est P, X₆₄ est I, X₆₅ est A, X₆₆ est R, X₆₇ est D et X₆₈ est S.

De préférence, le peptide C est choisi parmi les peptides de séquences SEQ ID N°129 à 146, le peptide de séquence SEQ ID N°129 étant particulièrement préféré.

Le peptide D est situé entre les positions 2570 et 2613 de la polyprotéine virale.

Selon encore un mode de réalisation, le peptide D est choisi parmi les peptides suivants:
- les peptides ayant au moins la séquence SEQ ID N°176 qui correspond à la séquence SEQ ID N°174 dans laquelle X₆₉ est R, X₇₀ est P, X₇₁ est L, X₇₂ est F, X₇₃ est D, X₇₄ est V, X₇₅ est M, X₇₆ est Y, X₇₇ est D, X₇₈ est V. X₇₉ est S, X₈₀ est T et X₈₁ est L, et au plus une séquence choisie parmi :
   i) la séquence SEQ ID N°188 qui correspond à la séquence SEQ ID N°175 dans laquelle X₆₉ à X₈₁ sont tels que définis pour la séquence SEQ TD N°176 et X₈₂ est P, X₈₃ est Q, X₈₄ est A, X₈₅ est P, X₈₆ est S, X₈₇ est G et X₈₈ est F,
   ii) la séquence SEQ ID N°192 qui correspond à la séquence SEQ ID N°175 dans laquelle X₆₉ à X₈₁ sont tels que définis pour la séquence SEQ ID N°176 et X₈₂ est P, X₈₃ est Q, X₈₄ est A, X₈₅ est S, X₈₆ est S, X₈₇ est G et X₈₈ est F,
   iii) la séquence SEQ ID N°193 qui correspond à la séquence SEQ ID N°175 dans laquelle X₆₉ à X₈₁ sont tels que définis pour la séquence SEQ ID N°176 et X₈₂ est P, X₈₃ est Q, X₈₄ est A, X₈₅ est A, X₈₆ est S, X₈₇ est G et X₈₈ est F,
   iv) la séquence SEQ ID N°194 qui correspond à la séquence SEQ ID N°175 dans laquelle X₆₉ à X₈₁ sont tels que définis pour la séquence SEQ ID N°176 et X₈₂ est P, X₈₃ est Q, X₈₄ est V, X₈₅ est S, X₈₆ est S, X₈₇ est G et X₈₈ est F,
   v) la séquence SEQ ID N°201 qui correspond à la séquence SEQ ID N°175 dans laquelle X₆₉ à X₈₁ sont tels que définis pour la séquence SEQ ID N°176 et X₈₂ est P, X₈₃ est R, X₈₄ est A, X₈₅ est S, X₈₆ est S, X₈₇ est G et X₈₈ est C,
   vi) la séquence SEQ ID N°202 qui correspond à la séquence SEQ ID N°175 dans laquelle X₆₉ à X₈₁ sont tels que définis pour la séquence SEQ ID N°176 et X₈₂ est P, X₈₃ est Q, X₈₄ est P, X₈₅ est S, X₈₆ est S, X₈₇ est G et X₈₈ est F,
   vii) la séquence SEQ ID N°203 qui correspond à la séquence SEQ ID N°175 dans laquelle X₆₉ à X₈₁ sont tels que définis pour la séquence SEQ ID N°176 et X₈₂ est P, X₈₃ est H, X₈₄ est A, X₈₅ est S, X₈₆ est S, X₈₇ est G et X₈₈ est F,
   viii) la séquence SEQ ID N°204 qui correspond à la séquence SEQ ID N°175 dans laquelle X₆₉ à X₈₁ sont tels que définis pour la séquence SEQ ID N°176 et X₈₂ est P, X₈₃ est Q, X₈₄ est A, X₈₅ est S, X₈₆ est A, X₈₇ est G et X₈₈ est F,
   ix) la séquence SEQ ID N°205 qui correspond à la séquence SEQ ID N°175 dans laquelle X₆₉ à X₈₁ sont tels que définis pour la séquence SEQ ID N°176 et X₈₂ est P, X₈₃ est Q, X₈₄ est A, X₈₅ est S, X₈₆ est S, X₈₇ est R et X₈₈ est F, et
   x) la séquence SEQ ID N°210 qui correspond à la séquence SEQ ID N°175 dans laquelle X₆₉ à X₈₁ sont tels que définis pour la séquence SEQ ID N°176 et X₈₂ est P, X₈₃ est H, X₈₄ est T, X₈₅ est S, X₈₆ est S, X₈₇ est G et X₈₈ est F,
- les peptides ayant au moins la séquence SEQ ID N°177 qui correspond à la séquence SEQ ID N°174 dans laquelle X₆₉ est R, X₇₀ est P, X₇₁ est L, X₇₂ est F, X₇₃ est D, X₇₄ est V, X₇₅ est M, X₇₆ est Y, X₇₇ est D, X₇₈ est V, X₇₉ est S, X₈₀ est K et X₈₁ est L, et au plus une séquence choisie parmi les séquences suivantes :
   i) la séquence SEQ ID N°189 qui correspond à la séquence SEQ ID N°175 dans laquelle X₆₉ à X₈₁ sont tels que définis pour la séquence SEQ ID N°177 et X₈₂ est P, X₈₃ est L, X₈₄ est A, X₈₅ est S, X₈₆ est S, X₈₇ est G et X₈₈ est F, et
   ii) la séquence SEQ ID N°190 qui correspond à la séquence SEQ ID N°175 dans laquelle X₆₉ à X₈₁ sont tels que définis pour la séquence SEQ ID N°177 et X₈₂ est P, X₈₃ est P, X₈₄ est A, X₈₅ est S, X₈₆ est S, X₈₇ est G et X₈₈ est F,
- les peptides ayant au moins la séquence SEQ ID N°178 qui correspond à la séquence SEQ ID N°174 dans laquelle X₆₉ est R, X₇₀ est P, X₇₁ est L, X₇₂ est F, X₇₃ est D, X₇₄ est V, X₇₅ est M, X₇₆ est Y, X₇₇ est D, X₇₈ est V, X₇₉ est T, X₈₀ est K et X₈₁ est L, et au plus la séquence SEQ ID N°191 qui correspond à la séquence SEQ ID N°175 dans laquelle X₆₉ à X₈₁, sont tels que définis pour la séquence SEQ ID N°178 et X₈₂ est P, X₈₃ est L, X₈₄ est A, X₈₅ est S; X₈₆ est S, X₈₇ est G et X₈₈ est F,
- les peptides ayant au moins la séquence SEQ ID N°179 qui correspond à la séquence SEQ ID N°174 dans laquelle X₆₉ est Q, X₇₀ est P, X₇₁ est L, X₇₂ est F, X₇₃ est D, X₇₄ est V, X₇₅ est M, X₇₆ est Y, X₇₇ est D, X₇₈ est V, X₇₉ est S, X₈₀ est T et X₈₁ est L, et au plus la séquence SEQ ID N°195 qui correspond à la séquence SEQ ID N°175 dans laquelle X₆₉ à X₈₁ sont tels que définis pour la séquence SEQ ID N°179 et X₈₂ est P, X₈₃ est Q, X₈₄ est A, X₈₅ est S, X₈₆ est S, X₈₇ est G et X₈₈ est F,
- les peptides ayant au moins la séquence SEQ ID N°180 qui correspond à la séquence SEQ ID N°174 dans laquelle X₆₉ est R, X₇₀ est A, X₇₁ est L, X₇₂ est F, X₇₃ est D, X₇₄ est V, X₇₅ est M, X₇₆ est Y, X₇₇ est D, X₇₈ est V, X₇₉ est S, X₈₀ est T et X₈₁ est L, et au plus une séquence choisie parmi les séquences suivantes :
   i) la séquence SEQ ID N°196 qui correspond à la séquence SEQ ID N°175 dans laquelle X₆₉ à X₈₁ sont tels que définis pour la séquence SEQ ID N°180 et X₈₂ est P, X₈₃ est Q, X₈₄ est A, X₈₅ est S, X₈₆ est S, X₈₇ est G et X₈₈ est F, et
   ii) la séquence SEQ ID N°206 qui correspond à la séquence SEQ ID N°175 dans laquelle X₆₉ à X₈₁ sont tels que définis pour la séquence SEQ ID N°180 et X₈₂ est P, X₈₃ est Q, X₈₄ est A, X₈₅ est P, X₈₆ est S, X₈₇ est G et X₈₈ est F,
- les peptides ayant au moins la séquence SEQ ID N°181 qui correspond à la séquence SEQ ID N°174 dans laquelle X₆₉ est R, X₇₀ est P, X₇₁ est L, X₇₂ est F, X₇₃ est D, X₇₄ est V, X₇₅ est M, X₇₆ est Y, X₇₇ est D, X₇₈ est V, X₇₉ est S, X₈₀ est I et X₈₁ est L, et au plus la séquence SEQ ID N°197 qui correspond à la séquence SEQ ID N°175 dans laquelle X₆₉ à X₈₁ sont tels que définis pour la séquence SEQ ID N°181 et X₈₂ est P, X₈₃ est Q, X₈₄ est A, X₈₅ est S, X₈₆ est S, X₈₇ est G et X₈₈ est F,
- les peptides ayant au moins la séquence SEQ ID N°182 qui correspond à la séquence SEQ ID N°174 dans laquelle X₆₉ est R, X₇₀ est P, X₇₁ est L, X₇₂ est F, X₇₃ est E, X₇₄ est V, X₇₅ est M, X₇₆ est Y, X₇₇ est D. X₇₈ est V, X₇₉ est S, X₈₀ est T et X₈₁ est L, et au plus une séquence choisie parmi les séquences suivantes :
   i) la séquence SEQ ID N°198 qui correspond à la séquence SEQ ID N°175 dans laquelle X₆₉ à X₈₁ sont tels que définis pour la séquence SEQ ID N°182 et X₈₂ est P, X₈₃ est Q, X₈₄ est A, X₈₅ est S, X₈₆ est S, X₈₇ est G et X₈₈ est F, et
   ii) la séquence SEQ ID N°209 qui correspond à la séquence SEQ ID N°175 dans laquelle X₆₉ à X₈₁ sont tels que définis pour la séquence SEQ ID N°182 et X₈₂ est P, X₈₃ est Q, X₈₄ est A, X₈₅ est P, X₈₆ est S, X₈₇ est G et X₈₈ est F,
- les peptides ayant au moins la séquence SEQ ID N°183 qui correspond à la séquence SEQ ID N°174 dans laquelle X₆₉ est R, X₇₀ est P, X₇₁ est F, X₇₂ est F, X₇₃ est D, X₇₄ est V, X₇₅ est M, X₇₆ est Y, X₇₇ est D, X₇₈ est V, X₇₉ est S, X₈₀ est T et X₈₁ est L, et au plus une séquence choisie parmi les séquences suivantes :
   i) la séquence SEQ ID N°199 qui correspond à la séquence SEQ ID N°175 dans laquelle X₆₉ à X₈₁ sont tels que définis pour la séquence SEQ ID N°183 et X₈₂ est P, X₈₃ est K, X₈₄ est A, X₈₅ est S, X₈₆ est S, X₈₇ est G et X₈₈ est F, et
   ii) la séquence SEQ ID N°200 qui correspond à la séquence SEQ ID N°175 dans laquelle X₆₉ à X₈₁ sont tels que définis pour la séquence SEQ ID N°183 et X₈₂ est P, X₈₃ est Q, X₈₄ est A, X₈₅ est S, X₈₆ est S, X₈₇ est G et X₈₈ est F,
- les peptides ayant au moins la séquence SEQ ID N°184 qui correspond à la séquence SEQ ID N°174 dans laquelle X₆₉ est R, X₇₀ est A, X₇₁ est L, X₇₂ est F, X₇₃ est D, X₇₄ est V, X₇₅ est M, X₇₆ est Y, X₇₇ est N, X₇₈ est V, X₇₉ est S, X₈₀ est T et X₈₁ est L, et au plus la séquence SEQ ID N°207 qui correspond à la séquence SEQ ID N°175 dans laquelle X₆₉ à X₈₁ sont tels que définis pour la séquence SEQ ID N°184 et X₈₂ est P, X₈₃ est Q, X₈₄ est A, X₈₅ est S, X₈₆ est S, X₈₇ est G et X₈₈ est F,
- les peptides ayant au moins la séquence SEQ ID N°185 qui correspond à la séquence SEQ ID N°174 dans laquelle X₆₉ est R, X₇₀ est P, X₇₁ est L, X₇₂ est F, X₇₃ est D, X₇₄ est V, X₇₅ est M, X₇₆ est Y, X₇₇ est D, X₇₈ est V, X₇₉ est S, X₈₀ est N et X₈₁ est L, et au plus la séquence SEQ ID N°208 qui correspond à la séquence SEQ ID N°175 dans laquelle X₆₉ à X₈₁ sont tels que définis pour la séquence SEQ ID N°185 et X₈₂ est P, X₈₃ est Q, X₈₄ est A, X₈₅ est S, X₈₆ est S, X₈₇ est G et X₈₈ est F,
- les peptides ayant au moins la séquence SEQ ID N°186 qui correspond à la séquence SEQ ID N°174 dans laquelle X₆₉ est R, X₇₀ est P, X₇₁ est L, X₇₂ est Y, X₇₃ est D, X₇₄ est V, X₇₅ est M, X₇₆ est Y, X₇₇ est D, X₇₈ est V, X₇₉ est S, X₈₀ est T et X₈₁ est L, et au plus la séquence SEQ ID N°211 qui correspond à la séquence SEQ ID N° 175 dans laquelle X₆₉ à X₈₁ sont tels que définis pour la séquence SEQ ID N°186 et X₈₂ est P, X₈₃ est Q. X₈₄ est A, X₈₅ est S. X₈₆ est S, X₈₇ est G et X₈₈ est F,
- les peptides ayant au moins la séquence SEQ ID N°187 qui correspond à la séquence SEQ ID N°174 dans laquelle X₆₉ est R, X₇₀ est P, X₇₁ est T., X₇₂ est Y, X₇₃ est D, X₇₄ est S, X₇₅ est R, X₇₆ est H, X₇₇ est D, X₇₈ est 1, X₇₉ est K, X₈₀ est K et X₈₁ est T, et au plus la séquence SEQ ID N°212 qui correspond à la séquence SEQ ID N°175 dans laquelle X₆₉ à X₈₁ sont tels que définis pour la séquence SEQ ID N°187 et X₈₂ est A, X₈₃ est L, X₈₄ est A, X₈₅ est A, X₈₆ est A, X₈₇ est G et X₈₈ est F.

De préférence, le peptide D est choisi parmi les peptides de séquences SEQ ID N°176 à 187, le peptide de séquence SEQ ID N°176 étant préféré.

Les peptides A, B, C ou D tels que définis par les séquences 1 à 212 précédentes sont nouveaux et constituent également un objet de l'invention.

Ainsi, selon l'invention :
- le peptide A a au moins la séquence en acides aminés SEQ ID N°1 et au plus la séquence SEQ ID N°2 ; en particulier il est choisi parmi les peptides :
   - ayant au moins la séquence SEQ ID N°3 et au plus la séquence SEQ ID N°19,
   - ayant au moins la séquence SEQ ID N°4 et au plus une séquence choisie parmi les séquences SEQ ID N°20, SEQ ID N°24, SEQ ID, N°32 et SEQ ID N°34,
   - ayant au moins la séquence SEQ ID N°5 et au plus une séquence choisie parmi les séquences SEQ ID N°21, SEQ ID N°28 et SEQ ID N°36,
   - ayant au moins la séquence SEQ ID N°6 et au plus une séquence choisie parmi les séquences SEQ ID N°22, SEQ ID N°27 et SEQ ID N°41,
   - ayant au moins la séquence SEQ ID N°7 et au plus une séquence choisie parmi les séquences SEQ ID N°23 et SEQ ID N°37,
   - ayant au moins la séquence SEQ ID N°8 et au plus la séquence SEQ ID N°25,
   - ayant au moins la séquence SEQ ID N°9 et au plus la séquence SEQ ID N°26,
   - ayant au moins la séquence SEQ ID N°10 et au plus la séquence SEQ ID N°29,
   - ayant au moins la séquence SEQ ID N°11 et au plus la séquence SEQ ID N°30,
   - ayant au moins la séquence SEQ ID N°12 et au plus la séquence SEQ ID N°31,
   - ayant au moins la séquence SEQ ID N°13 et au plus la séquence SEQ ID N°33,
   - ayant au moins la séquence SEQ ID N°14 et au plus une séquence choisie parmi les séquences SEQ ID N°35 et SEQ ID N°39,
   - ayant au moins la séquence SEQ ID N°15 et au plus la séquence SEQ ID N°38,
   - ayant au moins la séquence SEQ ID N°16 et au plus une séquence choisie parmi les séquences SEQ ID N°40 et SEQ ID N°42,
   - ayant au moins la séquence SEQ ID N°17 et au plus la séquence SEQ ID N°43, et
   - ayant au moins la séquence SEQ ID N°18 et au plus la séquence SEQ ID N°44, les peptides de séquence SEQ ID N°3 à 18 étant préférés et le peptide de séquence SEQ ID N°3 étant particulièrement préféré,
- le peptide B a au moins la séquence en acides aminés SEQ ID N°45 et au plus la séquence SEQ ID N°46 ; en particulier, le peptide B est choisi parmi les peptides :
   - ayant au moins la séquence SEQ ID N°47 et au plus la séquence SEQ ID N°81,
   - ayant au moins la séquence SEQ ID N°48 et au plus la séquence SEQ ID N°82,
   - ayant au moins la séquence SEQ ID N°49 et au plus la séquence SEQ ID N°83,
   - ayant au moins la séquence SEQ ID N°50 et au plus une séquence choisie parmi les séquences SEQ ID N°84, SEQ ID N°90. SEQ ID N°105, SEQ ID N°107 et SEQ ID N°116.
   - ayant au moins la séquence SEQ ID N°51 et au plus une séquence choisie parmi les séquences SEQ ID N°85 et SEQ ID N°124,
   - ayant au moins la séquence SEQ ID N°52 et au plus une séquence choisie parmi les séquences SEQ ID N°86 et SEQ ID N°120,
   - ayant au moins la séquence SEQ ID N°53 et au plus la séquence SEQ ID N°87,
   - ayant au moins la séquence SEQ ID N°54 et au plus une séquence choisie parmi les séquences SEQ ID N°88 et SEQ ID N°117,
   - ayant au moins la séquence SEQ ID N°55 et au plus la séquence SEQ ID N°89,
   - ayant au moins la séquence SEQ ID N°56 et au plus une séquence choisie parmi les séquences SEQ ID N°91 et SEQ ID N°92,
   - ayant au moins la séquence SEQ ID N°57 et au plus la séquence SEQ ID N°93,
   - ayant au moins la séquence SEQ ID N°58 et au plus une séquence choisie parmi les séquences SEQ ID N°94 et SEQ ID N°110,
   - ayant au moins la séquence SEQ ID N°59 et au plus la séquence SEQ ID N°95,
   - ayant au moins la séquence SEQ ID N°60 et au plus une séquence choisie parmi les séquences SEQ ID N°96 et SEQ ID N°115,
   - ayant au moins la séquence SEQ ID N°61 et au plus la séquence SEQ ID N°97,
   - ayant au moins la séquence SEQ ID N°62 et au plus une séquence choisie parmi les séquences SEQ ID N°98 et SEQ ID N°109,
   - ayant au moins la séquence SEQ ID N°63 et au plus la séquence SEQ ID N°99,
   - ayant au moins la séquence SEQ ID N°64 et au plus la séquence SEQ ID N°100,
   - ayant au moins la séquence SEQ ID N°65 et au plus la séquence SEQ ID N°101,
   - ayant au moins la séquence SEQ ID N°66 et au plus la séquence SEQ ID N°102,
   - ayant au moins la séquence SEQ ID N°67 et au plus la séquence SEQ ID N°103,
   - ayant au moins la séquence SEQ ID N°68 et au plus la séquence SEQ ID N°104,
   - ayant au moins la séquence SEQ ID N°69 et au plus la séquence SEQ ID N°106,
   - ayant au moins la séquence SEQ ID N°70 et au plus la séquence SEQ ID N°108,
   - ayant au moins la séquence SEQ ID N°71 et au plus la séquence SEQ ID N°111,
   - ayant au moins la séquence SEQ ID N°72 et au plus la séquence SEQ ID N°112,
   - ayant au moins la séquence SEQ ID N°73 et au plus la séquence SEQ ID N°113,
   - ayant au moins la séquence SEQ ID N°74 et au plus la séquence SEQ ID N°114,
   - ayant au moins la séquence SEQ ID N°75 et au plus une séquence choisie parmi les séquences SEQ ID N°118 et SEQ ID N°119
   - ayant au moins la séquence SEQ ID N°76 et au plus la séquence SEQ ID N°121,
   - ayant au moins la séquence SEQ ID N°77 et au plus la séquence SEQ ID N°122,
   - ayant au moins la séquence SEQ ID N°78 et au plus la séquence SEQ ID N°123,
   - ayant au moins la séquence SEQ ID N°79 et au plus la séquence SEQ ID N°125, et
   - ayant au moins la séquence SEQ ID N°80 et au plus la séquence SEQ ID N°126,
   les peptides de séquence SEQ ID N°47 à 80 étant préférés et le peptide de séquence SEQ ID N°47 étant particulièrement préféré,
- le peptide C a au moins la séquence en acides aminés SEQ ID N°127 et au plus la séquence SEQ ID N°128 ; en particulier, le peptide C est choisi parmi les peptides :
   - ayant au moins la séquence SEQ ID N°129 et au plus une séquence choisie parmi les séquences SEQ ID N°147, SEQ ID N°153, SEQ ID N°162 et SEQ ID N°167,
   - ayant au moins la séquence SEQ ID N°130 et au plus une séquence choisie parmi les séquences SEQ ID N°148 et SEQ ID N°150,
   - ayant au moins la séquence SEQ ID N°131 et au plus la séquence SEQ ID N° 149,
   - ayant au moins la séquence SEQ ID N°132 et au plus une séquence choisie parmi les séquences SEQ ID N°151, SEQ ID N°155, SEQ ID N°168 et SEQ ID N°171,
   - ayant au moins la séquence SEQ ID N°133 et au plus la séquence SEQ ID N° 152,
   - ayant au moins la séquence SEQ ID N°134 et au plus une séquence choisie parmi les séquences SEQ ID N°154 et SEQ ID N°169,
   - ayant au moins la séquence SEQ ID N°135 et au plus, la séquence SEQ ID N°156,
   - ayant au moins la séquence SEQ ID N°136 et au plus une séquence choisie parmi les séquences SEQ ID N°157 et SEQ ID N°170,
   - ayant au moins la séquence SEQ ID N°137 et au plus la séquence SEQ ID N°158,
   - ayant au moins la séquence SEQ ID N°138 et au plus la séquence SEQ ID N°159,
   - ayant au moins la séquence SEQ ID N°139 et au plus la séquence SEQ ID N°160,
   - ayant au moins la séquence SEQ ID N°140 et au plus la séquence SEQ ID N°161,
   - ayant au moins la séquence SEQ ID N°141 et au plus la séquence SEQ ID N°163,
   - ayant au moins la séquence SEQ ID N°142 et au plus la séquence SEQ ID N°164,
   - ayant au moins la séquence SEQ ID N°143 et au plus la séquence SEQ ID N° 165,
   - ayant au moins la séquence SEQ ID N°144 et au plus la séquence SEQ ID N°166.
   - ayant au moins la séquence SEQ ID N°145 et au plus la séquence SEQ ID N°172, et
   - ayant au moins la séquence SEQ ID N°146 et au plus la séquence SEQ ID N°173,
   les peptides de séquence SEQ ID N°129 à 146 étant préférés et le peptide de séquence SEQ ID N°129 étant particulièrement Préféré, et
- le peptide D a au moins la séquence en acides aminés SEQ ID N°174 et au plus la séquence SEQ ID N°175 ; en particulier, le peptide D est choisi parmi les peptides :
   - ayant au moins la séquence SEQ ID N°176 et au plus une séquence choisie parmi les séquences SEQ ID N°188, SEQ ID N°192, SEQ ID N°193, SEQ ID N°194, SEQ ID N°201, SEQ ID N°202, SEQ ID N°203, SEQ ID N°204, SEQ ID N°205 et SEQ ID N°210,
   - ayant au moins la séquence SEQ ID N°177 et au plus une séquence choisie parmi les séquences SEQ ID N°189 et SEQ ID N°190,
   - ayant au moins la séquence SEQ ID N°178 et au plus la séquence SEQ ID N°191,
   - ayant au moins la séquence SEQ ID N°179 et au plus la séquence SEQ ID N° 195,
   - ayant au moins la séquence SEQ ID N°180 et au plus une séquence choisie parmi les séquences SEQ ID N°196 et SEQ ID N°206,
   - ayant au moins la séquence SEQ ID N°181 et au plus la séquence SEQ ID N°197,
   - ayant au moins la séquence SEQ ID N°182 et au plus une séquence choisie parmi les séquences SEQ ID N°198 et SEQ ID N°209,
   - ayant au moins la séquence SEQ ID N°183 et au plus une séquence choisie parmi les séquences SEQ ID N°199 et SEQ ID N°200,
   - ayant au moins la séquence SEQ ID N°184 et au plus la séquence SEQ ID N°207,
   - ayant au moins la séquence SEQ ID N°185 et au plus la séquence SEQ ID N°208,
   - ayant au moins la séquence SEQ ID N°186 et au plus la séquence SEQ ID N°211, et
   - ayant au moins la séquence SEQ ID N°187 et au plus la séquence SEQ ID N°212,
   les peptides de séquence SEQ ID N°176 à 187 étant préférés et le peptide de séquence SEQ ID N°176 étant particulièrement préféré.

L'expression « le peptide A a au moins la séquence en acides aminés SEQ ID N°1 et au plus la séquence SEQ ID N°2 » signifie que l'extrémité N-terminale est délimitée par l'acide aminé situé à l'une des positions 1 à 8 de la SEQ ID N°2 et l'extrémité C-terminale est délimitée par l'acide aminé situé à l'une des positions 38 à 46 de la SEQ ID N°2.

Ainsi le peptide A a au moins les 31 acides aminés consécutifs de séquence SEQ ID N°1 et au plus les 46 acides aminés consécutifs de séquence SEQ ID N°2.

La séquence SEQ ID N°2 inclut les 31 acides aminés de la séquence SEQ ID N°1.

Ainsi le peptide A de l'invention a toujours au moins les 31 acides aminés de la séquence SEQ ID N° 1 et au plus 15 acides aminés supplémentaires distribués de part et d'autre de ces 31 acides aminés dans la limite de la séquence SEQ ID N°2. Par exemple, un peptide A de l'invention peut comporter 33 acides aminés constitués de 31 acides aminés de la SEQ ID N°1 et soit 2 acides aminés N-terminaux, soit 2 acides aminés C-terminaux, soit 1 acide aminé N-terminal et un acide aminé C-terminal.

L'expression « le peptide B a au moins la séquence en acides aminés SEQ ID N°45 et au plus la séquence SEQ ID N°46 » signifie que l'extrémité N-terminale est délimitée par l'acide aminé situé à l'une des positions 1 à 12 de la SEQ ID N°46 et l'extrémité C-terminale est délimitée par l'acide aminé situé à l'une des positions 56 à 63 de la SEQ ID N°46.

Ainsi le peptide B a au moins les 45 acides aminés consécutifs de séquence SEQ ID N° 45 et au plus les 63 acides aminés consécutifs de séquence SEQ ID N°46.

La séquence SEQ ID N°46 inclut les 45 acides aminés de la séquence SEQ ID N°45.

Ainsi le peptide B de l'invention a toujours au moins les 45 acides aminés de la séquence SEQ ID N° 45 et au plus 18 acides aminés supplémentaires distribués de part et d'autre de ces 45 acides aminés dans la limite de la séquence SEQ ID N°46.

L'expression « le peptide C a au moins la séquence en acides aminés SEQ ID N°127 et au plus la séquence SEQ ID N°128 » signifie que l'extrémité N-terminale est délimitée par l'acide aminé situé à l'une des positions 1 à 23 de la SEQ ID N°128 et l'extrémité C-terminale est délimitée par l'acide aminé situé à l'une des positions 54 à 57 de la SEQ ID N°128.

Ainsi le peptide C a au moins les 32 acides aminés consécutifs de séquence SEQ ID N°127 et au plus les 57 acides aminés consécutifs de séquence SEQ ID N°128.

La séquence SEQ ID N°128 inclut les 32 acides aminés de la séquence SEQ ID N°127.

Ainsi le peptide C de l'invention a toujours au moins les 32 acides aminés de la séquence SEQ ID N°127 et au plus 25 acides aminés supplémentaires distribués de part et d'autre de ces 32 acides aminés dans la limite de la séquence SEQ ID N°128.

L'expression « le peptide D a au moins la séquence en acides aminés SEQ ID N°174 et au plus la séquence SEQ ID N°175 » signifie que l'extrémité N-terminale est délimitée par l'acide aminé situé à l'une des positions 1 à 4 de la SEQ ID N°175 et l'extrémité C-terminale est délimitée par l'acide aminé situé à l'une des positions 32 à 44 de la SEQ ID N°175.

Ainsi le peptide D a au moins les 29 acides aminés consécutifs de séquence SEQ ID N°174 et au plus les 44 acides aminés consécutifs de séquence SEQ ID N°175.

La séquence SEQ ID N°175 inclut les 29 acides aminés de la séquence SEQ ID N°174.

Ainsi le peptide D de l'invention a toujours au moins les 29 acides aminés de la séquence SEQ ID N°174 et au plus 15 acides aminés supplémentaires distribués de part et d'autre de ces 29 acides aminés dans la limite de la séquence SEQ ID N°175.

Les peptides B et C contiennent de nouveaux épitopes présentant un fort pouvoir immunogène.

Un autre objet de l'invention concerne l'épitope B', contenu dans le peptide B et situé entre les positions 1038 et 1047 de la polyprotéine virale, lequel est choisi parmi les épitopes suivants :
- l'épitope de séquence SEQ ID N°215 qui correspond à la séquence SEQ ID N°213
dans laquelle X₁₈ est L, X₁₉ est F, X₂₀ est G, X₂₁ est C, X₂₂ est I et X₂₃ est I,
- l'épitope de séquence SEQ ID N°216 qui correspond à la séquence SEQ ID N°213
dans laquelle X₁₈ est V, X₁₉ est L, X₂₀ est G, X₂₁ est C, X₂₂ est V et X₂₃ est I,
- l'épitope de séquence SEQ ID N°217 qui correspond à la séquence SEQ ID N°213
dans laquelle X₁₈ est L, X₁₉ est V, X₂₀ est G, X₂₁ est C, X₂₂ est I et X₂₃ est I,
- l'épitope de séquence SEQ ID N°218 qui correspond à la séquence SEQ ID N°213
dans laquelle X₁₈ est L, X₁₉ est L, X₂₀ est G, X₂₁ est C, X₂₂ est I et X₂₃ est V,
- l'épitope de séquence SEQ ID N°219 qui correspond à la séquence SEQ ID N°213
dans laquelle X₁₈ est L, X₁₉ est F, X₂₀ est G, X₂₁ est C. X₂₂ est I et X₂₃ est V, et
- l'épitope de séquence SEQ ID N°220 qui correspond à la séquence SEQ ID N°213
dans laquelle X₁₈ est L, X₁₉ est F, X₂₀ est S, X₂₁ est T, X₂₂ est I et X₂₃ est I.

De préférence, l'épitupe B' possède la caractéristique suivante :
- il est restreint au HLA-A2.

Un autre objet de l'invention concerne l'épitope C', contenu dans le peptide C et situé entre les positions 1789 et 1821 de la polyprotéine virale, lequel est choisi parmi les épitopes suivants :
- l'épitope de séquence SEQ ID N°223 qui correspond à la séquence SEQ ID N°221 dans laquelle X₅₂ est T, X₅₃ est T, X₅₄ est S et X₅₃ est Q.
- l'épitope de séquence SEQ ID N°224 qui correspond à la séquence SEQ ID N°221 dans laquelle X₅₂ est T, X₅₃ est T, X₅₄ est G et X₅₅ est Q,
- l'épitope de séquence SEQ ID N°225 qui correspond à la séquence SEQ ID N°221 dans laquelle X₅₂ est T. X₅₃ est T, X₅₄ est Q et X₅₅ est H,
- l'épitope de séquence SEQ ID N°226 qui correspond à la séquence SEQ ID N°221 dans laquelle X₅₂ est T, X₅₃ est T, X₅₄ est Q et X₅₅ est S,
- l'épitope de séquence SEQ ID N°227 qui correspond à la séquence SEQ ID N°221 dans laquelle X₅₂ est T, X₅₃ est T, X₅₄ est Q et X₅₅ est Y,
- l'épitope de séquence SEQ ID N°228 qui correspond à la séquence SEQ ID N°221 dans laquelle X₅₂ est T, X₅₃ est T, X₅₄ est Q et X₅₅ est T,
- l'épitope de séquence SEQ ID N°229 qui correspond à la séquence SEQ ID N°221 dans laquelle X₅₂ est T, X₅₃ est I, X₅₄ est Q et X₅₅ est H,
- l'épitope de séquence SEQ ID N°230 qui correspond à la séquence SEQ ID N°221 dans laquelle X₅₂ est S, X₅₃ est T, X₅₄ est Q et X₅₅ est N,
- l'épitope de séquence SEQ ID N°231 qui correspond à la séquence SEQ ID N°221 dans laquelle X₅₂ est A, X₅₃ est T, X₅₄ est Q et X₅₅ est Y, et
- l'épitope de séquence SEQ ID N°232 qui correspond à la séquence SEQ ID N°221 dans laquelle X₅₂ est T, X₅₃ est T, X₅₄ est Q et X₅₅ est Q.

De préférence, l'épitope C' possède la caractéristique suivante :
- il est restreint au HLA-B7.

Les compositions de l'invention, outre les peptides A à D, peuvent également contenir les épitopes B' et C', ce qui constitue un autre objet de l'invention.

La présente invention concerne également les séquences nuctéotidiques codant pour l'un quelconque des peptides A à D tels que définis par les séquences SEQ ID N°1 à 212 et des épitopes B' et C' tels que définis par les séquences SEQ ID N° 215 à 220 et SEQ ID N° 223 à 232.

Les peptides de l'invention peuvent être obtenus par la technique du génie génétique qui comprend les étapes de :
- culture d'un microorganisme ou de cellules eucaryotes transformé(es) à l'aide d'une séquence nucléotidique selon l'invention et
- récupération du peptide produit par ledit microorganisme ou lesdites cellules eucaryotes.

Cette technique est bien connue de l'homme du métier. Pour plus de détail la concernant, on pourra se référer à l'ouvrage ci-après: Recombinant DNA Technology 1, Editors Ales Prokop, Raskesh K Bajpai ; Annals of the New-York Academy of Sciences, Volume 646, 1991.

Les peptides de l'invention peuvent également être préparés par tes synthèses peptidiques classiques bien connues de l'homme du métier.

Les séquences nucléotidiques selon l'invention peuvent être préparées par synthèse chimique et génie génétique en utilisant les techniques bien connues de l'homme du métier et décrites par exemple dans Sambrook J. et al., Molecular Cloning : A Laboratory Manual, 1989.

Les séquences nucléotidiques de l'invention peuvent être insérées dans des vecteurs d'expression afin d'obtenir les compositions ou les peptides de l'invention.

Ainsi, un autre objet de l'invention consiste en les vecteurs d'expression comprenant une séquence nucléotidique de l'invention, ainsi que les moyens nécessaires à son expression.

A titre de vecteur d'expression, on peut citer par exemple les plasmides, les vecteurs viraux du type virus de la vaccine, adenovirus, baculovirus, poxvirus, les vecteurs bactériens du type salmonelle, BCG.

On entend par moyen nécessaire à l'expression d'un peptide, tout moyen qui permet d'obtenir le peptide, tel que notamment un promoteur, un terminateur de transcription, une origine de réplication et de préférence un marqueur de sélection.

Les vecteurs de l'invention peuvent également comprendre des séquences nécessaires au ciblage des peptides vers des compartiments cellulaires particuliers. Un exemple de ciblage peut être le ciblage vers le réticulum endoplasmique obtenu en utilisant des séquences d'adresssage du type de la séquence leader issue de la protéine E3 de l'adénovirus (Ciernik I.F., et al., The Journal of Immunology, 1999, 162, 3915-3925).

Les vecteurs d'expression de l'invention peuvent comprendre soit une seule séquence nucléotidique codant pour l'un quelconque des peptides de l'invention, soit au moins deux séquences nueléotidiques, étant étendu que chaque séquence nucléotidique code pour un peptide de type différent.

Selon un mode de réalisation de l'invention, les vecteurs d'expression comprennent deux séquences nucléotidiques codant pour les peptides A et B, A et C, A et D, B et C, B et D, ou D et C.

De préférence, les vecteurs d'expression comprennent deux séquences nucléotidiques codant pour les peptides A et B, A et C, B et D, ou C et D, les vecteurs comprenant deux séquences codant pour les peptides A et B, C et D, et B et D étant particulièrement préférés.

Selon un autre mode de réalisation, les vecteurs d'expression comprennent trois séquences nucléotidiques codant pour les peptides A,13 et C, A, B et D, A, C et D, ou B, C et D.

Selon encore un autre mode, les vecteurs d'expression comprennent quatre séquences nucléotidiques codant pour les peptides A à D. De préférence, les vecteurs comprennent trois séquences nueléotidiques codant pour les peptides A, B et C, A. C et D, et B, C et D. Dans ce cas, l'ordre des séquences nucléotidiqucs importe peu, comme dans les combinaisons précédentes, de sorte que les combinaisons suivantes, données par rapport aux peptides, sont comprises dans la portée de l'invention : A/B/C/D. A/B/D/C, A/C/B/D, A/C/D/B, A/D/B/C, A/D/C/B, B/A/C/D, B/A/D/C, B/C/A/D, B/C/D/A, B/D/A/C, B/D/C/A, C/A/B/D, C/A/D/B, C/B/A/D, C/B/D/A, C/D/A/B, CID/B/A, D/A/B/C, D/A/C/B, D/B/A/C, D/B/C/A, D/C/A/B et D/C/B/A.

Les vecteurs d'expression de l'invention peuvent également comprendre au moins une séquence nucléotidique codant pour l'un quelconque des épitopes B' et C' tels que définis précédemment, cc qui constitue un autre mode de réalisation de l'invention.

Ainsi, les vecteurs de l'invention peuvent comprendre par exemple :
- une séquence nucléotidique codant pour l'un des épitopes suivants : B' et C',
- deux séquences nueléotidiques codant pour les épitopes B' et C' ou
- plusieurs séquences répétitives codant pour l'épitope B', pour l'épitope C' ou pour les deux.

Les vecteurs de l'invention peuvent également comprendre de deux à quatre séquences nucléotidiqueç choisies parmi les séquences codant pour les peptides A, B, C, D et les épitopes B' et C', étant entendu que:
- la séquence nucléotidique codant pour le peptide B et la séquence nucléotidique codant pour l'épitope B' ne sont pas présentes en même temps, et
- la séquence nucléotidique codant pour le peptide C et la séquence nucléotidique codant pour l'Gpitopc C' ne sont pas présentes en même temps.

Ainsi, par exemple, les vecteurs de l'invention peuvent comprendre les combinaisons suivantes, données par rapport aux dits peptides et épitopes : A/B', A/C', A'/D, B/C'. B'/C, B'/D, C'/D, A/B'/C, A/B'/C', A/B/C', A/B'/D, A/C'/D, B'/C/D, B/C'/D, B'/C'/D, A/B'/C/D, A/B/C'/D et A/B'/C'/D.

Bien entendu, comme précédemment, l'ordre des séquences nucléotidiques dans les vecteurs d'expression importe peu.

Lorsque les vecteurs d'expression de l'invention comprennent plusieurs séquences nucléotidiques, lesdites séquences peuvent être liées directement entre elles, ou bien par l'intermédiaire d'agents espaceurs ou lieurs qui sont typiquement constitués de petites molécules neutres telles que des acides aminés ou mimétiques d'acides aminés qui ont typiquement une charge neutre dans des conditions physiologiques.

A titre d'agents espaceurs, on peut citer les résidus Ala, Gly ou d'autres agents espaceurs neutres d'acides aminés non polaires ou d'acides aminés polaires neutres.

Ces acides aminés cspaceurs ont au moins un ou deux résidus et habituellement de 3 à 6 résidus.

L'invention a également pour objet les microorganismes et les cellules eucaryotes transformés par un vecteur d'expression de l'invention.

Lorsqu'on veut obtenir une composition de l'invention contenant au moins deux peptides A à D de l'invention, les microorganismes ou cellules eucaryotes sont transformés par un vecteur d'expression contenant au moins deux séquences nucléotidiques, ou bien ils sont cotransformés par au moins deux vecteurs d'expression contenant une seule séquence nucléotidique, chaque vecteur codant pour un peptide de type différent.

Selon un mode de réalisation de l'invention, les microorganismes et cellules eucaryotes sont cotransformés avec :
- deux vecteurs codant respectivement pour les peptides A et B, A et C, A et D, B et C, B et D, ou C et D,
- trois vecteurs codant respectivement pour les peptides A, B et C, A, B et D, A, C et D, ou B, C et D, ou bien
- quatre vecteurs codant respectivement pour les peptides A, B, C et D.

De même, lorsqu'on veut obtenir une composition de l'invention contenant au moins deux épitopes B' et C', ou bien au moins un épitope B' ou C' et au moins un peptide A à D, les microorganismes ou cellules eucaryotes sont transformés par un seul vecteur codant pour la combinaison d'épitopes ou épitopes/peptides souhaitée ou par plusieurs vecteurs codant chacun pour chaque constituant de la combinaison souhaitée.

A titre d'exemples de microorganismes qui conviennent aux fins de l'invention, on peut citer les levures, telles que celles des familles suivantes: *Saccharomyces, Schizosaccharomyces, Kluveromyces, Pichia, Hanseluna, Yarowia, Schwaniomyces, Zygosaccharomyces, Saccharomyces cerevitiae, Saccharomyces carlsbergensis* et *Kluveromyces lactis* étant préférées; et les bactéries, telles que *E*. *coli* et celles des familles suivantes : *Lactobacillus, Lactococcus, Salmonella, Streprococcus, Bacillus et Streptomyces.*

A titre d'exemples de cellules eucaryotes, on peut citer les cellules provenant d'animaux tels que les mammifères, les reptiles, les insectes et équivalent. Les cellules eucaryotes préférées sont les cellules provenant du hamster chinois (cellules CHO), du singe (cellules COS et Vero), du rein de hamster nain (cellules BHK), du rein de cochon (cellules PK 15) et du rein de lapin (cellules RK13, les lignées cellulaires humaines de l'ostéosacorme (cellules 143 B), les lignées cellulaires humaines HeLa et les lignées cellulaires humaines de l'hépatome (du type cellules Hep G2), ainsi que les lignées cellulaires d'insecte (par exemple de *Spodoptera frugiperda*).

Les cellules hôtes peuvent être fournies dans des cultures en suspension ou en flacon, dans des cultures tissulaires, des cultures d'organe et équivalent. Les cellules hôtes peuvent également être des animaux transgéniques.

L'invention concerne également des anticorps dirigés contre l'un des susdits peptides de l'invention ou contre l'une des compositions peptidiques de l'invention telles que définies précédemment, ou bien encore contre l'un des épitopes de l'invention.

Les anticorps selon l'invention sont soit des anticorps polyclonaux, soit monoclonaux.

Les anticorps polyclonaux sus-mentionnés peuvent être obtenus par immunisation d'un animal avec au moins un antigène viral d'intérêt, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un antigène viral d'intérêt.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec un antigène viral d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de rcconnaissance vis-à-vis de l'antigène viral d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les compositions de l'invention, contenant au moins deux peptides A à D ou au moins un des épitopes B' et C' tels que décrits précédemment, sont particulièrement efficaces pour l'inhibition, la prévention et le traitement du virus ou de l'infection des patients porteurs du virus appartenant plus particulièrement aux génotypes 1a, 1b et 4. de sorte que son utilisation pour la préparation d'un médicament constitue un autre objet de l'invention.

La présente invention concerne également une composition pharmaceutique, notamment vaccin, contenant à titre de substance active au moins deux peptides différents choisis parmi les peptides A à D tels que définis précédemment, ou bien au moins deux séquences nucléotidiques telles que décrites précédemment, placées sous le contrôle d'éléments nécessaires à une expression constitutive et/ou inductible desdits peptides, ou bien l'un au moins des anticorps tels que définis précédemment, ou bien encore au moins un des épitopes B et C' tels que définis précédemment, ou bien au moins une de leurs séquences nucléotidiques, en association avec un véhicule pharmaceutiquement approprié.

Par éléments nécessaires à une expression constitutive des peptides, on entend un promoteur ubiquitaire ou spécifique des cellules eucaryotes.

A titre d'éléments nécessaires à une expression inductible des peptides, on peut citer les éléments de régulation de l'opéron de *E. coli* pour la résistance à la tétracycline (Gossen M. et al, Proc Natl Acad Sci USA, 89 : 5547-5551 (1992).

Bien entendu, l'homme du métier déterminera facilement le véhicule pharmaceutiquement approprié et la quantité de peptides à utiliser en fonction des constituants de la composition pharmaceutique.

L'invention concerne également une composition diagnostique pour la détection et/ou la quantification du virus de l'hépatite C comprenant au moins deux peptides différents choisis parmi les peptides A à D tels que définis précédemment, ou bien au moins un épitope B' ou C' tels que définis précédemment, ou bien au moins un anticorps tel que défini précédemment.

Là encore, l'homme du métier déterminera facilement la quantité de peptides à utiliser en fonction de la technique diagnostique utilisée.

L'invention concerne également un procédé de détection et/ou de quantification du virus de l'hépatite C dans un échantillon biologique prélevé chez un individu susceptible d'être infecté par ledit virus, tel que plasma, sérum ou tissu, caractérisé en ce qu'il comprend les étapes consistant à :
mettre en contact ledit échantillon biologique avec l'un au moins des anticorps de l'invention dans des conditions permettant la formation d'un complexe entre le virus et l'anticorps,
- détecter et/ou quantifier la formation dudit complexe par tout moyen approprié.

Les procédés de détection et/ou quantification du virus sont mis en oeuvre à l'aide de techniques classiques bien connues de l'homme du métier et on peut citer, à titre d'illustration, les blots, les techniques dites sandwich, les techniques de compétition et les techniques de détection par PCR, notamment celles dites « en temps réel ».

L'invention concerne également l'utilisation des compositions de l'invention pour le diagnostic *in vitro* du virus de l'hépatite C dans un échantillon ou prélèvement biologique.

Enfin, l'invention concerne l'utilisation des compositions de l'invention pour la préparation d'une composition vaccinale.

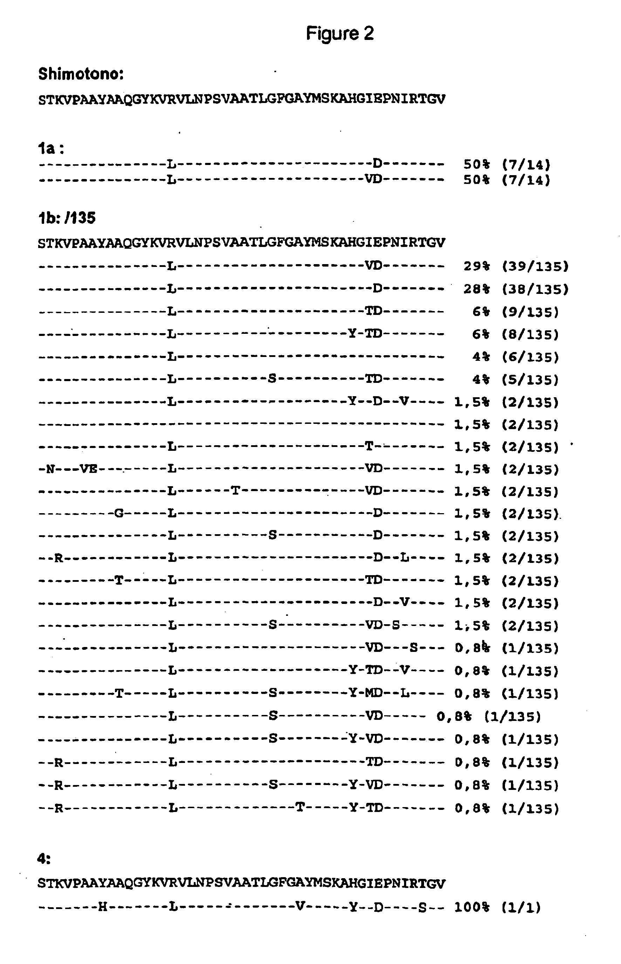

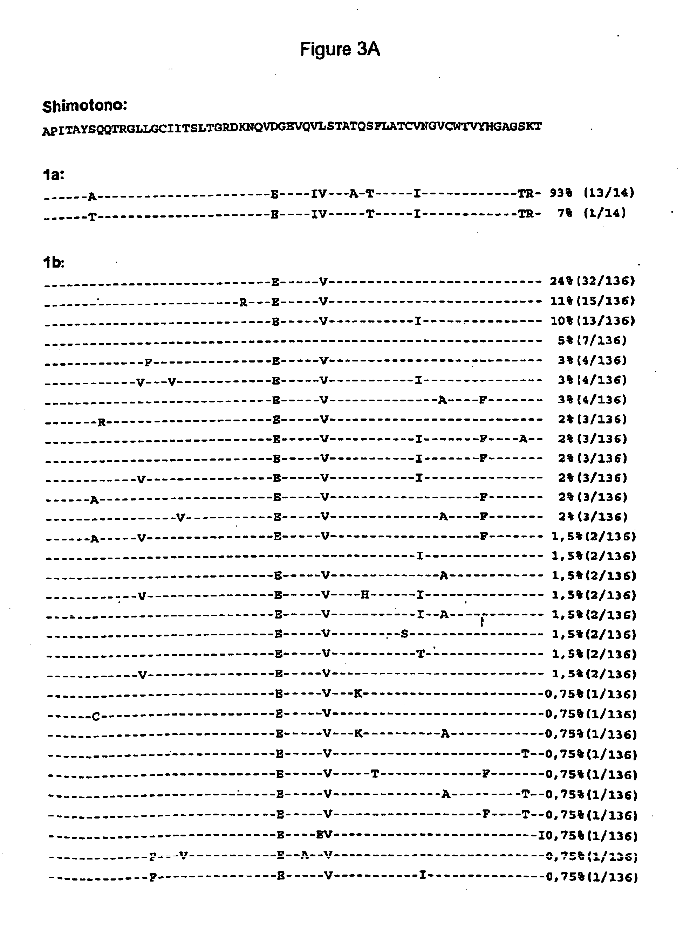

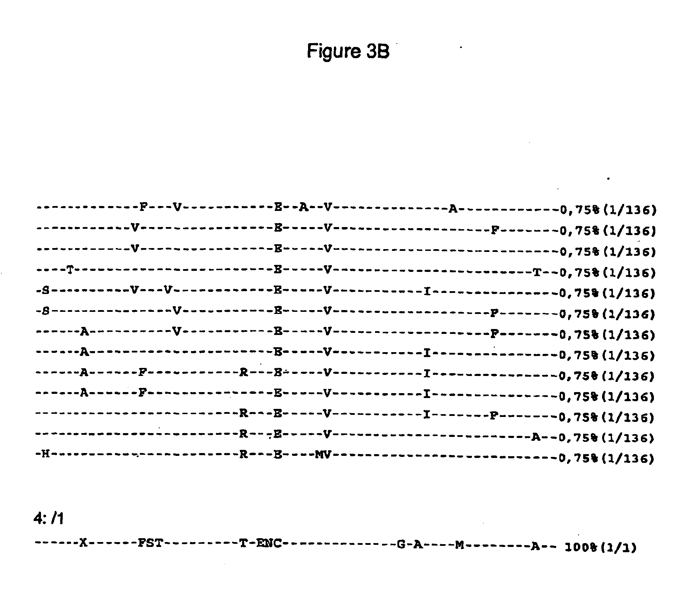

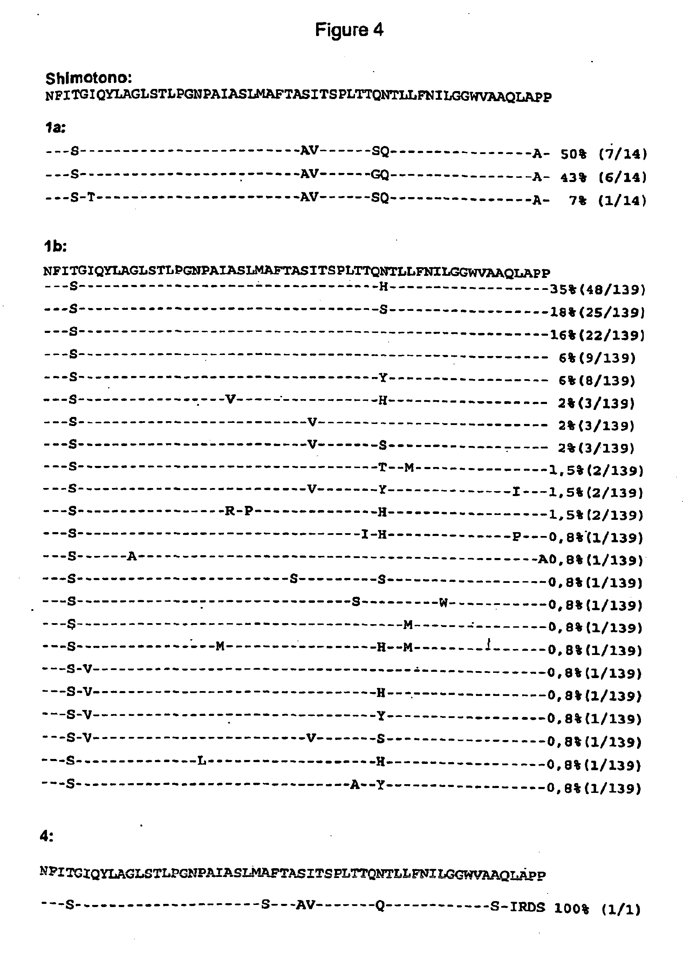

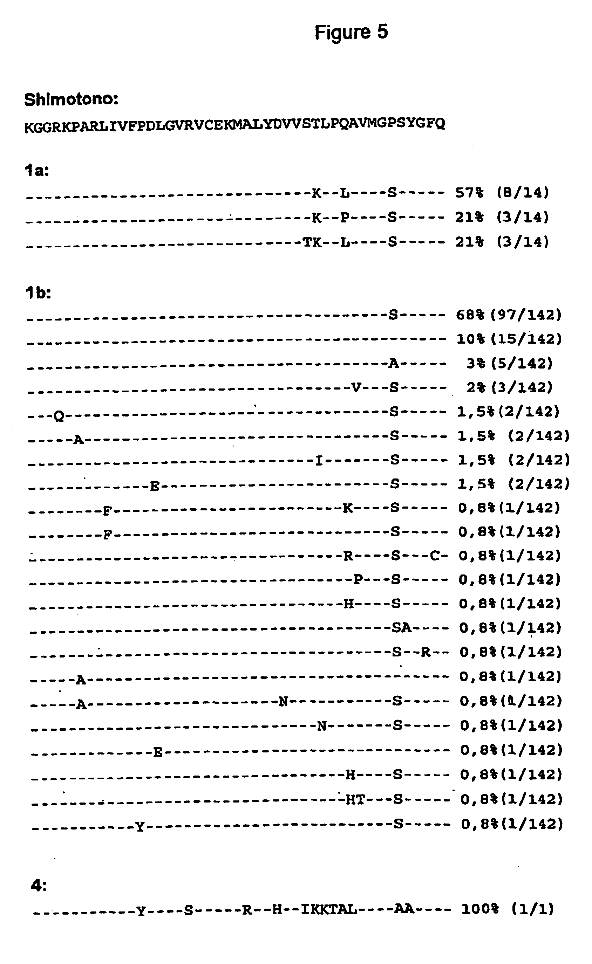

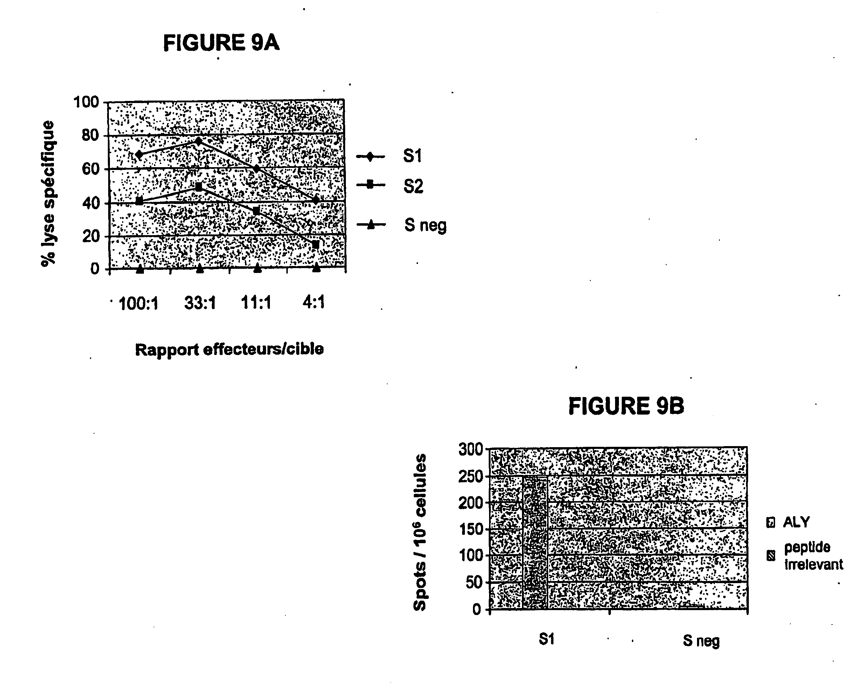

La présente invention sera mieux comprise à l'aide des exemples suivants donnés uniquement à titre illustratif et non limitatif, ainsi qu'à l'aide des figures 1 à 14 annexées, sur lesquelles :
- la figure 1A représente la production moyenne d'interféron gamma pour 3 patients (Pt 1 à 3) infectés par des souches HCV de génotype 1 et présentant un HLA. A2+. Cette production est déterminée par le nombre de spots observés par million de cellules de sang de mises en contact avec les peptides FLAT (FLATCVNGV) et LLG (LLGCIITSL),
- la figure 1B représente la production moyenne d'interleukine 10 pour 3 patients (Pt 1 à 3) infectés par des souches HCV de génotype 1 et présentant un HLA. A2+. Cette production est déterminée par le nombre de spots observés par million de cellules de sang mises en contact avec les peptides FLAT et LLG,
- la figure 2 représente l'alignement des peptides A de l'invention,
- la figure 3 représente l'alignement des peptides B de l'invention,
- la figure 4 représente l'alignement des peptides C de l'invention et
- la figure 5 représente l'alignement des peptides D de l'invention.
- la figure 6 concerne le peptide A et montre un graphe donnant le pourcentage de lyse spécifique de splénocytes de souris transgéniques HTA-A2 selon les conditions du test CTI, après immunisation des souris par le peptide A de l'invention,
- les figures 7A et 7B concernent le peptide B et montrent d'une part un graphe donnant le pourcentage de lyse spécifique de splénocytes de souris transgéniques HLA-A2 selon les conditions du test CTL après immunisation des souris par le peptide B de l'invention (figure 7A) et d'autre part un autre graphe donnant le nombre de cellules productrices d'interféron gamma révélées par la méthode ELISPOT issues des mêmes souris transgéniques et mises en contact avec l'épitope ATL de l'art antérieur contenu dans le peptide A (figure 7B),
- les figures 8A et 8B concernent le peptide C et montrent d'une part un graphe donnant le pourcentage de lyse spécifique de splénocytes de souris transgéniques HLA-A2 selon les conditions du test CTL après immunisation des souris par le peptide C de l'invention (figure 8A) et d'autre part un autre graphe donnant le nombre de cellules productrices d'interféron gamma révélées par la méthode FL1SYOT issues des mêmes souris transgéniques et mises en contact avec le peplidc C (figure 8B),
- les figures: 9A et 9B concernent le peptide D et montrent d'une part un graphe donnant le pourcentage de lyse spécifique de splénocytes de souris transgéniques HLA-A2 selon les conditions du test CTL après immunisation des souris par le peptide D de l'invention (figure 9A) et d'autre pan un autre graphe donnant le nombre de cellules productrices d'interféron gamma révélées par la méthode ELISPOT issues des mêmes souris transgéniques et mises en contact avec l'épitope ALY de l'art antérieur contenu dans le peptide D (figure 9C), et

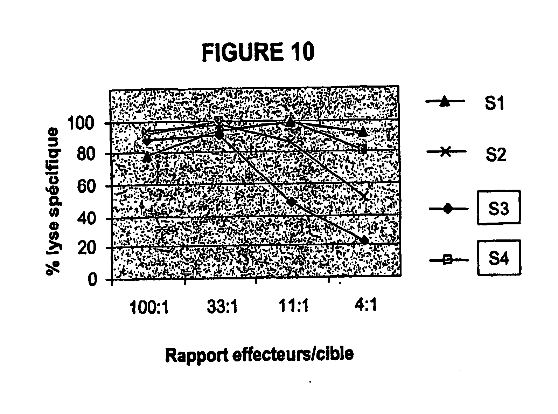

- la figure 10 est un graphe donnant le pourcentage de lyse spécifique de splénocytes de souris transgéniques HLA-A2 selon les conditions du test CTL après immunisation des souris par le peptide D de l'invention ou après immunisation avec l'épitope ALY,

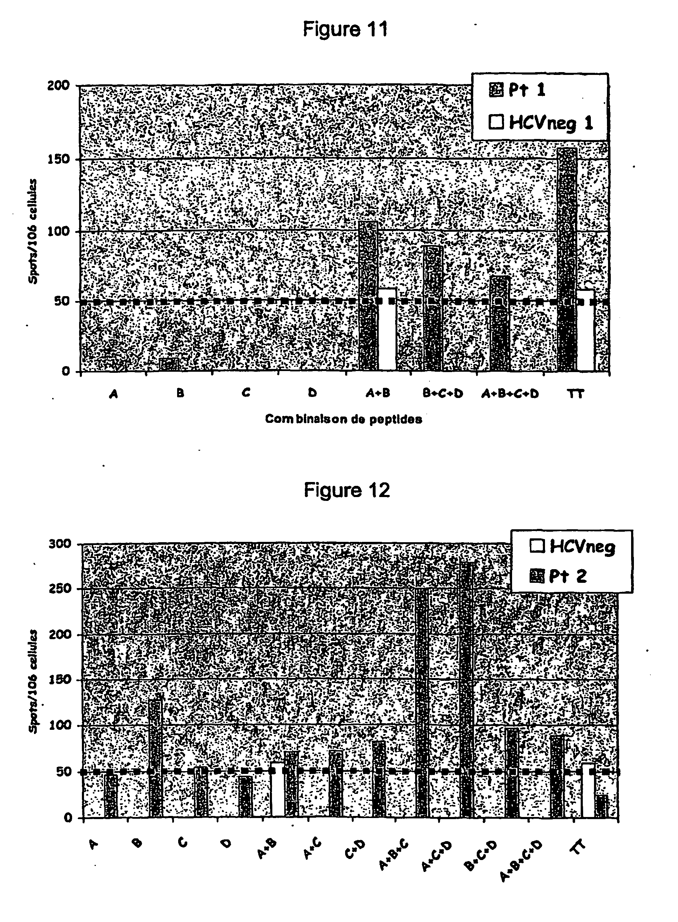

- la figure 11 est un graphe représentant le nombre de cellules productrices d'interféron gamma révélées par la méthode ELISPOT issues d'un patient HCV séropositif et mises en contact avec les peptides A, B, C et D, ainsi qu'avec les compositions A/B, B/C/D et A/B/C/D,
- la figure 12 est un graphe représentant le nombre de cellules productrices d'interféron gamma révélées par la méthode FFISPOT issues d'un autre patient HCV séropositif et mises en contact avec les peptides A, B, C et D, ainsi qu'avec les compositions A/B. A/C, C/D, A/B/C, A/C/D, B/C/D et A/B/C/D,

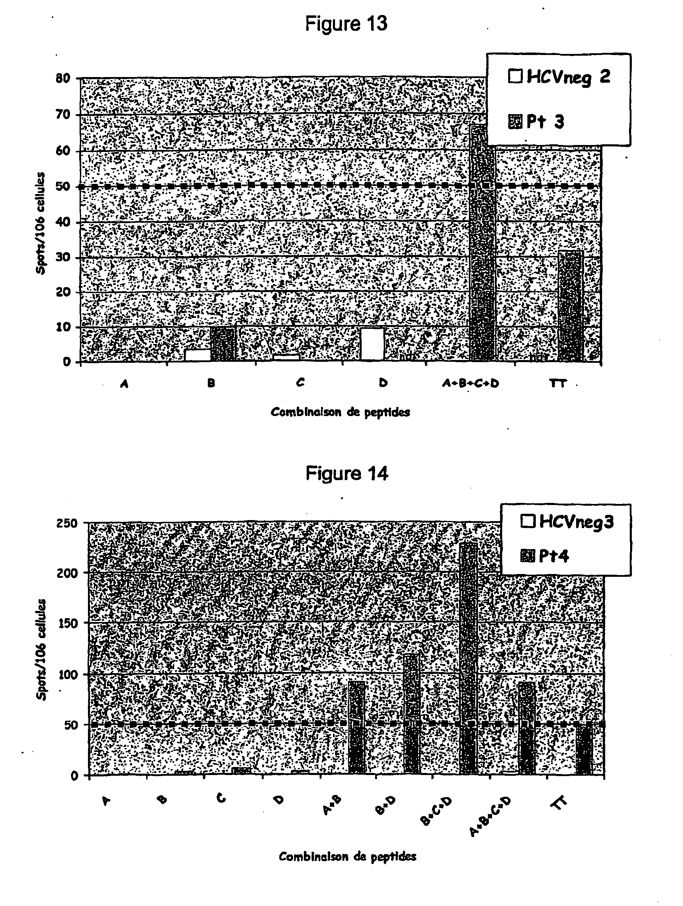

- la figure 13 est un graphe représentant le nombre de cellules productrices d'interféron gamma révélées par la méthode ELISPOT issues d'un autre patient HCV séropositif et mises en contact avec les peptides A, B, C et D, ainsi qu'avec la composition A/B/C/D,
- la figure 14 est un graphe représentant le nombre de cellules productrices d'interféron gamma révélées par la méthode ELISPOT issues d'un autre patient HCV séropositif et mises en contact avec les peptides A, B, C et D, ainsi qu'avec les compositions A/B, B/D, B/C/D et A/B/C/D,

### Exemple 1 : Absence de corrélation entre le score de fixation et le pouvoir immunogène d'un peptide-épitope

### 1a : Test sur les cellules humaines

On a testé la capacité de cellules du sang de 3 patients infectés par le virus de l'hépatite C de génotype 1 et présentant un HLA, de type HLA-A2+, à produire les cytokines interféron gamma et interlcukine 10. en réponse à deux peptides prédits par des logiciels développés par bioMérieux (Centre d'Immunologie de Pierre Fabre) pour se fixer sur la molécule HLA-A2.

Le dosage de ces cytokines reflète la capacité des peptides à induire des réponses immunes à médiation cellulaire, soit du type 1 (Interféron gamma), soit du type 2 (interleukine 10)

Pour ce faire, on a utilisé les épitopes FLAT (FLATCVNGV) et LLG (LLGCIITSL) inclus dans le peptide B de l'invention, lesquels ont des scores de fixation équivalents (694 pour FLAT et 619 pour LLG).

On a incubé, dans une plaque ELISPOT (Human IL10 Elispot Set, San Diego, California, USA) sur laquelle avaient été préalablement fixés des anticorps biotinylés spécifiques de l'interféron gamma (kit BD) ou de l'interleukine 10 (purified mouse anti-human IFN-γ monoclonal antibody, BD, n°554548) des cellules mononucleées de sang de 3 patients infectés (200 000 cellules par puits) en présence des peptides FLAT et LLG.

Après 48h d'incubation à 37°C, période pendant laquelle les cellules spécifiques des peptides vont produire localement des cytokines qui vont se fixer sur les anticorps spécifiques, on a ajouté des anticorps anti-interféron gamma ou anti-interleukine 10, de la phosphatase alcaline couplée à de l'avidine et le substrat de la phosphatase alcaline (NBT/BCIP).

On a compté les spots bleu violet, qui représentent chaque cellule productrice d'interféron gamma ou d'interleukine 10, à l'aide du lecteur ELISPOT automatisé de Zeiss (KS Elispot) ct on n'a considéré comme positif que lorsque le nombre de spot par puits était supérieur à 20.

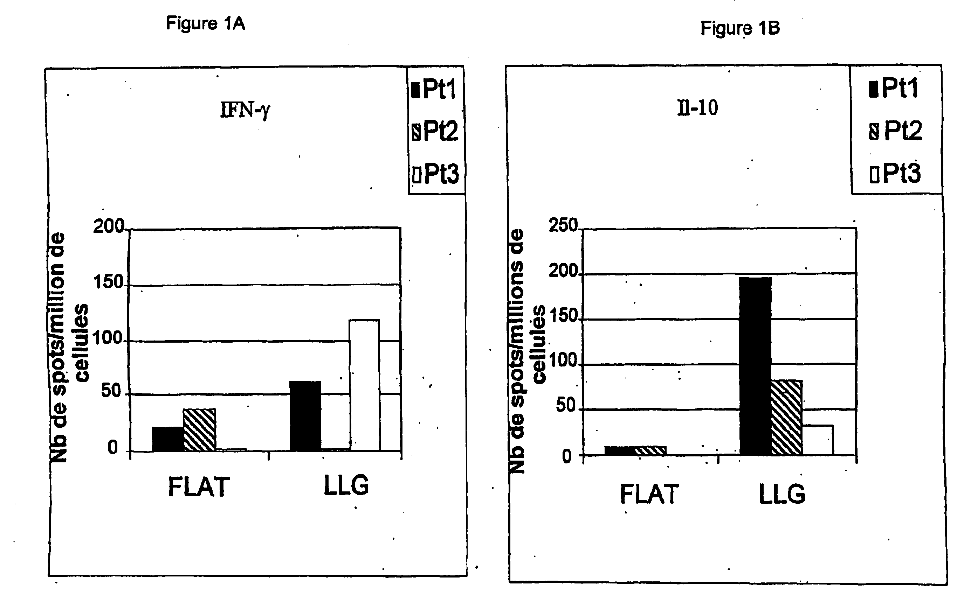

Les résultats obtenus sont indiqués sur la figure 1, sur laquelle Pt représente patient. Ils montrent que, malgré un score de fixation équivalent sur la molécule HLA-A2, ces deux peptides n'induisent pas de réponses équivalentes.

En effet, le peptide FLAT induit peu, voire pas de production des deux cytokines chez les trois patients, tandis que le peptide LLG induit une production significative d'interféron gamma chez 2 des 3 patients et d'interleukine 10 chez les trois patients.

Les résultats obtenus montrent également que les peptides de l'invention, ainsi que leur association n'étaient pas évidents au vu des enseignements de l'état de la technique.

### 1b : Test sur des cellules murines

On a immunisé des souris transgéniques pour la molécule III.A.A2.1 et dénuées des molécules de classe I murines (Pascolo S., et al. (1997), J. Exp Med., 185, 2043-2051) avec les épitopes FLL et ILA prédits comme étant des épitopes de classe I restreints par la molécule BLA.A2.1. Le peptide FLL a un score moyen (515) alors que le peptide ILA présente un score très haut (893).

Pour ce faire, on a immunisé les souris au niveau de la base de leur queue avec un mélange peptidique contenant l'un des épitopes ci-dessus (60 µM) et le peptide T-helper (Lone, Y.C. et al., (1998), J.Immunother. 21 :283-294) (60 µM) émulsifié dans un adjuvant incomplet de Freund (Sigma St Louis, MO). Les souris ont reçu deux injections à deux semaines d'intervalle et on a effectué l'analyse des réponses immunitaires deux semaines après la dernière immunisation comme indiqué dans l'exemple 1 (Elispot).

Les résultats sont indiqués dans le tableau 1 ci-dessous.

**Tableau 1 :**

| **Epitope** | **Antigéne viral** | **Score** | **Nbre de spots/10⁶ cellules^{1*}** |
|---|---|---|---|
| FLLLADARV | E2 | 515 | 333 ; 215 ; 622 |
| ILAGYAGAGV | NS4 | 893 | 5 ; 2 ; 0 |

| | | | |
|---|---|---|---|
| ¹ : Cellules productrices d'interféron gamma | | | |
| * : Nombre de spots de trois souris individuelles testées. | | | |

Ces résultats montrent que le score attribué à un peptide ne reflète pas son immunogénicité.

### Exemple 2: Mise en évidence de l'immunogénicité des peptides/compositions peptidiques/épitopes de l'invention chez la souris

On a immunisé des souris transgéniques HLA-A2.1 soit avec le peptide A de séquence SEQ ID N°3, soit avec le peptide B de séquence SEQ ID N°147, soit avec le peptide C de séquence SEQ ID N°129. soit avec le peptide D de séquence SEQ ID N°176, soit avec l'épitope B' de séquence SEQ ID N°214 et soit avec les épitopes ATL et ALY de l'art antérieur contenus respectivement dans les peptides A et D (ATLGFGAYM, acides aminés 1260-1268 de la polyprotrinc virale et ALYDV'VSTL, acides aminés 2594-2602 de la polyprotéine virale).

Pour ce làirc, on a procédé à 2 injections sous-cutanées à la base de la queue par souris, à 15 jours d'intervalle, avec un mélange contenant 18 nmoles de peptide de l'invention et 18 nmoles du peptide helpeur du core d'HBV dans de l'adjwant incomplet de Freund (lFA, Brinster et al., Hepatology 2001) ou bien contenant 60 nmoles d'épitope de l'invention et 60 nmoles du peptide helpeur dans de l'IFA. Les souris témoin n'ont reçu que le peptide helpeur dans l'IFA.

Quinze jours après la 2^{ème} injection, on a analysé la réponse cellulaire en isolant les cellules de la rate (splénocyles) des souris et on a effectué un test CTI, et un test ELISPOT comme suit :

Pour le test CTL, on a cultivé ces splénocytes en plaque 24 puits en présence de 5 µM du peptide ou de l'épitope d'intérêt et de 10 U d'interleukine 2 recombinante murine (Brinster et al., Hepatology 2001) par ml dans du milieu minimum essentiel alpha (αMEM) pendant 5 jours. Au 5^{éme} jour, on a effectué l'étape de restimulation qui consiste à rajouter aux splénacytes en culture des splénocytes de souris naïves en présence du peptide ou de l'épitope d'intérêt pendant 2 jours. Au 7^{ème} jour, on a réalisé le test CTI, en lui-même qui consiste à mettre en présence les splénocytes des souris immunisées après les 7 jours de culture (cellules effectrices) et des cellules EL4 S3-Rob HDD chargées avec 10µM du peptide ou de l'épitope d'intérêt marquées au Cr⁵¹ (cellules cibles). On a déterminé l'activité cytotoxique spécifique des cellules effectrices par la mesure, après 4 h d'incubation avec les cellules cibles, du Cr⁵¹ libéré suite à la lyse des cellules cibles en utilisant un appareil de comptage γ-Cobra II (Packard, Rungis, France). On a déterminé la libération spontanée et maximale à partir de puits contenant soit du milieu seul, soit du tampon de lyse (HCI IN). On a calculé le pourcentage spécifique de cytotoxicité par la formule:
(libération dans l'essai - libération spontanée)/(libération maximale - libération spontanée) ×100. On a déterminé la lyse spécifique de peptide ou d'épitope par la différence entre le pourcentage de lyse spécifique obtenu en présence ou en l'absence du peptide ou de l'épitope.

On a effectué le test ELISPOT en cultivant les spténocytes pendant 48 h dans des plaques 96 puits Multiscreen (Millipore) préalablement « coatées » avec de l'anticorps anti-interféron γ (IFNγ) (10µg/ml final). On a mis en culture les splénocytes en présence de 10µM de peptide ou d'épitope d'intérêt et de 10 U. d'interleukine 2 recombinante murine par ml dans du αMEM. Pour le contrôle positif, on a Cultivé les splénocytes en présence de concanavaline A (5 µg/ml). Pour le contrôle négatif, on a cultivé les splénocytes soit en présence d'un peptide non spécifique appartenant à la protéine de capside du VHC, de séquence DLMGYIPLV (également appelé peptide irrelevant), soit en milieu seul sans peptide ou épitope d'intérêt. On a lavé les puits à trois reprises, respectivement avec du PBS- Tween 0,05% puis du PBS, opération suivie d'une incubation de 2 h avec des anticorps anti-IFNγ de souris biotinylés. Après lavage, on a incubé les puits pendant 1 h avec un conjugué streptavidine-peroxydase de raifort et on a révélé l'activité enzymatique par dégradation du substrat AEC (aminoethylcarbazole). Les spots obtenus ont été comptés grâce à un lecteur ELISpot Zeiss (microscope Zeiss couplé au logiciel KS-ELISpot).

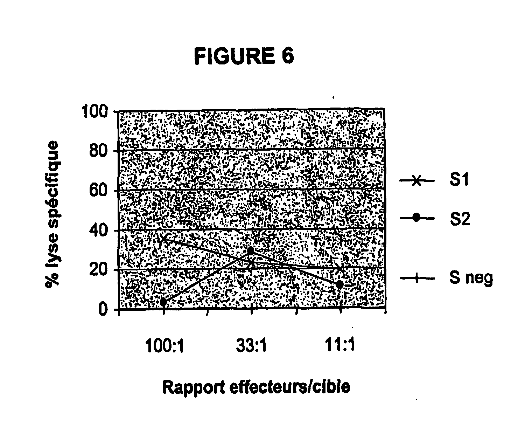

Les résutlats des tests CTL sont données sur les figures 6, 7A à 9A et 10 où S1 est souris 1, S2 est souris 2, S3 est souris 3, S4 est souris 4 et S neg est la souris témoin et où :
- la figure 6 donne le pourcentage de lyse spécifique, en fonction du rapport effecteurs/cible, après injection du peptide A en prenant pour cible l'épitope ATL,

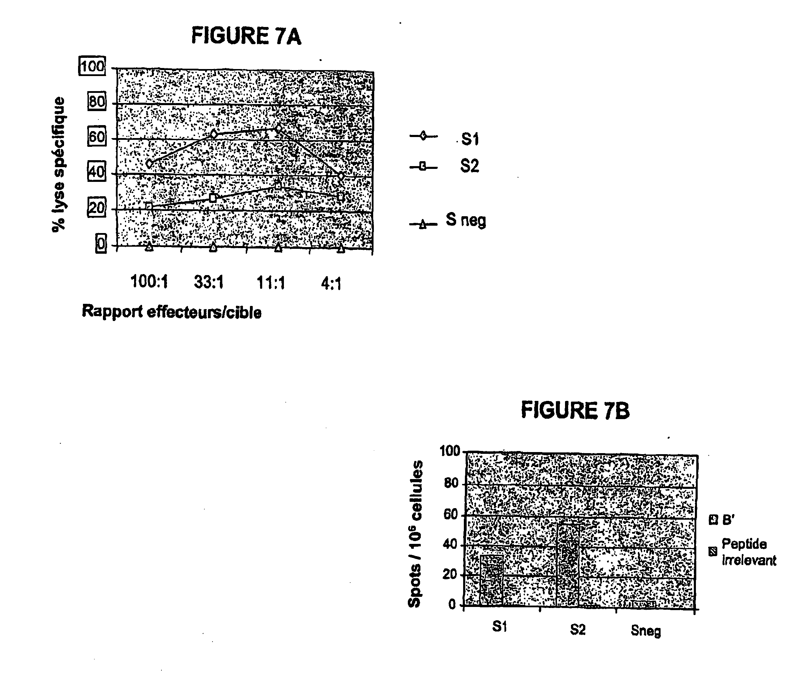

- la figure 7A donne le pourcentage de lyse spécifique, en fonction du rapport effecteurs/cible, après injection du peptide B en prenant pour cible l'épitope B',

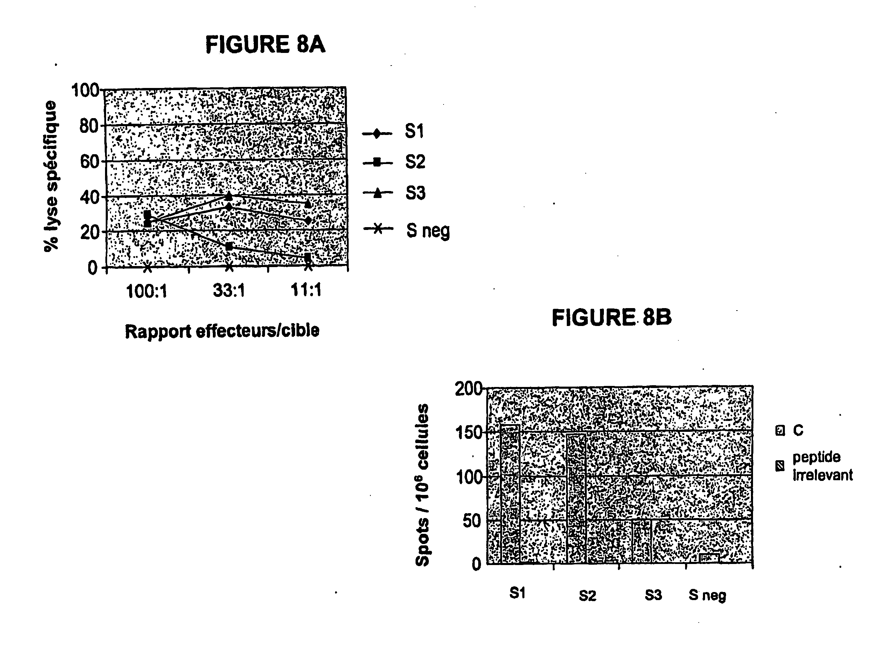

- la figure 8A donne le pourcentage de lyse spécifique, en fonction du rapport effecteurs/cible, après injection du peptide C en prenant pour cible le peptide C,
- la figure 9A donne le pourcentage de lyse spécifique, en fonction du rapport effecteur/cible, après injection du peptide D en prenant pour cible l'épitope ALY, et
- la figure 10 donne le pourcentage de lyse spécifique, en fonction du rapport effecteurs/cible, après injection du peptide D en prenant pour cible l'épitope ALY (souris S1 et S3) ou bien de l'épitope ALY en prenant pour cible l'épitope ALY (souris S3 et S4).

Les résultats tels que montrés dans les figures 6 à 9 montrent que l'injection de chaque peptide induit une réponse cytotoxique contre les épitopes correspondants de sorte que :
(i) tant lesdits peptides que lesdits épitopes ont un pouvoir immunogénique, et
(ii) les peptides de l'invention sont capables d'induire des réponses immunitaires spécifiques d'épitopes présents dans l'infection naturelle.

Il est à noter que la figure 10 montre que le peptide D et l'épitope ont des efficacités de lyse identique. Il ressort donc de cette expérience, au vu des quantités de peptide et d'épitopcs utilisées (injection de 18 nmoles du peptide D (souris S1 et S2) et injection de 60 nmoles de l'épitope ALY (souris S3 et S4), en prenant pour cible l'épitope ALY,), que les peptides de l'invention ont pour avantage qu'ils sont immunogènes à des doses plus faibles que les épitopes qu'ils contiennent.

On a également répété le test CTL en injectant la composition ABCD ou le peptide B en prenant pour cible l'épitope B' et en injectant la composition ABCD ou le peptide C en prenant pour cible le peptide C. Les résultats sont indiqués dans le tableau 3 ci-dessous.

**Tableau 3**

| | | | Rapport effecteurs/cibles | |
|---|---|---|---|---|
| | | | 100:1 | 33:1 |
| | | S1 | 55 | 52 |
| Injection ABCD | | S2 | 32 | 55 |
| | Cible B' | S3 | 53 | 65 |
| Injection B | | S1 | 46 | 63 |
| | | S2 | 22 | 27 |
| Injection ABCD | | S1 | 52 | 69 |
| | Cible C | S2 | 1 | 67 |
| Injection C | | S1 | 2 | 9 |
| | | S2 | 0 | 0 |

Les résultats dans le tableau ci-dessus mettent en évidence que l'injection de la combinaison des 4 peptides A, B, C et D induit une réponse cytotoxique plus efficace que l'injection des peptides seuls.

Les résultats des tests ELISPOT sont indiqués sur les figures 7B à 9B où S1, S2 S3 et S neg ont les mêmes définitions que précédemment et où :
- la figure 7B donne le nombre de spots par rapport à 10⁶ cellules pour des souris ayant reçu le peptide B par rapport à la cible épitope B', ainsi que le nombre de spots pour le peptide irrelevant.
- la figure 8B donne le nombre de spots par rapport à 10⁶ cellules pour des souris ayant reçu le peptide C par rapport à la cible peptide C, ainsi que le nombre de spots pour le peptide irrelevant.
- la figure 9B donne le nombre de spots par rapport à 10⁶ cellules pour des souris ayant reçu le peptide D par rapport à la cible épitope ALY, ainsi que le nombre de spots pour le peptide irrelevant.
Les résultats indiqués sur les figures 7B à 9B confirment le bon pouvoir immunogène des peptides et épitopes de l'invention.

### Exemple 3: Mise en évidence de l'immunogénicité des peptides/compositions peptidiques de l'invention chez l'homme

On a purifié sur gradient de Ficoll les cellules mononuclées du sang périphérique de quatre patients injectés chroniquement par le virus de l'hépatite C. On a pré-incubé deux cents milles cellules à 37°C, 5% de CO₂, toute la nuit dans des tubes en polypropylène en présence des peptides A à D, ayant les séquences en acides aminés telles que définies dans l'exemple 2 ci-dessus, et de leurs compositions, dans du milieu de culture composé de RPMI₁₆₄₀ (Invitrogen Life technology, Cergy Pontoise, France) supplémenté avec 2mM de L-Glutamine (Invitrogen Life Technology), 50 UI/ml de pénicilline (Invitrogen Life Technology), 50 µg/ml de streptomycine (Invitrogen Life Technology) et 10% de sérum de veau foetal (Hyclone, Logan, Utah, USA) selon le tableau 4 suivant :

**Tableau 4**

| Patient | Génotype | Statut clinique | Peptides/compositions peptidiques |
|---|---|---|---|
| 1 | 1b | Non traité | A, B, C, D, AB, BCD, ABCD |
| 2 | 1 | Non répondeur à la ribaviridine M12 | A, B, C, D, AB, AC, CD, ABC, ACD, BCD, ABCD |
| 3 | 4f | Non traité | A, B, C, D, ABCD |
| 4 | 1 | Non répondeurà l'IFNγ + ribavirine M6 | A, B, C, D, AB, BD, BCD, ABCD |

Les peptides, seuls ou en association, étaient présents selon les concentrations suivantes : A à 10 µM, B à 5µM, C à 1µM et D à µM

On a ensuite transféré les cellules dans une plaque ELISPOT en PVDF (poly(fluorure de vinylidene)) préalablement revêtue avec des anticorps anti-IFNγ selon les recommandations du fabricant (Diaclone, Besançon, France) et on les a incubées pendant 24 h supplémentaires à 37°C, 5% de CO₂. Comme précédemment, on recherche ici les cellules productrices de cytokines qui vont former des spots bleus qui sont révélés, après incubation séquentielle avec un anticorps anti- IFNγ biotinylé et de la PAL couplée à la streptavidine, par dégradation du substrat BCIP/NBT (sel de 5-bromo-4-chloro-3-indolylphosphate p-toluidine/chlorure de tétrazolium nitrobleu) et qui sont comptés à l'aide du lecteur ELISPOT Zeiss.

A titre de contrôle positif, on a remplacé les peptides/compositions peptidiques par de la toxine tétanique (TT) à 1 µg/ml.

On a répété ce mode opératoire à partir de cellules de patients HCV-séronégatifs.

Les résultats sont indiqués sur les figures 11 à 14 donnant le nombre de spots par million de cellules en fonction des peptides et de leurs combinaisons (moyenne des triplicates) obtenus avec les patients 1 à 4, respectivement, et sur lesquelles la ligne pointillée représente le seuil de significativité du test (50 spots), Pt est patient et HCVneg est un patient HCV scronégatif.

Les résultats mettent en évidence que les compositions peptidiques de l'invention présentent un fort pouvoir immunogène.

## Revendications

1. Composition peptidique, **caractérisée en ce qu'**elle comprend au moins deux composés choisis parmi :
- un peptide A ayant au moins la séquence en acides aminés SEQ ID N°1 suivante :
X₁AX₂QGYKVX₃VLNPSVX₄ATLX₅FGX₆YMSKAX₇GX₈,
dans laquelle X₁ est Y ou H, X₂ est A, G ou T, X₃ est R ou L, X₄ est A ou T, X₅ est G ou S, X₆ est A, T ou V, X₇ est H ou Y et X₈ est I, T, M ou V,
et ayant au plus les 46 acides aminés tels que décrits dans la séquence SEQ ID N°2 suivante:
SX₉X₁₀VPX₁₁X₁₂X₁AX₂QGYKVX₃VLNPSVX₄ATLX₅FGX₆YMSKAX₇GX₈X₁₃PX₁₄ X₁₅X₁₆X₁₇GV,
dans laquelle X₁ à X₈ sont tels que définis dans la revendication 1, et X₉ est T ou N, X₁₀ est K ou R, X₁₁ est A ou V, X₁₂ est A ou E, X₁₃ est E ou D, X₁₄ est N ou S, X₁₅ est I, L ou V, X₁₆ est R ou S et X₁₇ est T ou S,
- un peptide B ayant au moins la séquence en acide aminé SEQ ID N°45 suivante :
GX₁₈X₁₉X₂₀X₂₁X₂₂X₂₃TSLTGRDX₂₄NX₂₅X₂₆X₂₇GEX₂₈QX₂₉X₃₀STAX₃₁X₃₂X₃₃FLX₃₄ X₃₅X₃₆X₃₇NGX₃₈X₃₉WTVX₄₀
dans laquelle X₁₈ est L ou V, X₁₉ est L ou F, X₂₀ est G ou S, X₂₁ est C ou T, X₂₂ est I ou V, X₂₃ est I ou V, X₂₄ est K, R ou T, X₂₅ est Q ou E, X₂₆ est V ou N, X₂₇ est D, E ou C, X₂₈ est V ou A, X₂₉ est V, I, E ou M, X₃₀ est L ou V, X₃₁ est T, K ou A, X₃₂ est Q ou H, X₃₃ est S ou T, X₃₄ est A ou G, X₃₅ est T ou S, X₃₆ est C ou A, X₃₇ est V, I ou T, X₃₈ est V ou A, X₃₉ est C ou M et X₄₀ est Y ou F,
et ayant au plus les 63 acides aminés tels que décrits dans la séquence SEQ ID N°46 suivante :
AX₄₁ITX₄₂YX₄₃X₄₄QTRGX₁₈X₁₉X₂₀X₂₁X₂₂X₂₃TSLTGRDX₂₄NX₂₅X₂₆X₂₇GEX₂₈QX₂₉ X₃₀STAX₃₁X₃₂X₃₃FLX₃₄X₃₅X₃₆X₃₇NGX₃₈X₃₉WTVX₄₀HGAGX₄₅X₄₆X₄₇
dans laquelle X₁₈ à X₄₀ sont tels que définis dans la revendication 1 et X₄₁ est P, S ou H, X₄₂ est A ou T, X₄₃ est S, A, T ou C, X₄₄ est Q ou R, X₄₅ est S, T ou A, X₄₆ est K ou R et X₄₇ est T ou I,
- un peptide C ayant au moins la séquence en acides aminés SEQ ID N°127 suivante :
SX₄₇MX₄₈FTX₄₉X₅₀X₅₁TSPLX₅₂X₅₃X₅₄X₅₅TLX₅₆FNIX₅₇GGWVAX₅₈QX₅₉
dans laquelle X₄₇ est L ou P, X₄₈ est A ou S, X₄₉ est A ou S, X₅₀ est A ou S, X₅₁ est I ou V, X₅₂ est T, S ou A, X₅₃ est T ou I, X₅₄ est Q, S ou G, X₅₅ est N, Q, H, S, Y ou T, X₅₆ est L ou M,X₅₇ est L ou W, X₅₈ est A ou S et X₅₉ est L, P ou I,
et ayant au plus les 57 acides aminés tels que décrits dans la séquence SEQ ID N°128 suivante :
NFIX₆₀GX₆₁QYLAX₆₂LSTLPGNX₆₃AX₆₄X₆₅SX₄₇MX₄₈FTX₄₉X₅₀X₅₁TSPLX₅₂X₅₃X₅₄ X₅₅TLX₅₆FNIX₅₇GGWVAX₅₈QX₅₉X₆₆X₆₇X₆₈
dans laquelle X₄₇ à X₅₉ sont tels que définis dans la revendication 1 et X₆₀ est T ou S, X₆₁ est I, T ou V, X₆₂ est G ou A, X₆₃ est P ou L, X₆₄ est I ou M, X₆₅ est A, V ou R, X₆₆ est A ou R, X₆₇ est P, A ou D et X₆₈ est P, A ou S,
- un peptide D ayant au moins la séquence en acides aminés SEQ ID N°172 suivante :
X₆₉KX₇₀ARX₇₁IVX₇₂PX₇₃LGX₇₄RVCEKX₇₅ALX₇₆X₇₇VX₇₈X₇₉X₈₀X₈₁
dans laquelle X₆₉ est R ou Q, X₇₀ est P ou A, X₇₁ est L ou F, X₇₂ est F ou Y, X₇₃ est D ou E, X₇₄ est V ou S, X₇₅ est M ou R, X₇₆ est Y ou H, X₇₇, est D ou N, X₇₈ est V ou I, X₇₉ est S, T ou K, X₈₀ est T, K, I ou N et X₈₁ est L ou T,
et ayant au plus les 44 acides aminés tels que décrits dans la séquence SEQ ID N°175 suivante:
KGGX₆₉KX₇₀ARX₇₁IVX₇₂PX₇₃LGX₇₄RVCEKX₇₅ALX₇₆X₇₇VX₇₈X₇₉X₈₀X₈₁X₈₂X₈₃X₈₄ VMGX₈₅X₈₆YX₈₇X₈₈Q
dans laquelle X₆₉ à X₈₁ sont tels que définis dans la revendication 1 et X₈₂ est P ou A, X₈₃ est Q, L, H, R, K ou P, X₈₄ est A, T, V ou P, X₈₅ est P, S ou A, X₈₆ est S ou A, X₈₇ est G ou R et X₈₈ est F ou C,
- un épitope B' ayant la séquence SEQ ID N°213 suivante :
GX₁₈X₁₉X₂₀X₂₁X₂₂X₂₃TSL
dans laquelle X₁₈ est L ou V, X₁₉ est L ou F, X₂₀ est G ou S, X₂₁ est C ou T, X₂₂ est I ou V et X₂₃ est I ou V, et
- un épitope C' ayant la séquence SEQ ID N°221 suivante :
SPLX₅₂X₅₃X₅₄X₅₅TL
dans laquelle X₅₂ est T, S ou A, X₅₃ est T ou I, X₅₄ est Q, S ou G et X₅₅ est N, Q, H, S, Y ou T.

2. Composition peptidique selon la revendication 1, **caractérisée en ce qu'**elle comprend au moins deux peptides choisis parmi les peptides A, B, C, D.

3. Composition peptidique selon l'une quelconque des revendications 1 ou 2, **caractérisée en ce que** le peptide A est choisi parmi les peptides suivants :
- les peptides ayant au moins la séquence SEQ ID N°3 et au plus la séquence SEQ ID N°19,
- les peptides ayant au moins la séquence SEQ ID N°4 et au plus une séquence choisie parmi :
i) la séquence SEQ ID N°20,
ii) la séquence SEQ ID N°24,
iii) la séquence SEQ ID N°32 et
iv) la séquence SEQ ID N°34,
- les peptides ayant au moins la séquence SEQ ID N°5 et au plus une séquence choisie parmi :
i) la séquence SEQ ID N°21,
ii) la séquence SEQ ID N°28 et
iii) la séquence SEQ ID N°36,
- les peptides ayant au moins la séquence SEQ ID N°6 et au plus une séquence choisie parmi :
i) la séquence SEQ ID N°22,
ii) la séquence SEQ ID N°27 et
iii) la séquence SEQ ID N°41,
- les peptides ayant au moins la séquence SEQ ID N°7 et au plus une séquence choisie parmi :
i) la séquence SEQ ID N°23 et
ii) la séquence SEQ ID N°37,
- les peptides ayant au moins la séquence SEQ ID N°8 et au plus la séquence SEQ ID N°25,
- les peptides ayant au moins la séquence SEQ ID N°9 et au plus la séquence SEQ ID N°26,
- les peptides ayant au moins la séquence SEQ ID N°10 et au plus la séquence SEQ ID N°29,
- les peptides ayant au moins la séquence SEQ ID N°11 et au plus la séquence SEQ ID N°30,
- les peptides ayant au moins la séquence SEQ ID N°12 et au plus la séquence SEQ ID N°31,
- les peptides ayant au moins la séquence SEQ ID N°13 et au plus la séquence SEQ ID N°33,
- les peptides ayant au moins la séquence SEQ ID N°14 et au plus une séquence choisie parmi :
i) la séquence SEQ ID N°35 et
ii) la séquence SEQ ID N°39,
- les peptides ayant au moins la séquence SEQ ID N°15 et au plus la séquence SEQ ID N°38,
- les peptides ayant au moins la séquence SEQ ID N°16 et au plus une séquence choisie parmi :
i) la séquence SEQ ID N°40 et
ii) la séquence SEQ ID N°42,
- les peptides ayant au moins la séquence SEQ ID N°17 et au plus la séquence SEQ ID N°43, et
- les peptides ayant au moins la séquence SEQ ID N°18 et au plus la séquence SEQ ID N°44.

4. Composition peptidique selon la revendication 3, **caractérisée en ce que** le peptide A est choisi parmi les peptides de séquences SEQ ID N°3 à 18.

5. Composition peptidique selon la revendication 4, **caractérisée en ce que** le peptide A a la séquence SEQ ID N°3.

6. Composition peptidique selon l'une quelconque des revendications 1 à 5, **caractérisée en ce que** le peptide B est choisi parmi :
- les peptides ayant au moins la séquence SEQ ID N°47 et au plus la séquence SEQ ID N°81,
- les peptides ayant au moins la séquence SEQ ID N°48 et au plus la séquence SEQ ID N°82,
- les peptides ayant au moins la séquence SEQ ID N°49 et au plus la séquence SEQ ID N°83,
- les peptides ayant au moins la séquence SEQ ID N°50 et au plus une séquence choisie parmi:
i) la séquence SEQ ID N°84,
ii) la séquence SEQ ID N°90,
iii) la séquence SEQ ID N°105,
iv) la séquence SEQ ID N°107 et
v) la séquence SEQ ID N°116,
- les peptides ayant au moins la séquence SEQ ID N°51 et au plus une séquence choisie parmi :
i) la séquence SEQ ID N°85 et
ii) la séquence SEQ ID N°124,
- les peptides ayant au moins la séquence SEQ ID N°52 et au plus une séquence choisie parmi :
i) la séquence SEQ ID N°86 et
ii) la séquence SEQ ID N°120,
- les peptides ayant au moins la séquence SEQ ID N°53 et au plus la séquence SEQ ID N°87,
- les peptides ayant au moins la séquence SEQ ID N°54 et au plus une séquence choisie parmi :
i) la séquence SEQ ID N°88 et
ii) la séquence SEQ ID N°117,
- les peptides ayant au moins la séquence SEQ ID N°55 et au plus la séquence SEQ ID N°89,
- les peptides ayant au moins la séquence SEQ ID N°56 et au plus une séquence choisie parmi :
i) la séquence SEQ ID N°91 et
ii) la séquence SEQ ID N°92,
- les peptides ayant au moins la séquence SEQ ID N°57 et au plus la séquence SEQ ID N°93,
- les peptides ayant au moins la séquence SEQ ID N°58 et au plus une séquence choisie parmi :
i) la séquence SEQ ID N°94 et
ii) la séquence SEQ ID N°110,
- les peptides ayant au moins la séquence SEQ ID N°59 et au plus la séquence SEQ ID N°95,
- les peptides ayant au moins la séquence SEQ ID N°60 et au plus une séquence choisie parmi :
i) la séquence SEQ ID N°96 et
ii) la séquence SEQ ID N°115,
- les peptides ayant au moins la séquence SEQ ID N°61 et au plus la séquence SEQ ID N°97,
- les peptides ayant au moins la séquence SEQ ID N°62 et au plus une séquence choisie parmi :
i) la séquence SEQ ID N°98 et
ii) la séquence SEQ ID N°109,
- les peptides ayant au moins la séquence SEQ ID N°63 et au plus la séquence SEQ ID N°99,
- les peptides ayant au moins la séquence SEQ ID N°64 et au plus la séquence SEQ ID N° 100,
- les peptides ayant au moins la séquence SEQ ID N°65 et au plus la séquence SEQ ID N°101,
- les peptides ayant au moins la séquence SEQ ID N°66 et au plus la séquence SEQ ID N°102,
- les peptides ayant au moins la séquence SEQ ID N°67 et au plus la séquence SEQ ID N°103,
- les peptides ayant au moins la séquence SEQ ID N°68 et au plus la séquence SEQ ID N°104,
- les peptides ayant au moins la séquence SEQ ID N°69 et au plus la séquence SEQ ID N°106,
- les peptides ayant au moins la séquence SEQ ID N°70 et au plus la séquence SEQ ID N°108,
- les peptides ayant au moins la séquence SEQ ID N°71 et au plus la séquence SEQ ID N°111,
- les peptides ayant au moins la séquence SEQ ID N°72 et au plus la séquence SEQ ID N°112,
- les peptides ayant au moins la séquence SEQ ID N°73 et au plus la séquence SEQ ID N°113,
- les peptides ayant au moins la séquence SEQ ID N°74 et au plus la séquence SEQ ID N°114,
- les peptides ayant au moins la séquence SEQ ID N°75 et au plus une séquence choisie parmi :
i) la séquence SEQ ID N°118 et
ii) la séquence SEQ ID N°119,
- les peptides ayant au moins la séquence SEQ ID N°76 et au plus la séquence SEQ ID N°121,
- les peptides ayant au moins la séquence SEQ ID N°77 et au plus la séquence SEQ ID N°122,
- les peptides ayant au moins la séquence SEQ ID N°78 et au plus la séquence SEQ ID N°123,
- les peptides ayant au moins la séquence SEQ ID N°79 et au plus la séquence SEQ ID N°125 et
- les peptides ayant au moins la séquence SEQ ID N°80 et au plus la séquence SEQ ID N°126.

7. Composition peptidique selon la revendication 6, **caractérisé en ce que** le peptide B est choisi parmi les peptides de séquences SEQ ID N°47 à 80.

8. Composition peptidique selon la revendication 7, **caractérisé en ce que** le peptide B a la séquence SEQ ID N°47.

9. Composition peptidique selon l'une quelconque des revendications 1 à 8, **caractérisée en ce que** le peptide C est choisi parmi :
- les peptides ayant au moins la séquence SEQ ID N°129 et au plus une séquence choisie parmi :
i) la séquence SEQ ID N°147,
ii) la séquence SEQ ID N°153,
iii) la séquence SEQ ID N°162 et
iv) la séquence SEQ ID N°167,
- les peptides ayant au moins la séquence SEQ ID N°130 et au plus une séquence choisie parmi :
i) la séquence SEQ ID N°148 et
ii) la séquence SEQ ID N°150,
- les peptides ayant au moins la séquence SEQ ID N°131 et au plus la séquence SEQ ID N°149,
- les peptides ayant au moins la séquence SEQ ID N°132 et au plus une séquence choisie parmi :
i) la séquence SEQ ID N°151,
ii) la séquence SEQ ID N°155,
iii) la séquence SEQ ID N°168 et
iv) la séquence SEQ ID N°171,
- les peptides ayant au moins la séquence SEQ ID N°133 et au plus la séquence SEQ ID N°152,
- les peptides ayant au moins la séquence SEQ ID N°134 et au plus une séquence choisie parmi :
i) la séquence SEQ ID N°154 et
ii) la séquence SEQ ID N°169,
- les peptides ayant au moins la séquence SEQ ID N°135 et au plus la séquence SEQ ID N°156,
- les peptides ayant au moins la séquence SEQ ID N°136 et au plus une séquence choisie parmi :
i) la séquence SEQ ID N°157 et
ii) la séquence SEQ ID N°170,
- les peptides ayant au moins la séquence SEQ ID N°137 et au plus la séquence SEQ ID N°158,
- les peptides ayant au moins la séquence SEQ ID N°138 et au plus la séquence SEQ ID N°159,
- les peptides ayant au moins la séquence SEQ ID N°139 et au plus la séquence SEQ ID N°160,
- les peptides ayant au moins la séquence SEQ ID N°140 et au plus la séquence SEQ ID N°161,
- les peptides ayant au moins la séquence SEQ ID N°141 et au plus la séquence SEQ ID N°163,
- les peptides ayant au moins la séquence SEQ ID N°142 et au plus la séquence SEQ ID N°164,
- les peptides ayant au moins la séquence SEQ ID N°143 et au plus la séquence SEQ ID N°165,
- les peptides ayant au moins la séquence SEQ ID N°144 et au plus la séquence SEQ ID N°166,
- les peptides ayant au moins la séquence SEQ ID N°145 et au plus la séquence SEQ ID N°172,
- les peptides ayant au moins la séquence SEQ ID N°146 et au plus la séquence SEQ ID N°173.

10. Composition peptidique selon la revendication 9, **caractérisé en ce que** le peptide C est choisi parmi les peptides de séquences SEQ ID N° 129 à 146.

11. Composition peptidique selon la revendication 10, **caractérisé en ce que** le peptide C a la séquence SEQ ID N°129.

12. Composition peptidique selon l'une quelconque des revendications 1 à 11, **caractérisée en ce que** le peptide D est choisi parmi :
- les peptides ayant au moins la séquence SEQ ID N°176 et au plus une séquence choisie parmi :
i) la séquence SEQ ID N°188,
ii) la séquence SEQ ID N°192,
iii) la séquence SEQ ID N°193,
iv) la séquence SEQ ID N°194,
v) la séquence SEQ ID N°201,
vi) la séquence SEQ ID N°202,
vii) la séquence SEQ ID N°203,
viii) la séquence SEQ ID N°204,
ix) la séquence SEQ ID N°205 et
x) la séquence SEQ ID N°210,
- les peptides ayant au moins la séquence SEQ ID N°177 et au plus une séquence choisie parmi :
i) la séquence SEQ ID N°189 et
ii) la séquence SEQ ID N°190,
- les peptides ayant au moins la séquence SEQ ID N°178 et au plus la séquence SEQ ID N°191,
- les peptides ayant au moins la séquence SEQ ID N°179 et au plus la séquence SEQ ID N°195,
- les peptides ayant au moins la séquence SEQ ID N°180 et au plus une séquence choisie parmi :
i) la séquence SEQ ID N°196 et
ii) la séquence SEQ ID N°206,
- les peptides ayant au moins la séquence SEQ ID N°181 et au plus la séquence SEQ ID N°197,
- les peptides ayant au moins la séquence SEQ ID N°182 et au plus une séquence choisie parmi :
i) la séquence SEQ ID N°198 et
ii) la séquence SEQ ID N°209,
- les peptides ayant au moins la séquence SEQ ID N°183 et au plus une séquence choisie parmi :
i) la séquence SEQ ID N°199 et
ii) la séquence SEQ ID N°200,
- les peptides ayant au moins la séquence SEQ ID N°184 et au plus la séquence SEQ ID N°207,
- les peptides ayant au moins la séquence SEQ ID N°185 et au plus la séquence SEQ ID N°208,
- les peptides ayant au moins la séquence SEQ ID N°186 et au plus la séquence SEQ ID N°211,
- les peptides ayant au moins la séquence SEQ ID N°187 et au plus la séquence SED ID N°212.

13. Composition peptidique selon la revendication 12, **caractérisé en ce que** le peptide D est choisi parmi les peptides de séquences SEQ ID N° 176 à 187.

14. Composition peptidique selon la revendication 13, **caractérisé en ce que** le peptide D a la séquence SEQ ID N°176.

15. Composition peptidique selon l'une quelconque des revendications 1 à 14, **caractérisée en ce qu'**elle comprend les deux peptides suivants :
i) le peptide A tel que défini dans l'une quelconque des revendications 1 et 3 à 5 et un autre peptide choisi parmi les peptides B à D tels que définis dans l'une quelconque des revendications 1 et 6 à 14, ou
ii) le peptide B tel que défini dans l'une quelconque des revendications 1 et 6 à 8 et un autre peptide choisi parmi les peptides A, C ou D tels que définis dans l'une quelconque des revendications 1 et 3 à 5 et 9 à 14, ou
iii) le peptide C tel que défini dans l'une quelconque des revendications 1 et 9 à 11 et un autre peptide choisi parmi les peptides A, B ou D tels que définis dans l'une quelconque des revendications 1 et 3 à 8 et 12 à 14, ou
iv) le peptide D tel que défini dans l'une quelconque des revendications 1 et 12 à 14 et un autre peptide choisi parmi les peptides A, B ou C tels que définis dans l'une quelconque des revendications 1 et 3 à 11.

16. Composition peptidique selon l'une quelconque des revendications 1 à 14, **caractérisée en ce qu'**elle comprend les trois peptides suivants :
i) le peptide A tel que défini dans l'une quelconque des revendications 1 et 3 à 5 et deux autres peptides choisis parmi les peptides B à D tels que définis dans l'une quelconque des revendications 1 et 6 à 14, ou
ii) le peptide B tel que défini dans l'une quelconque des revendications 1 et 6 à 8 et deux autres peptides choisis parmi les peptides A, C ou D tels que définis dans l'une quelconque des revendications 1 et 3 à 5 et 9 à 14, ou
iii) le peptide C tel que défini dans l'une quelconque des revendications 1 et 9 à 11 et deux autres peptides choisis parmi les peptides A, B ou D tels que définis dans l'une quelconque des revendications 1 et 3 à 8 et 12 à 14, ou
iv) le peptide D tel que défini dans l'une quelconque des revendications 1 et 12 à 14 et deux autres peptides choisis parmi les peptides A, B ou C tels que définis dans l'une quelconque des revendications 1 et 3 à 11.

17. Composition peptidique selon l'une quelconque des revendications 1 à 14, **caractérisée en ce qu'**elle comprend les quatre peptides A, B, C, D tels que définis dans l'une quelconque des revendications 1 et 3 à 14.

18. Peptide A ayant au moins la séquence en acides aminés SEQ ID N°1 et au plus la séquence SEQ ID N°2.

19. Peptide A selon la revendication 18, **caractérisé en ce qu'**il est choisi parmi :
- les peptides ayant au moins la séquence SEQ ID N°3 et au plus la séquence SEQ ID N°19,
- les peptides ayant au moins la séquence SEQ ID N°4 et au plus une séquence choisie parmi les séquences SEQ ID N°20, SEQ ID N°24, SEQ ID, N°32 et SEQ ID N°34,
- les peptides ayant au moins la séquence SEQ ID N°5 et au plus une séquence choisie parmi les séquences SEQ ID N°21, SEQ ID N°28 et SEQ ID N°36,
- les peptides ayant au moins la séquence SEQ ID N°6 et au plus une séquence choisie parmi les séquences SEQ ID N°22, SEQ ID N°27 et SEQ ID N°41,
- les peptides ayant au moins la séquence SEQ ID N°7 et au plus une séquence choisie parmi les séquences SEQ ID N°23 et SEQ ID N°37,
- les peptides ayant au moins la séquence SEQ ID N°8 et au plus la séquence SEQ ID N°25,
- les peptides ayant au moins la séquence SEQ ID N°9 et au plus la séquence SEQ ID N°26,
- les peptides ayant au moins la séquence SEQ ID N°10 et au plus la séquence SEQ ID N°29,
- les peptides ayant au moins la séquence SEQ ID N°11 et au plus la séquence SEQ ID N°30,
- les peptides ayant au moins la séquence SEQ ID N°12 et au plus la séquence SEQ ID N°31,
- les peptides ayant au moins la séquence SEQ ID N°13 et au plus la séquence SEQ ID N°33,
- les peptides ayant au moins la séquence SEQ ID N°14 et au plus une séquence choisie parmi les séquences SEQ ID N°35 et SEQ ID N°39,
- les peptides ayant au moins la séquence SEQ ID N°15 et au plus la séquence SEQ ID N°38,
- les peptides ayant au moins la séquence SEQ ID N°16 et au plus une séquence choisie parmi les séquences SEQ ID N°40 et SEQ ID N°42,
- les peptides ayant au moins la séquence SEQ ID N°17 et au plus la séquence SEQ ID N°43, et
- les peptides ayant au moins la séquence SEQ ID N°18 et au plus la séquence SEQ ID N°44.

20. Peptide A selon la revendication 19, **caractérisé en ce qu'**il est choisi parmi les peptides de séquences SEQ ID N°3 à 18.

21. Peptide A selon le revendication 20, **caractérisé en ce qu'**il a la séquence SEQ ID N°3.

22. Peptide B ayant au moins la séquence en acides aminés SEQ ID N°45 et au plus la séquence SEQ ID N°46.

23. Peptide B selon la revendication 22, **caractérisé en ce qu'**il est choisi parmi :
- les peptides ayant au moins la séquence SEQ ID N°47 et au plus la séquence SEQ ID N°81,
- les peptides ayant au moins la séquence SEQ ID N°48 et au plus la séquence SEQ ID N°82,
- les peptides ayant au moins la séquence SEQ ID N°49 et au plus la séquence SEQ ID N°83,
- les peptides ayant au moins la séquence SEQ ID N°50 et au plus une séquence choisie parmi les séquences SEQ ID N°84, SEQ ID N°90, SEQ ID N°105, SEQ ID N°107 et SEQ ID N°116,
- les peptides ayant au moins la séquence SEQ ID N°51 et au plus une séquence choisie parmi les séquences SEQ ID N°85 et SEQ ID N°124,
- les peptides ayant au moins la séquence SEQ ID N°52 et au plus une séquence choisie parmi les séquences SEQ ID N°86 et SEQ ID N°120,
- les peptides ayant au moins la séquence SEQ ID N°53 et au plus la séquence SEQ ID N°87,
- les peptides ayant au moins la séquence SEQ ID N°54 et au plus une séquence choisie parmi les séquences SEQ ID N°88 et SEQ ID N°117,
- les peptides ayant au moins la séquence SEQ ID N°55 et au plus la séquence SEQ ID N°89,
- les peptides ayant au moins la séquence SEQ ID N°56 et au plus une séquence choisie parmi les séquences SEQ ID N°91 et SEQ ID N°92,
- les peptides ayant au moins la séquence SEQ ID N°57 et au plus la séquence SEQ ID N°93,
- les peptides ayant au moins la séquence SEQ ID N°58 et au plus une séquence choisie parmi les séquences SEQ ID N°94 et SEQ ID N°110,
- les peptides ayant au moins la séquence SEQ ID N°59 et au plus la séquence SEQ ID N°95,
- les peptides ayant au moins la séquence SEQ ID N°60 et au plus une séquence choisie parmi les séquences SEQ ID N°96 et SEQ ID N°115,
- les peptides ayant au moins la séquence SEQ ID N°61 et au plus la séquence SEQ ID N°97,
- les peptides ayant au moins la séquence SEQ ID N°62 et au plus une séquence choisie parmi les séquences SEQ ID N°98 et SEQ ID N° 109,
- les peptides ayant au moins la séquence SEQ ID N°63 et au plus la séquence SEQ ID N°99,
- les peptides ayant au moins la séquence SEQ ID N°64 et au plus la séquence SEQ ID N°100,
- les peptides ayant au moins la séquence SEQ ID N°65 et au plus la séquence SEQ ID N°101,
- les peptides ayant au moins la séquence SEQ ID N°66 et au plus la séquence SEQ ID N°102,
- les peptides ayant au moins la séquence SEQ ID N°67 et au plus la séquence SEQ ID N°103,
- les peptides ayant au moins la séquence SEQ ID N°68 et au plus la séquence SEQ ID N°104,
- les peptides ayant au moins la séquence SEQ ID N°69 et au plus la séquence SEQ ID N°106,
- les peptides ayant au moins la séquence SEQ ID N°70 et au plus la séquence SEQ ID N°108,
- les peptides ayant au moins la séquence SEQ ID N°71 et au plus la séquence SEQ ID N°111,
- les peptides ayant au moins la séquence SEQ ID N°72 et au plus la séquence SEQ ID N°112,
- les peptides ayant au moins la séquence SEQ ID N°73 et au plus la séquence SEQ ID N°113,
- les peptides ayant au moins la séquence SEQ ID N°74 et au plus la séquence SEQ ID N°114,
- les peptides ayant au moins la séquence SEQ ID N°75 et au plus une séquence choisie parmi les séquences SEQ ID N°118 et SEQ ID N°119,
- les peptides ayant au moins la séquence SEQ ID N°76 et au plus la séquence SEQ ID N°121,
- les peptides ayant au moins la séquence SEQ ID N°77 et au plus la séquence SEQ ID N°122,
- les peptides ayant au moins la séquence SEQ ID N°78 et au plus la séquence SEQ ID . N°123,
- les peptides ayant au moins la séquence SEQ ID N°79 et au plus la séquence SEQ ID N°125, et
- les peptides ayant au moins la séquence SEQ ID N°80 et au plus la séquence SEQ ID N°126.

24. Peptide B selon la revendication 23, **caractérisé en ce qu'**il est choisi parmi les peptides de séquences SEQ ID N°47 à 80.

25. Peptide B selon la revendication 24, **caractérisé en ce qu'**il a la séquence SEQ ID N°47.

26. Peptide C ayant au moins la séquence SEQ ID N°127 et au plus la séquence SEQ ID N°128.

27. Peptide C selon la revendication 26, **caractérisé en ce qu'**il est choisi parmi :
- les peptides ayant au moins la séquence SEQ ID N°129 et au plus une séquence choisie parmi les séquences SEQ ID N°147, SEQ ID N°153, SEQ ID N°162 et SEQ ID N°167,
- les peptides ayant au moins la séquence SEQ ID N°130 et au plus une séquence choisie parmi les séquences SEQ ID N°148 et SEQ ID N°150,
- les peptides ayant au moins la séquence SEQ ID N°131 et au plus la séquence SEQ ID N°149,
- les peptides ayant au moins la séquence SEQ ID N°132 et au plus une séquence choisie parmi les séquences SEQ ID N°151, SEQ ID N°155, SEQ ID N°168 et SEQ ID N°171,
- les peptides ayant au moins la séquence SEQ ID N°133 et au plus la séquence SEQ ID N°152,
- les peptides ayant au moins la séquence SEQ ID N°134 et au plus une séquence choisie parmi les séquences SEQ ID N°154 et SEQ ID N°169,
- les peptides ayant au moins la séquence SEQ ID N°135 et au plus la séquence SEQ ID N°156,
- les peptides ayant au moins la séquence SEQ ID N°136 et au plus une séquence choisie parmi les séquences SEQ ID N°157 et SEQ ID N°170,
- les peptides ayant au moins la séquence SEQ ID N°137 et au plus la séquence SEQ ID N°158,
- les peptides ayant au moins la séquence SEQ ID N°138 et au plus la séquence SEQ ID N°159,
- les peptides ayant au moins la séquence SEQ ID N°139 et au plus la séquence SEQ ID N°160,
- les peptides ayant au moins la séquence SEQ ID N°140 et au plus la séquence SEQ ID N°161,
- les peptides ayant au moins la séquence SEQ ID N°141 et au plus la séquence SEQ ID N°163,
- les peptides ayant au moins la séquence SEQ ID N°142 et au plus la séquence SEQ ID N°164,
- les peptides ayant au moins la séquence SEQ ID N°143 et au plus la séquence SEQ ID N°165,
- les peptides ayant au moins la séquence SEQ ID N°144 et au plus la séquence SEQ ID N°166,
- les peptides ayant au moins la séquence SEQ ID N°145 et au plus la séquence SEQ ID N°172, et
- les peptides ayant au moins la séquence SEQ ID N°146 et au plus la séquence SEQ ID N°173.

28. Peptide C selon la revendication 27, **caractérisé en ce qu'**il est choisi parmi les séquences SEQ ID N°129 à 146.

29. Peptide C selon la revendication 28, **caractérisé en ce qu'**il a la séquence SEQ ID N°129.

30. Peptide D ayant au moins la séquence SEQ ID N°174 et au plus la séquence SEQ ID N°176.

31. Peptide D selon la revendication 30, **caractérisé en ce qu'**il est choisi parmi :
- les peptides ayant au moins la séquence SEQ ID N°176 et au plus une séquence choisie parmi les séquences SEQ ID N°188, SEQ ID N°192, SEQ ID N°193, SEQ ID N°194, SEQ ID N°201, SEQ ID N°202, SEQ ID N°203, SEQ ID N°204, SEQ ID N°205 et SEQ ID N°210,
- les peptides ayant au moins la séquence SEQ ID N°177 et au plus une séquence choisie parmi les séquences SEQ ID N°189 et SEQ ID N°190,
- les peptides ayant au moins la séquence SEQ ID N°178 et au plus la séquence SEQ ID N°191,
- les peptides ayant au moins la séquence SEQ ID N°179 et au plus la séquence SEQ ID N°195,
- les peptides ayant au moins la séquence SEQ ID N°180 et au plus une séquence choisie parmi les séquences SEQ ID N° 196 et SEQ ID N°206,
- les peptides ayant au moins la séquence SEQ ID N°181 et au plus la séquence SEQ ID N°197,
- les peptides ayant au moins la séquence SEQ ID N°182 et au plus une séquence choisie parmi les séquences SEQ ID N°198 et SEQ ID N°209,
- les peptides ayant au moins la séquence SEQ ID N°183 et au plus une séquence choisie parmi les séquences SEQ ID N° 199 et SEQ ID N°200,
- les peptides ayant au moins la séquence SEQ ID N°184 et au plus la séquence SEQ ID N°207,
- les peptides ayant au moins la séquence SEQ ID N°185 et au plus la séquence SEQ ID N°208,
- les peptides ayant au moins la séquence SEQ ID N°186 et au plus la séquence SEQ ID N°211, et
- les peptides ayant au moins la séquence SEQ ID N°187 et au plus la séquence SEQ ID N°212.

32. Peptide D selon la revendication 31, **caractérisé en ce qu'**il est choisi parmi les peptides de séquences SEQ ID N°176 à 187.

33. Peptide D selon la revendication 32, **caractérisé en ce qu'**il a la séquence SEQ ID N°176.

34. Epitope B', **caractérisé en ce qu'**il est choisi parmi les épitopes de séquences SEQ ID N°215 à SEQ ID N°220.

35. Epitope C' **caractérisé en ce qu'**il est choisi parmi les épitopes de séquences SEQ ID N°223 à SEQ ID N°232.

36. Séquences nucléotidiques codant pour l'un quelconque des peptides A à D tels que définis dans l'une quelconque des revendications 18 à 33 ou pour l'un quelconque des épitopes B' et C' tels que définis dans les revendications 34 et 35.

37. Vecteur d'expression **caractérisé en ce qu'**il comprend une séquence nucléotidique selon la revendication 36, ainsi que les moyens nécessaires à son expression.

38. Vecteur d'expression, **caractérisé en ce qu'**il comprend au moins deux séquences nucléotidiques codant pour les peptides A à D tels que définis dans les revendications 18 à 33, ainsi que les moyens nécessaires à leur expression, chaque séquence nucléotidique codant pour un peptide différent, lesdites au moins deux séquences nucléotidiques étant liées directement entre elles, ou bien par l'intermédiaire d'agents espaceurs constitués d'acides aminés ayant une charge neutre dans des conditions physiologiques et ayant une taille de 3 à 6 résidus.

39. Vecteur d'expression selon la revendication 38, **caractérisé en ce** les séquences nucléotidiques codent pour les peptides A et B, A et C, A et D, B et C, B et D, ou C et D.

40. Vecteur d'expression selon la revendication 38, **caractérisé en ce qu'**il comprend trois séquences nucléotidiques codant pour les peptides A, B et C, A, B et D, A, C et D, ou B, C et D.

41. Vecteur d'expression selon la revendication 38, **caractérisé en ce qu'**il comprend quatre séquences nucléotidiques codant pour les peptides A à D.

42. Vecteur d'expression selon la revendication 37, **caractérisé en ce qu'**il comprend au moins une séquence nucléotidique codant pour un épitope B' ou C' tel que défini dans l'une quelconque des revendications 34 et 35.

43. Vecteur d'expression selon la revendication 37, **caractérisé en ce qu'**il comprend de deux à quatre séquences nucléotidiques choisies parmi celles codant pour les peptides A, B, C, D et les épitopes B' à C', tels que définis dans les revendications 18 à 35, étant entendu que :
- la séquence nucléotidique codant pour le peptide B et la séquence nucléotidique codant pour l'épitope B' ne sont pas présentes en même temps, et
- la séquence nucléotidique codant pour le peptide C et la séquence nucléotidique codant pour l'épitope C' ne sont pas présentes en même temps,
lesdites deux à quatre séquences nucléotidiques étant liées directement entre elles, ou bien par l'intermédiaire d'agents espaceurs constitués d'acides aminés ayant une charge neutre dans des conditions physiologiques et ayant une taille de 3 à 6 résidus.

44. Microorganisme ou cellule hôte transformé par un vecteur d'expression tel que défini dans les revendications 37 à 43.

45. Microorganisme ou cellule hôte cotransformé par au moins deux vecteurs d'expression tels que définis dans la revendication 37, chaque vecteur d'expression codant pour un peptide différent.

46. Microorganisme ou cellule hôte selon la revendication 45, **caractérisé en ce qu'**il est cotransformé avec deux vecteurs d'expression codant, respectivement, pour les peptides A et B, A et C, A et D, B et C, B et D, ou C et D.

47. Microorganisme ou cellule hôte selon la revendication 45, **caractérisé en ce qu'**il est cotransformé avec trois vecteurs d'expression codant, respectivement, pour les peptides A, B et C, A, B et D, A, C et D, ou B, C et D.

48. Microorganisme ou cellule hôte selon la revendication 45, **caractérisé en ce qu'**il est cotransformé avec quatre vecteurs d'expression codant respectivement pour les peptides A, B, C et D.

49. Anticorps dirigés contre l'un au moins des peptides A à D tels que définis dans les revendications 18 à 33 ou contre l'une des compositions telles que définies dans l'une quelconque des revendications 1 à 17 ou bien contre l'un au moins des épitopes B' et C' tels que définis dans l'une quelconque des revendications 34 et 35.

50. Utilisation d'une composition selon l'une quelconque des revendications 1 à 17 pour la préparation d'un médicament destiné à l'inhibition, la prévention ou le traitement d'une infection provoquée par le virus de l'hépatite C chez un animal, de préférence l'homme.

51. Utilisation d'au moins un épitope B' ou C' tels que définis dans les revendications 34 et 35 pour la préparation d'un médicament destiné à l'inhibition, la prévention ou le traitement d'une infection provoquée par le virus de l'hépatite C chez un animal, de préférence l'homme.

52. Composition pharmaceutique, notamment vaccin, contenant à titre de substance active au moins une composition peptidique selon l'une des revendications 1 à 16, ou bien au moins deux séquences nucléotidiques selon la revendication 36 codant pour des peptides différents choisis parmi lesdits peptides A à D, placées sous le contrôle d'éléments nécessaires à une expression constitutive et/ou inductible desdits peptides A à D, ou bien l'un au moins des anticorps selon la revendication 49, ou bien encore au moins un des épitopes B' et C' tels que définis dans les revendications 34 et 35, ou bien au moins l'une de leur séquence nucléotidique telle que définie dans la revendication 36, en association avec un véhicule pharmaceutiquement approprié.

53. Composition diagnostique pour la détection et/ou la quantification du virus de l'hépatite C comprenant au moins une composition peptidique selon l'une des revendications 1 à 16, ou bien au moins un anticorps selon la revendication 49, ou bien au moins un épitopes selon l'une des revendications 34 et 35.

54. Procédé de détection et/ou de quantification du virus de l'hépatite C dans un échantillon biologique prélevé chez un individu susceptible d'être infecté par ledit virus, tel que plasma, sérum ou tissu, **caractérisé en ce qu'**il comprend les étapes consistant à :
- mettre en contact ledit échantillon biologique avec les anticorps selon la revendication 49 dans des conditions permettant la formation d'un complexe entre le virus et l'anticorps, et
- détecter et/ou quantifier la formation dudit complexe par tout moyen approprié.

55. Utilisation de la composition selon la revendication 53 pour le diagnostic *in vitro* du virus de l'hépatite C dans un échantillon ou un prélèvement biologique.

## Claims

1. Peptide composition, **characterized in that** it comprises at least two compounds chosen from:
- a A peptide having at least the following amino acid sequence SED ID No. 1:
X₁AX₂QGYKVX₃VLNPSVX₄ATLX₅FGX₆YMSKAX₇GX₈,
in which X₁ is Y or H, X₂ is A, G or T, X₃ is R or L, X₄ is A or T, X₅ is G or S, X₆ is A, T or V, X₇ is H or Y and X₈ is I, T, M or V,
and having at most the 46 amino acids as described in following SEQ ID No. 2:
SX₉X₁₀VPX₁₁X₁₂X₁AX₂QGYKVX₃VLNPSVX₄ATLX₅FGX₆YMSKAX₇GX₈X₁₃PX₁₄ X₁₅X₁₆X₁₇GV.
in which X₁ to X₈ are as defined in claim 1, and X₉ is T or N, X₁₀ is K or R, X₁₁ is A or V, X₁₂ is A or E, X₁₃ is E or D, X₁₄ is N or S, X₁₅ is I, L or V, X₁₆ is R or S and X₁₇ is T or S,
- a B peptide having at least the following amino acid sequence SED ID No. 45: '
GX₁₈X₁₉X₂₀X₂₁X₂₂X₂₃TSLTORDX₂₄NX₂₅X₂₆X₂₇GEX₂₈QX₂₉X₃₀STAX₃₁X₃₂X₃₃FLX₃₄ X₃₅X₃₆X₃₇NGX₃₉X₃₉X₄₀
in which X₁₈ is L or V, X₁₉ is L or F, X₂₀ is G or S, X₂₁ is C or T, X₂₂ is I or V, X₂₃ is I or V, X₂₄ is K, R or T, X₂₅ is Q or E, X₂₆ is V or N, X₂₇ is D, E or C, X₂₈ is V or A, X₂₉ is V, I, E or M, X₃₀ is L or V, X₃₁ is T, K or A, X₃₂ is Q or H, X₃₃ is S or T, X₃₄ is A or G, X₃₅ is T or S, X₃₆ is C or A, X₃₇ is V, I or T, X₃₈ is V or A, X₃₉ is C or M and X₄₀ is Y or F,
and having at most the 63 amino acids as described in following sequence SED ID No. 46:
AX₄₁ITX₄₂YX₄₃X₄₄TRGX₁₈X₁₉X₂₀X₂₁X₂₂X₂₃TSLTGRDX₂₄NX₂₅X₂₆X₂₇GEX₂₈QX₂₉ X₃₀STAX₃₁X₃₂X₃₃FLX₃₄X₃₅X₃₆X₃₇NGX₃₈X₃₉WTVX₄₀HGAGX₄₅X₄₆X₄₇
in which X₁₈ to X₄₀ are as defined in claim 1 and X₄₁ is P, S or H, X₄₂ is A or T, X₄₃ is S,A,T or C, X₄₄ is Q or R, X₄₅ is S, T or A, X₄₆ is K or R and X₄₇ is T or I,
- a peptide C having at least the following amino acid sequence SEQ ID No. 127:
SX₄₇M X₄₈FTX₄₉X₅₀X₅₁TSPLX₅₂X₅₃X₅₄X₅₅TLX₅₆FNIX₅₇GGWVAX₅₈QX₅₉
in which X₄₇ is L or P, X₄₈ is A or S, X₄₉ is A or S, X₅₀ is A or S, X₅₁ is I or V, X₅₂ is T,S or A, X₅₃ is T or I, X₅₄ is Q, S or G, X₅₅ is N,Q,H,S,Y or T, X₅₆ is L or M, X₅₇ is L or W, X₅₈ is A or S and X₅₉ is L, P or I,
and having at most the 57 amino acids such as described in following SEQ ID No. 128:
NFIX₆₀GX₆₁QYLAX₆₂LSTLPGNX₆₃AX₆₄X₆₅SX₄₇MX₄₈FTX₄₉X₅₀X₅₁TSPLX₅₂X₅₃X₅₄ X₅₅TLX₅₆FNIX₅₇GGWAX₅₈QX₅₉X₆₆X₆₇X₆₈
in which X₄₇ to X₅₉ are as defined in claim 1 and X₆₀ is T or S, X₆₁ is I, T or V, X₆₂ is G or A, X₆₃ is P or L, X₆₄ is I or M, X₆₅ is A,V or R, X₆₆ is A or R, X₆₇ is P, A or D and X₆₈ is P, A or S,
- a D peptide having at least the following amino acid sequence SEQ ID No. 172:
X₆₉KX₇₀ARX₇₁IVX₇₂PX₇₃LGX₇₄RVCEKX₇₅ALX₇₆X₇₇VX₇₈X₇₉X₈₀X₈₁
in which X₆₉ is R or Q, X₇₀ is P or A, X₇₁ is L or F, X₇₂ is F or Y, X₇₃ is D or E, X₇₄ is V or S, X₇₅ is M or R, X₇₆ is Y or H, X₇₇ is D or N, X₇₈ is V or I, X₇₉ is S,T or K, X₈₀ is T,K,I or N, and X₈₁ is L or T,
and having at most the 44 amino acids as described in following sequence SEQ ID No. 175:
KGGX₆₉KX₇₀ARX₇₁IVX₇₂PX₇₃LGX₇₄RVCEKX₇₅ALX₇₆X₇₇VX₇₈X₇₉X₈₀X₈₁X₈₂X₈₃X₈₄ VMGX₈₅X₈₆YX₈₇X₈₈Q
in which X₆₉ to X₈₁ are as defined in claim 1 and X₈₂ is P or A, X₈₃ is Q, L, H, R, K or P, X₈₄ is A, T, V or P, X₈₅ is P, S or A, X₈₆ is S or A, X₈₇ is G or R and X₈₈ is F or C,
- a B' epitope having the following sequence SEQ ID No. 213:
GX₁₈X₁₉X₂₀X₂₁X₂₂X₂₃TSL
in which X₁₈ is L or V, X₁₉ is L or F, X₂₀ is G or S, X₂₁ is C or T,X₂₂ is I or V, and X₂₃ is I or V, and
- a C' epitope having the following sequence SEQ ID No. 221:
SPLX₅₂X₅₃X₅₄X₅₅TL
in which X₅₂ is T, S or A, X₅₃ is T or I, X₅₄ is Q,S or G and X₅₅ is N, Q, H, S, Y or T.

2. Peptide composition according to claim 1, **characterized in that** it comprises at least two peptides chosen from the peptides A, B, C, D.

3. Peptide composition according to either of claims 1 or 2, **characterized in that** the A peptide is chosen from the following peptides:
- the peptides having at least sequence SEQ ID No. 3 and at most sequence SEQ ID No. 19,
- the peptides having at least SEQ ID No. 4 and at most a sequence chosen from:
i) sequence SEQ ID No. 20,
ii) sequence SEQ ID No. 24,
iii) sequence SEQ ID No. 32, and
iv) sequence SEQ ID No. 34,
- the peptides having at least sequence SEQ ID No. 5 and at most a sequence chosen from:
i) sequence SEQ ID No. 21
ii) sequence SEQ ID No. 28 and
iii sequence SEQ ID No. 36,
- the peptides having at least sequence SEQ ID No. 6 and at most a sequence chosen from:
i) sequence SEQ ID No. 22,
ii) sequence SEQ ID No. 27 and
iii) sequence SEQ ID No. 41,
- the peptides having at least sequence SEQ ID No. 7 and at most a sequence chosen from:
i) sequence SEQ ID No. 23 and
ii) sequence SEQ ID No. 37,
- the peptides having at least sequence SEQ ID No. 8 and at most sequence SEQ ID No. 25,
- the peptides having at least sequence SEQ ID No. 9 and at most sequence SEQ ID No. 26,
- the peptides having at least sequence SEQ ID No. 10 and at most sequence SEQ ID No. 29,
- the peptides having at least sequence SEQ ID No. 11 and at most sequence SEQ ID No. 30,
- the peptides having at least sequences SEQ ID No. 12 and at most sequence SEQ ID No. 31,
- the peptides having at least sequence SEQ ID No. 13 and at most sequence SEQ ID No. 33,
- the peptides having at least sequences SEQ ID No. 14 and at most a sequence from:
i) sequence SEQ ID No. 35 and
ii) sequence SEQ ID No. 39,
- the peptides having at least sequence SEQ ID No. 15 and at most sequence SEQ ID No. 38,
- the peptides having at least sequence SEQ ID No. 16 and at most a sequence from:
i) sequence SEQ ID No. 40 and
ii) sequence SEQ ID No. 42,
- the peptides having at least SEQ ID No. 17 and at most sequence No. 43, and
- the peptides having at least sequence SEQ ID No. 18 and at most sequence SEQ ID No. 44.

4. Peptide composition according to claim 3, **characterized in that** the A peptide is chosen from the peptides of sequences SEQ ID No. 3 to 18.

5. Peptide composition according to claim 4, **characterized in that** the A peptide has the sequence SEQ ID No. 3.

6. Peptide composition according to any one of claims 1 to 5, **characterized in that** the B peptide is chosen from:
- the peptides having at least sequence SEQ ID No. 47 and at most sequence SEQ ID No. 81,
- the peptides having at least sequence SEQ ID No. 48 and at most sequence SEQ ID No. 82,
- the peptides having at least sequence SEQ ID No. 49 and at most SEQ ID No. 83,
- the peptides having at least sequence SEQ ID No. 50 and at most a sequence chosen from:
i) sequence SEQ ID No. 84,
ii) sequence SEQ ID No. 90,
iii) sequence SEQ ID No. 105,
iv) sequence SEQ ID No. 107 and
v) sequence SEQ ID No. 116,
- the peptides having at least sequence SEQ ID No. 51 and at most a sequence chosen from:
i) sequence SEQ ID No. 85 and
ii) sequence SEQ ID No. 124,
- the peptides having at least sequence SEQ ID No. 52 and at most a sequence chosen from:
i) sequence SEQ ID No. 86 and
ii) sequence SEQ ID No. 120,
- the peptides having at least sequence SEQ ID No. 53 and at most sequence SEQ ID No. 87,
- the peptides having at least sequence SEQ ID No. 54 and at most a sequence from :
i) sequence SEQ ID No. 88 and
ii) sequence SEQ ID No. 117,
- the peptides having at least sequence SEQ ID No. 55 and at most sequence SEQ ID No. 89,
- the peptides having at least sequence SEQ ID No. 56 and at most a sequence chosen from :
i) sequence SEQ ID No. 91 and
ii) sequence SEQ ID No. 92,
- the peptides having at least sequence SEQ ID No. 57 and at most sequence SEQ ID No. 93,
- the peptides having at least sequence SEQ ID No. 58 and at most a sequence from :
i) sequence SEQ ID No. 94 and
ii) sequence SEQ ID No. 110,
- the peptides having at least sequence SEQ ID No. 59 and at most sequence SEQ ID No. 95,
- the peptides having at least sequence SEQ ID No. 60 and at most a sequence from :
i) sequence SEQ ID No. 96 and
ii) sequence SEQ ID No. 115,
- the peptide having at least sequence SEQ ID No. 61 and at most sequence SEQ ID No. 97,
- the peptides having at least sequence SEQ ID No. 62 and at most a sequence chosen from :
i) sequence SEQ ID No. 98 and
ii) sequence SEQ ID No. 109,
- the peptides having at least sequence SEQ ID No. 63 and at most sequence SEQ ID No. 99,
- the peptides having at least sequence SEQ ID No. 64 and at most sequence SEQ ID No. 100,
- the peptides having at least sequence SEQ ID No. 65 and at most sequence SEQ ID No. 101,
- the peptides having at least sequence SEQ ID No. 66 and at most sequence SEQ ID No. 102,
- the peptides having at least sequence SEQ ID No. 67 and at most sequence SEQ ID No. 103,
- the peptides having at least sequence SEQ ID No. 68 and at most sequence SEQ ID No. 104,
- the peptides having at least sequence SEQ ID No. 69 and at most sequence SEQ ID No. 106,
- the peptides having at least sequence SEQ ID No. 70 and at most sequence SEQ ID No. 108,
- the peptides having at least sequence SEQ ID No. 71 and at most sequence SEQ ID No. 111,
- the peptides having at least sequence SEQ ID No. 72 and at most sequence SEQ ID No. 112
- the peptides having at least sequence SEQ ID No. 73 and at most sequence SEQ ID No. 113,
- the peptides having at least sequence SEQ ID No. 74 and at most sequence SEQ ID No. 114,
- the peptides having at least sequence SEQ ID No. 75 and at most a sequence chosen from :
i) sequence SEQ ID No. 118 and
ii) sequence SEQ ID No. 119,
- the peptides having at least sequence SEQ ID No. 76 and at most sequence SEQ ID No. 121,
- the peptides having at least sequence SEQ ID No. 77 and at most sequence SEQ ID No. 122,
- the peptides having at least sequence SEQ ID No. 78 and at most sequence SEQ ID No. 123,
- the peptides having at least sequence SEQ ID No. 79 and at most sequence SEQ ID No. 125, and
- the peptides having at least sequence SEQ ID No. 80 and at most sequence SEQ ID No. 126.

7. Peptide composition according to claim 6, **characterized in that** the B peptide is chosen from the peptides of sequences SEQ ID No. 47 to 80.

8. Peptide composition according to claim 7, **characterized in that** the B peptide has sequence SEQ ID No. 47.

9. Peptide composition according to any one of claims 1 to 8, **characterized in that** the C peptide is chosen from :
- the peptides having at least sequence SEQ ID No. 129 and at most a sequence chosen from :
i) sequence SEQ ID No. 147,
ii) sequence SEQ ID No. 153,
iii) sequence SEQ ID No. 162, and
iv) sequence SEQ ID No. 167,
- the peptides having at least sequence SEQ ID No. 130 and at most a sequence chosen from :
i) sequence SEQ ID No. 148 and
ii) sequence SEQ ID No. 150,
- the peptides having at least sequence SEQ ID No. 131 and at most sequence SEQ ID No. 149,
- the peptides having at least sequence SEQ ID No. 132 and at most a sequence chosen from :
i) sequence SEQ ID No. 151,
ii) sequence SEQ ID No. 155,
iii) sequence SEQ ID No. 168, and
iv) sequence SEQ ID No. 171,
- the peptides having at least sequence SEQ ID No. 133 and at most sequence SEQ ID No. 152,
- the peptides having at least sequence SEQ ID No. 134 and at most a sequence chosen from :
i) sequence SEQ ID No. 154 and
ii) sequence SEQ ID No. 169,
- the peptides having at least sequence SEQ ID No. 135 and at most sequence SEQ ID No. 156,
- the peptides having at least sequence SEQ ID No. 136 and at most a sequence chosen from :
i) sequence SEQ ID No. 157 and
ii) sequence SEQ ID No. 170,
- the peptides having at least sequence SEQ ID No. 137 and at most sequence SEQ ID No. 158,
- the peptides having at least sequence SEQ ID No. 138 and at most sequence SEQ ID No. 159,
- the peptides having at least sequence SEQ ID No. 139 and at most sequence SEQ ID No. 160,
- the peptides having at least sequence SEQ ID No. 140 and at most sequence SEQ ID No. 161,
- the peptides having at least sequence SEQ ID No. 141 and at most sequence SEQ ID No. 163,
- the peptides having at least sequence SEQ ID No. 142 and at most sequence SEQ ID No. 164,
- the peptides having at least sequence SEQ ID No. 143 and at most sequence SEQ ID No. 165,
- the peptides having at least sequence SEQ ID No. 144 and at most sequence SEQ ID No. 166,
- the peptides having at least sequence SEQ ID No. 145 and at most sequence SEQ ID No. 172,
- the peptides having at least sequence SEQ ID No. 146 and at most sequence SEQ ID No. 173.

10. Peptide composition according to claim 9, **characterized in that** the C peptide is chosen from the peptides of sequences SEQ ID No. 129 to 146.

11. Peptide composition according to claim 10, **characterized in that** the C peptide has the sequence SEQ ID No. 129.

12. Peptide composition according to any one of claims 1 to 11, **characterized in that** the D peptide is chosen from :
- the peptides having at least sequence SEQ ID No. 176 and at most a sequence chosen from :
i) sequence SEQ ID No. 188,
ii) sequence SEQ ID No. 192,
iii) sequence SEQ ID No. 193,
iv) sequence SEQ ID No. 194,
v) sequence SEQ ID No. 201,
vi) sequence SEQ ID No. 202,
vii) sequence SEQ ID No. 203,
viii) sequence SEQ ID No. 204,
ix) sequence SEQ ID No. 205, and
x) sequence SEQ ID No. 210,
- the peptides having at least sequence SEQ ID No. 177 and at most a sequence chosen from :
i) sequence SEQ ID No. 189 and
ii) sequence SEQ ID No. 190,
- the peptides having at least sequence SEQ ID No. 178 and at most sequence SEQ ID No. 191,
- the peptides having at least sequence SEQ ID No. 179 and at most sequence SEQ ID No. 195,
- the peptides having at least sequence SEQ ID No. 180 and at most a sequence chosen from :
i) sequence SEQ ID No. 196 and
ii) sequence SEQ ID No. 206,
- the peptides having at least sequence SEQ ID No. 181 and at most sequence SEQ ID No. 197,
- the peptides having at least sequence SEQ ID No. 182 and at most a sequence chosen from :
i) sequence SEQ ID No. 198, and
ii) sequence SEQ ID No. 209,
- the peptides having at least sequence SEQ ID No. 183 and at most a sequence chosen from :
i) sequence SEQ ID No. 199 and
ii) sequence SEQ ID No. 200,
- the peptides having at least sequence SEQ ID No. 184 and at most sequence SEQ ID No. 207,
- the peptides having at least sequence SEQ ID No. 185 and at most sequence SEQ ID No. 208
- the peptides having at least sequence SEQ ID No. 186 and at most sequence SEQ ID No. 211
- the peptides having at least sequence SEQ ID No. 187 and at most sequence SEQ ID No. 212,

13. Peptide composition according to claim 12, **characterized in that** the D peptide is chosen from the peptides of sequences SEQ ID No. 176 to 187.

14. Peptide composition according to claim 13, **characterized in that** the D peptide has the sequence SEQ ID No. 176.

15. Peptide composition according to any one of claims 1 to 14, **characterized in that** it comprises the two following peptides:
i) the A peptide as defined in any one of claims 1 and 3 to 5 and another peptide chosen from the B to D peptides as defined in any one of claims 1 and 6 to 14, or
ii) the B peptide as defined in any one of claims 1 and 6 to 8, and another peptide chosen from the A, C, or D peptides as defined in any one of claims 1 and 3 to 5 and 9 to 14, or
iii) the C peptide such as defined in any one of claims 1 and 9 to 11 and another peptide chosen from the A, B or D peptides such as defined in any one of claims 1 and 3 to 8 and 12 to 14, or
iv) the D peptide such as defined in any one of claims 1 and 12 to 14 and another peptide chosen from the A, B or C peptides such as defined in any one of claims 1 and 3 to 11.

16. Peptide composition according to any one of claims 1 to 14, **characterized in that** it comprises the three following peptides:
i) the A peptide as defined in any one of claims 1 and 3 to 5 and two other peptides chosen from the B to D peptides as defined in any one of claims 1 and 6 to 14, or
ii) the B peptide as defined in any one of claims 1 and 6 to 8 and two other peptides chosen from the A, C or D peptides as defined in any one of claims 1 and 3 to 5 and 9 to 14, or
iii) the C peptide as defined in any one of claims 1 and 9 to 11 and two other peptides chosen from the A, B or D peptides as defined in any one of claims 1 and 3 to 8 and 12 to 14, or
iv) the D peptide as defined in any one of claims 1 and 12 to 14 and two other peptides chosen from the A, B or C peptides as defined in any one of claims 1 and 3 to 11.

17. Peptide composition according to any one of claims 1 to 14, **characterized in that** it comprises the four A, B, C, D peptides as defined in any one of claims 1 and 3 to 14.

18. Peptide A having at least the amino acid sequence SEQ ID No. 1 and at most sequence SEQ ID No. 2.

19. Peptide A according to claim 18, **characterized in that** it is chosen from :
- the peptides having at least sequence SEQ ID No. 3 and at most sequence SEQ ID No. 19,
- the peptides having at least sequence SEQ ID No. 4 and at most a sequence chosen from sequences SEQ ID No. 20, SEQ ID No. 24, SEQ ID No. 32 and SEQ ID No. 34,
- the peptides having at least sequence SEQ ID No. 5 and at most a sequence chosen from the sequences SEQ ID No. 21, SEQ ID No. 28 and SEQ ID No. 36,
- the peptides having at least sequence SEQ ID No. 6 and at most a sequence chosen from the sequences SEQ ID No. 22, SEQ ID No. 27 and SEQ ID No. 41,
- the peptides having at least sequence SEQ ID No. 7 and at most a sequence chosen from sequences SEQ ID No. 23 and SEQ ID No. 37,
- the peptides having at least sequence SEQ ID No. 8 and at most sequence SEQ ID No. 25,
- the peptides having at least sequence SEQ ID No. 9 and at most sequence SEQ ID No. 26,
- the peptides having at least sequence SEQ ID No. 10 and at most sequence SEQ ID No. 29,
- the peptides having at least sequence SEQ ID No. 11 and at most sequence SEQ ID No. 30,
- the peptides having at least sequence SEQ ID No. 12 and at most sequence SEQ ID No. 31,
- the peptides having at least sequence SEQ ID No. 13 and at most sequence SEQ ID No. 33,
- the peptides having at least sequence SEQ ID No. 14 and at most a sequence chosen from sequences SEQ ID No. 35 and SEQ ID No. 39,
- the peptides having at least sequence SEQ ID No. 15 and at most sequence SEQ ID No. 38,
- the peptides having at least sequence SEQ ID No. 16 and at most a sequence chosen from sequences SEQ ID No. 40 and SEQ ID No. 42,
- the peptides having at least sequence SEQ ID No. 17 and at most sequence SEQ ID No. 43,
- the peptides having at least sequence SEQ ID No. 18 and at most sequence SEQ ID No. 44.

20. Peptide A according to claim 19, **characterized in that** it is chosen from the peptides of sequences SEQ ID No. 3 to 18.

21. Peptide A according to claim 20, **characterized in that** it has the sequence SEQ ID No. 3.

22. Peptide B having at least the amino acid sequence SEQ ID No. 45 and at most sequence SEQ ID No. 46.

23. Peptide B according to claim 22, **characterized in that** it is chosen from :
- the peptides having at least sequence SEQ ID No. 47 and at most sequence SEQ ID No. 81,
- the peptides having at least sequence SEQ ID No. 48 and at most sequence SEQ ID No. 82,
- the peptides having at least sequence SEQ ID No. 49 and at most sequence SEQ ID No. 83,
- the peptides having at least sequence SEQ ID No. 50 and at most a sequence chosen from the sequences SEQ ID No. 84, SEQ ID No. 90, SEQ ID No. 105, SEQ ID No. 107, and SEQ ID No. 116,
- the peptides having at least sequence SEQ ID No. 51 and at most a sequence chosen from sequences SEQ ID No. 85 and SEQ ID No. 124,
- the peptides having at least sequence SEQ ID No. 52 and at most a sequence chosen from sequences SEQ ID No. 86 and SEQ ID No. 120,
- the peptides having at least sequence SEQ ID No. 53 and at most sequence SEQ ID No. 87,
- the peptides having at least sequence SEQ ID No. 54 and at most a sequence chosen from sequences SEQ ID No. 88 and SEQ ID No. 117,
- the peptides having at least sequence SEQ ID No. 55 and at most sequence SEQ ID No. 89,
- the peptides having at least sequence SEQ ID No. 56 and at most a sequence chosen from sequences SEQ ID No. 91 and SEQ ID No. 92,
- the peptides having at least sequence SEQ ID No. 57 and at most sequence SEQ ID No. 93,
- the peptides having at least sequence SEQ ID No. 58 and at most a sequence chosen from sequences SEQ ID No. 94 and SEQ ID No. 110,
- the peptides having at least sequence SEQ ID No. 59 and at most sequence SEQ ID No. 95,
- the peptides having at least sequence SEQ ID No. 60 and at most a sequence chosen from sequences SEQ ID No. 96 and SEQ ID No. 115,
- the peptides having at least sequence SEQ ID No. 61 and at most sequence SEQ ID No. 97,
- the peptides having at least sequence SEQ ID No. 62 and at most a sequence from sequences SEQ ID No. 98 and SEQ ID No. 109,
- the peptides having at least sequence SEQ ID No. 63 and at most sequence SEQ ID No. 99,
- the peptides having at least sequence SEQ ID No. 64 and at most sequence SEQ ID No. 100,
- the peptides having at least sequence SEQ ID No. 65 and at most sequence SEQ ID No. 101,
- the peptides having at least sequence SEQ ID No. 66 and at most sequence SEQ ID No. 102,
- the peptides having at least sequence SEQ ID No. 67 and at most sequence SEQ ID No. 103,
- the peptides having at least sequence SEQ ID No. 68 and at most sequence SEQ ID No. 104,
- the peptides having at least sequence SEQ ID No. 69 and at most sequence SEQ ID No. 106,
- the peptides having at least sequence SEQ ID No. 70 and at most sequence SEQ ID No. 108,
- the peptides having at least sequence SEQ ID No. 71 and at most sequence SEQ ID No. 111,
- the peptides having at least sequence SEQ ID No. 72 and at most sequence SEQ ID No. 112,
- the peptides having at least sequence SEQ ID No. 73 and at most sequence SEQ ID No. 113,
- the peptides having at least sequence SEQ ID No. 74 and at most sequence SEQ ID No. 114,
- the peptides having at least sequence SEQ ID No. 75 and at most a sequence from sequences SEQ ID No. 118 and SEQ ID No. 119,
- the peptides having at least sequence SEQ ID No. 76 and at most sequence SEQ ID No. 121,
- the peptides having at least sequence SEQ ID No. 77 and at most sequence SEQ ID No. 122,
- the peptides having at least sequence SEQ ID No. 78 and at most sequence SEQ ID No. 123,
- the peptides having at least sequence SEQ ID No. 79 and at most sequence SEQ ID No. 125, and
- the peptides having at least sequence SEQ ID No. 80 and at most sequence SEQ ID No. 126.

24. Peptide B according to claim 23, **characterized in that** it is chosen from the peptides of sequences SEQ ID No. 47 to 80.

25. Peptide B according to claim 24, **characterized in that** it has sequence SEQ ID No. 47.

26. Peptide C having at least sequence SEQ ID No. 127 and at most sequence SEQ ID No. 128.

27. Peptide C according to claim 26, **characterized in that** it is chosen from:
- the peptides having at least sequence SEQ ID No. 129 and at most a sequence chosen from sequences SEQ ID No. 147, SEQ ID No. 153, SEQ ID No. 162 and SEQ ID No. 167,
- the peptides having at least sequence SEQ ID No. 130 and at most a sequence chosen from sequences SEQ ID No. 148 and SEQ ID No. 150,
- the peptides having at least sequence SEQ ID No. 131 and at most sequence SEQ ID No. 149,
- the peptides having at least sequence SEQ ID No. 132 and at most a sequence chosen from the sequences SEQ ID No. 151, SEQ ID No. 155, SEQ ID No. 168 and SEQ ID No. 171,
- the peptides having at least sequence SEQ ID No. 133 and at most sequence SEQ ID No. 152,
- the peptides having at least sequence SEQ ID No. 134 and at most a sequence chosen from sequences SEQ ID No. 154 and SEQ ID No. 169,
- the peptides having at least sequence SEQ ID No. 135 and at most sequence SEQ ID No. 156,
- the peptides having at least sequence SEQ ID No. 136 and at most a sequence chosen from sequences SEQ ID No. 157 and SEQ ID No. 170,
- the peptides having at least sequence SEQ ID No. 137 and at most sequence SEQ ID No. 158,
- the peptides having at least sequence SEQ ID No. 138 and at most sequence SEQ ID No. 159,
- the peptides having at least sequence SEQ ID No. 139 and at most sequence SEQ ID No. 160,
- the peptides having at least sequence SEQ ID No. 140 and at most sequence SEQ ID No. 161,
- the peptides having at least sequence SEQ ID No. 141 and at most sequence SEQ ID No. 163,
- the peptides having at least sequence SEQ ID No. 142 and at most sequence SEQ ID No. 164,
- the peptides having at least sequence SEQ ID No. 143 and at most sequence SEQ ID No. 165,
- the peptides having at least sequence SEQ ID No. 144 and at most sequence SEQ ID No. 166,
- the peptides having at least sequence SEQ ID No. 145 and at most sequence SEQ ID No. 172,
- the peptides having at least sequence SEQ ID No. 146 and at most sequence SEQ ID No. 173.

28. Peptide C according to claim 27, **characterized in that** it is chosen from the sequences SEQ ID No. 129 to 146.

29. Peptide C according to claim 28, **characterized in that** it has sequence SEQ ID No. 129.

30. Peptide D having at least sequence SEQ ID No. 174 and at most sequence SEQ ID No. 176.

31. Peptide D according to claim 30, **characterized in that** it is chosen from:
- the peptides having at least sequence SEQ ID No. 176 and at most a sequence chosen from the sequences SEQ ID No. 188, SEQ ID No. 192, SEQ ID No. 193, SEQ ID No. 194, SEQ ID No. 201 SEQ ID No. 202, SEQ ID No. 203 SEQ ID No. 204, SEQ ID No. 205 and SEQ ID No. 210,
- the peptides having at least sequence SEQ ID No. 177 and at most a sequence chosen from sequences SEQ ID No. 189 and SEQ ID No. 190,
- the peptides having at least sequence SEQ ID No. 178 and at most sequence SEQ ID No. 191,
- the peptides having at least sequence SEQ ID No. 179 and at most sequence SEQ ID No. 195
- the peptides having at least sequence SEQ ID No. 180 and at most a sequence chosen from sequences SEQ ID No. 196 and SEQ ID No. 206,
- the peptides having at least sequence SEQ ID No. 181 and at most sequence SEQ ID No. 197,
- the peptides having at least sequence SEQ ID No. 182 and at most a sequence from sequences SEQ ID No. 198 and SEQ ID No. 209,
- the peptides having at least sequence SEQ ID No. 183 and at most a sequence from sequences SEQ ID No. 199 and SEQ ID No. 200,
- the peptides having at least sequence SEQ ID No. 184 and at most sequence SEQ ID No. 207,
- the peptides having at least sequence SEQ ID No. 185 and at most sequence SEQ ID No. 208,
- the peptides having at least sequence SEQ ID No. 186 and at most sequence SEQ ID No. 211, and
- the peptides having at least sequence SEQ ID No. 187 and at most sequence SEQ ID No. 212.

32. Peptide D according to claim 31, **characterized in that** it is chosen from the peptides of sequences SEQ ID No. 176 to 187.

33. Peptide D according to claim 32, **characterized in that** it has the sequence SEQ ID No. 176.

34. Epitope B', **characterized in that** it is chosen from the epitopes of sequences SEQ ID No. 215 to SEQ ID No. 220.

35. Epitope C', **characterized in that** it is chosen from the epitopes of sequences SEQ ID No. 223 to SEQ ID No. 232.

36. Nucleotide sequences encoding any one of the A to D peptides such as defined in any one of claims 18 to 33 or encoding any on of the B' and C' epitopes as defined in claims 34 and 35.

37. Expression vector **characterized in that** it comprises a nucleotide sequence according to claim 36, as well as the means necessary for its expression.

38. Expression vector, **characterized in that** it comprises at least two nucleotide sequences encoding the A to D peptides as defined in claims 18 to 33, as well as the means necessary for their expression, each nucleotide sequence encoding a different peptide, said at least two nucleotide sequences being linked directly to the others or via spacer agents consisting of amino acides having a neutral charge under physiological conditions and having a size of 3 to 6 residues.

39. Expression vector according to claim 38, **characterized in that** the nucleotide sequences encode the A and B, A and C, A and D, B and C, B and D, or C and D peptides.

40. Expression vector according to claim 38, **characterized in that** it comprises three nucleotide sequences encoding the A, B and C, A, B and D, A, C and D, or B, C and D peptides.

41. Expression vector according to claim 38, **characterized in that** it comprises four nucleotide sequences encoding the A to D peptides.

42. Expression vector according to claim 37, **characterized in that** it comprises at least one nucleotide sequence encoding a B' or C' epitope as defined in either of claims 34 and 35.

43. Expression vector according to claim 37, **characterized in that** it comprises from two to four nucleotide sequences chosen from those encoding the A, B, C, D peptides and the B' to C' epitopes, as defined in claims 18 to 35, on the understanding that:
- the nucleotide sequence encoding the B peptide and the nucleotide sequence encoding the B' epitope are not present at the same time, and
- the nucleotide sequence encoding the C peptide and the nucleotide sequence encoding the C' epitope are not present at the same time,
said two to four nucleotide sequences being linked directly to the others or via spacer agents consisting of amino acids having a neutral charge under physiological conditions and having a size of 3 to 6 residues.

44. Microorganism or host cell transformed by an expression vector as defined in claims 37 to 43.

45. Microorganism or host cell co-transformed by at least two expression vectors as defined in claim 37, each expression vector encoding a different peptide.

46. Microorganism or host cell according to claim 45, **characterized in that** it is co-transformed with two expression vectors respectively encoding the A and B, A and C, A and D, B and C, B and D, or C and D peptides.

47. Microorganism or host cell according to claim 45, **characterized in that** it is co-transformed with three expression vectors respectively encoding the A, B and C, A, B and D, A, C and D, or B, C and D peptides.

48. Microorganism or host cell according to claim 45, **characterized in that** it is co-transformed with four expression vectors respectively encoding the A, B, C and D peptides.

49. Antibodies directed against at least one of the A to D peptides as defined in claims 18 to 33, or against one of the compositions as defined in any one of claims 1 to 17, or else against at least one of the B' and C' epitopes as defined in either of claims 34 and 35.

50. Use of a composition according to any one of claims 1 to 17 for the preparation of a medicinal product intended to inhibit, prevent or treat an infection caused by the hepatitis C virus in an animal, preferably human.

51. Use of at least one B' or C' epitope as defined in either of claims 34 and 35 for the preparation of a medicinal product intended to inhibit, prevent or treat an infection caused by the hepatitis C virus in an animal, preferably human.

52. Pharmaceutical composition, notably a vaccine, containing as active ingredient at least one peptide composition according to any one of claims 1 to 16, or else at least two nucleotide sequences according to claim 36 encoding different peptides chosen from said A to D peptides, placed under the control of elements necessary for constitutive and/or inducible expression of said A to D peptides, or else at least one of the antibodies according to claim 49, or else at least one of the B' and C' epitopes as defined in claims 34 and 35, or else at least one of their nucleotide sequences such as defined in claim 36, in association with a pharmaceutically suitable vehicle.

53. Diagnostic composition for the detection and/or quantification of the hepatitis C virus, comprising at least one peptide composition according to any one of claims 1 to 16, or else at least one antibody according to claim 49, or else at least one epitope according to either of claims 34 and 35.

54. Process for detection and/or quantification of the hepatitis C virus in a biological sample taken from an individual who may be infected with said virus, such as plasma, serum or tissue, **characterized in that** it comprises the steps consisting of:
- contacting said biological sample with an antibody according to claim 49 under conditions allowing the formation of a complex between the virus and the antibody, and
- detecting and/or quantifying the formation of said complex using any suitable means.

55. Use of the composition according to claim 53 for the in vitro diagnosis of the hepatitis C virus in a biological sample.

## Patentansprüche

1. Peptidzusammensetzungen, **dadurch gekennzeichnet, dass** sie mindestens zwei Komponenten enthält, ausgewählt aus:
- einem Peptid A, das zumindest die folgende Aminosäurensequenz SEQ ID Nr. 1 hat:
X₁AX₂QGYKVX₃VLNPSVX₄ATLX₅FGX₆YMSKAX₇GX₈,
in welcher X₁ Y oder H ist, X₂ A, G oder T ist, X₃ R oder L ist, X₄ A oder T ist, X₅ G oder S ist, X₆ A, T oder V ist, X₇ H oder Y ist und X₈ I, T, M oder V ist,
und das höchstens die 46 Aminosäuren, wie sie in der folgenden Sequenz SEQ ID Nr. 2 beschrieben sind, hat:
SX₉X₁₀VPX₁₁X₁₂X₁AX₂QGYKVX₃VLNPSVX₄ATLX₅FGX₆YMSKAX₇GX₈X₁₃ PX₁₄X₁₅X₁₆X₁₇GV,
in welcher X₁ bis X₈ wie in Anspruch, 1 definiert sind und X₉ T oder N ist, X₁₀ K oder R ist, X₁₁ A oder V ist, X₁₂ A oder E ist, X₁₃ E oder D ist, X₁₄ N oder S ist, X₁₅ I, L oder V ist, X₁₆ R oder S ist und X₁₇ T oder S ist,
- einem Peptid B, das zumindest die folgende Aminosäurensequenz SEQ ID Nr. 45 hat:
GX₁₈X₁₉X₂₀X₂₁X₂₂X₂₃TSLTGROX₂₄NX₂₅X₂₆X₂₇GEX₂₈QX₂₉X₃₀STAX₃₁X₃₂X₃₃ FLX₃₄X₃₅X₃₆X₃₇NGX₃₈X₃₉WTVX₄₀,
in welcher X₁₈ L oder V ist, X₁₉ L oder F ist, X₂₀ G oder S ist, X₂₁ C oder T ist, X₂₂ I oder V ist, X₂₃ I oder V ist, X₂₄ K, R oder T ist, X₂₅ Q oder E ist, X₂₆ V oder N ist, X₂₇ D, E oder C ist, X₂₈ V oder A ist, X₂₉ V, I, E oder M ist, X₃₀ L oder V ist, X₃₁ T, K oder A ist, X₃₂ Q oder H ist, X₃₃ S oder T ist, X₃₄ A oder G ist, X₃₅ T oder S ist, X₃₆ C oder A ist, X₃₇ V, I oder T ist, X₃₈ V oder A ist, X₃₉ C oder M ist und X₄₀ Y oder F ist,
und das höchstens die 63 Aminosäuren, wie sie in der folgenden Sequenz SEQ ID Nr. 46 beschrieben sind, hat:
AX₄₁ITX₄₂YX₄₃X₄₄QTRGX₁₈X₁₉X₂₀X₂₁X₂₂X₂₃TSLTGROX₂₄NX₂₅X₂₆X₂₇GEX₂₈ QX₂₉X₃₀STAX₃₁X₃₂X₃₃FLX₃₄X₃₅X₃₆X₃₇NGX₃₈X₃₉WTVX₄₀HGAGX₄₅X₄₆X₄₇,
in welcher X₁₈ bis X₄₀ wie in Anspruch 1 definiert sind und X₄₁ P, S oder H ist, X₄₂ A oder T ist, X₄₃ S, A, T oder C ist, X₄₄ Q oder R ist, X₄₅ S, T oder A ist, X₄₆ K oder R ist und X₄₇ T oder I ist,
- einem Peptid C, das zumindest die folgende Aminosäurensequenz SEQ ID Nr. 127 hat:
SX₄₇M X₄₈FTX₄₉X₅₀X₅₁TSPLX₅₂X₅₃X₅₄X₅₅TLX₅₆FNIX₅₇GGWVAX₅₈QX₅₉,
in welcher X₄₇ L oder P ist, X₄₈ A oder S ist, X₄₉ A oder S ist, X₅₀ A oder S ist, X₅₁ I oder V ist, X₅₂ T, S oder A ist, X₅₃ T oder I ist, X₅₄ Q, S oder G ist, X₅₅ N, Q, H, S, Y oder T ist, X₅₆ L oder M ist, X₅₇ L oder W ist, X₅₈ A oder S ist und X₅₉ L, P oder I ist,
und das höchstens 57 Aminosäuren, wie sie in der folgenden Sequenz SEQ ID Nr. 128 beschrieben sind, hat:
NFIX₆₀GX₆₁QYLAX₆₂LSTLPGNX₆₃AX₆₄X₆₅SX₄₇MX₄₈FTX₄₉X₅₀X₅₁TSPLX₅₂X ₅₃X₅₄X₅₅TLX₅₆FNIX₅₇GGWVAX₅₈QX₅₉X₆₆X₆₇X₆₈,
in welcher X₄₇ bis X₅₉ wie in Anspruch 1 definiert sind und X₆₀ T oder S ist, X₆₁ I, T oder V ist, X₆₂ G oder A ist, X₆₃ P oder L ist, X₆₄ I oder M ist, X₆₅ A, V oder R ist, X₆₆ A oder R ist, X₆₇ P, A oder D ist und X₆₈ P, A oder S ist,
- einem Peptid D, das zumindest die folgende Aminosäurensequenz SEQ ID Nr. 172 hat:
X₆₉KX₇₀ARX₇₁IVX₇₂PX₇₃LGX₇₄RVCEKX₇₅ALX₇₆X₇₇VX₇₈X₇₉X₈₀X₈₁,
in welcher X₆₉ R oder Q ist, X₇₀ P oder A ist, X₇₁ L oder F ist, X₇₂ F oder Y ist, X₇₃ D oder E ist, X₇₄ V oder S ist, X₇₅ M oder R ist, X₇₆ Y oder H ist, X₇₇ D oder N ist, X₇₈ V oder I ist, X₇₉ S, T oder K ist, X₈₀ T, K, I oder N ist und X₈₁ L oder T ist,
und das höchstens 44 Aminosäuren, wie sie in der folgenden Sequenz SEQ ID Nr. 175 beschrieben sind, hat:
KGGX₆₉KX₇₀ARX₇₁IVX₇₂PX₇₃LGX₇₄RVCEKX₇₅ALX₇₆X₇₇VX₇₈X₇₉X₈₀X₈₁X₈₂X ₈₃X₈₄VMG X₈₅X₈₆YX₈₇X₈₈Q,
in welcher X₆₉ bis X₈₁ wie in Anspruch 1 definiert sind und X₈₂ P oder A ist, X₈₃ Q, L, H, R, K oder P ist, X₈₄ A, T, V oder P ist, X₈₅ P, S oder A ist, X₈₆ S oder A ist, X₈₇ G oder R ist und X₈₈ F oder C ist,
- einem Epitop B', das die folgende Sequenz SEQ ID Nr. 213 hat:
GX₁₈X₁₉X₂₀X₂₁X₂₂X₂₃TSL,
in welcher X₁₈ L oder V ist, X₁₉ L oder F ist, X₂₀ G oder S ist, X₂₁ C oder T ist, X₂₂ I oder V ist und X₂₃ I oder V ist, und
- einem Epitop C', das die folgende Sequenz SEQ ID Nr. 221 hat:
SPLX₅₂X₅₃X₅₄X₅₅TL,
in welcher X₅₂ T, S oder A ist, X₅₃ T oder I ist, X₅₄ Q, S oder G ist und X₅₅ N, Q, H, S, Y oder T ist.

2. Peptidzusammensetzung nach Anspruch 1, **dadurch gekennzeichnet, dass** sie mindestens zwei Peptide, ausgewählt unter den Peptiden A, B, C, D, enthält.

3. Peptidzusammensetzung nach Anspruch 1 oder 2, **dadurch gekennzeichnet, dass** das Peptid A ausgewählt ist aus den folgenden Peptiden:
- den Peptiden, die zumindest die Sequenz SEQ ID Nr. 3 und höchstens die Sequenz SEQ ID Nr. 19 haben,
- den Peptiden, die zumindest die Sequenz SEQ ID Nr. 4 und höchstens eine Sequenz haben, die ausgewählt ist aus
i) der Sequenz SEQ ID Nr. 20,
ii) der Sequenz SEQ ID Nr. 24,
iii) der Sequenz SEQ ID Nr. 32 und
iv) der Sequenz SEQ ID Nr. 34,
- den Peptiden, die zumindest die Sequenz SEQ ID Nr. 5 und höchstens eine Sequenz haben, die ausgewählt ist aus
i) der Sequenz SEQ ID Nr. 21,
ii) der Sequenz SEQ ID Nr. 28, und
iii) der Sequenz SEQ ID Nr. 36,
- den Peptiden, die zumindest die Sequenz SEQ ID Nr. 6 und höchstens eine Sequenz haben, die ausgewählt ist aus
i) der Sequenz SEQ ID Nr. 22,
ii) der Sequenz SEQ ID Nr. 27 und
iii) der Sequenz SEQ ID Nr. 41,
- den Peptiden, die zumindest die Sequenz SEQ ID Nr. 7 und höchstens eine Sequenz haben, die ausgewählt ist aus
i) der Sequenz SEQ ID Nr. 23 und
ii) der Sequenz SEQ ID Nr. 37,
- den Peptiden, die zumindest die Sequenz SEQ ID Nr. 8 und höchstens die Sequenz SEQ ID Nr. 25 haben,
- den Peptiden, die zumindest die Sequenz SEQ ID Nr. 9 und höchstens die Sequenz SEQ ID Nr. 26 haben,
- den Peptiden, die zumindest die Sequenz SEQ ID Nr. 10 und höchstens die Sequenz SEQ ID Nr. 29 haben,
- den Peptiden, die zumindest die Sequenz SEQ ID Nr. 11 und höchstens die Sequenz SEQ ID Nr. 30 haben,
- den Peptiden, die zumindest die Sequenz SEQ ID Nr. 12 und höchstens die Sequenz SEQ ID Nr. 31 haben,
- den Peptiden, die zumindest die Sequenz SEQ ID Nr. 13 und höchstens die Sequenz SEQ ID Nr. 33 haben,
- den Peptiden, die zumindest die Sequenz SEQ ID Nr. 14 und höchstens eine Sequenz, ausgewählt aus
i) der Sequenz SEQ ID Nr. 35 und
ii) der Sequenz SEQ ID Nr. 39 haben,
- den Peptiden, die zumindest die Sequenz SEQ ID Nr. 15 und höchstens die Sequenz SEQ ID Nr. 38 haben,
- den Peptiden, die zumindest die Sequenz SEQ ID Nr. 16 und höchstens eine Sequenz, ausgewählt aus
i) der Sequenz SEQ ID Nr. 40 und
ii) der Sequenz SEQ ID Nr. 42 haben,
- den Peptiden, die zumindest die Sequenz SEQ ID Nr. 17 und höchstens die Sequenz SEQ ID Nr. 43 haben, und
- den Peptiden, die zumindest die Sequenz SEQ ID Nr. 18 und höchstens die Sequenz SEQ ID Nr. 44 haben.

4. Peptidzusammensetzung nach Anspruch 3, **dadurch gekennzeichnet, dass** das Peptid A ausgewählt ist unter den Peptiden der Sequenzen SEQ ID Nr. 3 bis 18.

5. Peptidzusammensetzung nach Anspruch 4, **dadurch gekennzeichnet, dass** das Peptid A die Sequenz SEQ ID Nr. 3 hat.

6. Peptidzusammensetzung nach einem der Ansprüche 1 bis 5, **dadurch gekennzeichnet, dass** das Peptid B ausgewählt ist aus:
- den Peptiden, die zumindest die Sequenz SEQ ID Nr. 47 und höchstens die Sequenz SEQ ID Nr. 81 haben,
- den Peptiden, die zumindest die Sequenz SEQ ID Nr. 48 und höchstens die Sequenz SEQ ID Nr. 82 haben,
- den Peptiden, die zumindest die Sequenz SEQ ID Nr. 49 und höchstens die Sequenz SEQ ID Nr. 83 haben,
- den Peptiden, die zumindest die Sequenz SEQ ID Nr. 50 und höchstens eine Sequenz haben, die ausgewählt ist aus:
i) der Sequenz SEQ ID Nr. 84,
ii) der Sequenz SEQ ID Nr. 90,
iii) der Sequenz SEQ ID Nr. 105,
iv) der Sequenz SEQ ID Nr. 107 und
v) der Sequenz SEQ ID Nr. 116,
- den Peptiden, die zumindest die Sequenz SEQ ID Nr. 51 und höchstens eine Sequenz haben, die ausgewählt ist aus:
i) der Sequenz SEQ ID Nr. 85 und
ii) der Sequenz SEQ ID Nr. 124,
- den Peptiden, die zumindest die Sequenz SEQ ID Nr. 52 und höchstens eine Sequenz haben, die ausgewählt ist aus:
i) der Sequenz SEQ ID Nr. 86 und
ii) der Sequenz SEQ ID Nr. 120,
- den Peptiden, die zumindest die Sequenz SEQ ID Nr. 53 und höchstens die Sequenz SEQ ID Nr. 87 haben,
- den Peptiden, die zumindest die Sequenz SEQ ID Nr. 54 und höchstens eine Sequenz haben, die ausgewählt ist aus:
i) der Sequenz SEQ ID Nr. 88 und
ii) der Sequenz SEQ ID Nr. 117,
- den Peptiden, die zumindest die Sequenz SEQ ID Nr. 55 und höchstens die Sequenz SEQ ID Nr. 89 haben,
- den Peptiden, die zumindest die Sequenz SEQ ID Nr. 56 und höchstens eine Sequenz haben, die ausgewählt ist aus:
i) der Sequenz SEQ ID Nr. 91 und
ii) der Sequenz SEQ ID Nr. 92,
- den Peptiden, die zumindest die Sequenz SEQ ID Nr. 57 und höchstens die Sequenz SEQ ID Nr. 93 haben,
- den Peptiden, die zumindest die Sequenz SEQ ID Nr. 58 und höchstens eine Sequenz haben, die ausgewählt ist aus:
i) der Sequenz SEQ ID Nr. 94 und
ii) der Sequenz SEQ ID Nr. 110,
- den Peptiden, die zumindest die Sequenz SEQ ID Nr. 59 und höchstens die Sequenz SEQ ID Nr. 95 haben,
- den Peptiden, die zumindest die Sequenz SEQ ID Nr. 60 und höchstens eine Sequenz haben, die ausgewählt ist aus:
i) der Sequenz SEQ ID Nr. 96 und
ii) der Sequenz SEQ ID Nr. 115,
- den Peptiden, die zumindest die Sequenz SEQ ID Nr. 61 und höchstens die Sequenz SEQ ID Nr. 97 haben,
- den Peptiden, die zumindest die Sequenz SEQ ID Nr. 62 und höchstens eine Sequenz haben, die ausgewählt ist aus:
i) der Sequenz SEQ ID Nr. 98 und
ii) der Sequenz SEQ ID Nr. 109,
- den Peptiden, die zumindest die Sequenz SEQ ID Nr. 63 und höchstens die Sequenz SEQ ID Nr. 99 haben,
- den Peptiden, die zumindest die Sequenz SEQ ID Nr. 64 und höchstens die Sequenz SEQ ID Nr. 100 haben,
- den Peptiden, die zumindest die Sequenz SEQ ID Nr. 65 und höchstens die Sequenz SEQ ID Nr. 101 haben,
- den Peptiden, die zumindest die Sequenz SEQ ID Nr. 66 und höchstens die Sequenz SEQ ID Nr. 102 haben,
- den Peptiden, die zumindest die Sequenz SEQ ID Nr. 67 und höchstens die Sequenz SEQ ID Nr. 103 haben,
- den Peptiden, die zumindest die Sequenz SEQ ID Nr. 68 und höchstens die Sequenz SEQ ID Nr. 104 haben,
- den Peptiden, die zumindest die Sequenz SEQ ID Nr. 69 und höchstens die Sequenz SEQ ID Nr. 106 haben,
- den Peptiden, die zumindest die Sequenz SEQ ID Nr. 70 und höchstens die Sequenz SEQ ID Nr. 108 haben,
- den Peptiden, die zumindest die Sequenz SEQ ID Nr. 71 und höchstens die Sequenz SEQ ID Nr. 111 haben,
- den Peptiden, die zumindest die Sequenz SEQ ID Nr. 72 und höchstens die Sequenz SEQ ID Nr. 112 haben,
- den Peptiden, die zumindest die Sequenz SEQ ID Nr. 73 und höchstens die Sequenz SEQ ID Nr. 113 haben,
- den Peptiden, die zumindest die Sequenz SEQ ID Nr. 74 und höchstens die Sequenz SEQ ID Nr. 114 haben,
- den Peptiden, die zumindest die Sequenz SEQ ID Nr. 75 und höchstens eine Sequenz haben, die ausgewählt ist aus:
i) der Sequenz SEQ ID Nr. 118 und
ii) der Sequenz SEQ ID Nr. 119,
- den Peptiden, die zumindest die Sequenz SEQ ID Nr. 76 und höchstens die Sequenz SEQ ID Nr. 121 haben,
- den Peptiden, die zumindest die Sequenz SEQ ID Nr. 77 und höchstens die Sequenz SEQ ID Nr. 122 haben,
- den Peptiden, die zumindest die Sequenz SEQ ID Nr. 78 und höchstens die Sequenz SEQ ID Nr. 123 haben,
- den Peptiden, die zumindest die Sequenz SEQ ID Nr. 79 und höchstens die Sequenz SEQ ID Nr. 125 haben, und
- den Peptiden, die zumindest die Sequenz SEQ ID Nr. 80 und höchstens die Sequenz SEQ ID Nr. 126 haben.

7. Peptidzusammensetzung nach Anspruch 6, **dadurch gekennzeichnet, dass** das Peptid B ausgewählt ist unter den Peptiden der Sequenzen SEQ ID Nr. 47 bis 80.

8. Peptidzusammensetzung nach Anspruch 7, **dadurch gekennzeichnet, dass** das Peptid B die Sequenz SEQ ID Nr. 47 hat.

9. Peptidzusammensetzung nach einem der Ansprüche 1 bis 8, **dadurch gekennzeichnet, dass** das Peptid C ausgewählt ist aus:
- den Peptiden, die zumindest die Sequenz SEQ ID Nr. 129 und höchstens eine Sequenz haben, die ausgewählt ist aus:
i) der Sequenz SEQ ID Nr. 147,
ii) der Sequenz SEQ ID Nr. 153,
iii) der Sequenz SEQ ID Nr. 162 und
iv) der Sequenz SEQ ID Nr. 167,
- den Peptiden, die zumindest die Sequenz SEQ ID Nr. 130 und höchstens eine Sequenz haben, die ausgewählt ist aus:
i) der Sequenz SEQ ID Nr. 148 und
ii) der Sequenz SEQ ID Nr. 150,
- den Peptiden, die zumindest die Sequenz SEQ ID Nr. 131 und höchstens die Sequenz SEQ ID Nr. 149 haben,
- den Peptiden, die zumindest die Sequenz SEQ ID Nr. 132 und höchstens eine Sequenz haben, die ausgewählt ist aus:
i) der Sequenz SEQ ID Nr. 151,
ii) der Sequenz SEQ ID Nr. 155,
iii) der Sequenz SEQ ID Nr. 168 und
iv) der Sequenz SEQ ID Nr. 171,
- den Peptiden, die zumindest die Sequenz SEQ ID Nr. 133 und höchstens die Sequenz SEQ ID Nr. 152 haben,
- den Peptiden, die zumindest die Sequenz SEQ ID Nr. 134 und höchstens eine Sequenz haben, die ausgewählt ist aus:
i) der Sequenz SEQ ID Nr. 154 und
ii) der Sequenz SEQ ID Nr. 169,
- den Peptiden, die zumindest die Sequenz SEQ ID Nr. 135 und höchstens die Sequenz SEQ ID Nr. 156 haben,
- den Peptiden, die zumindest die Sequenz SEQ ID Nr. 136 und höchstens eine Sequenz haben, die ausgewählt ist aus:
i) der Sequenz SEQ ID Nr. 157 und
ii) der Sequenz SEQ ID Nr. 170,
- den Peptiden, die zumindest die Sequenz SEQ ID Nr. 137 und höchstens die Sequenz SEQ ID Nr. 158 haben,
- den Peptiden, die zumindest die Sequenz SEQ ID Nr. 138 und höchstens die Sequenz SEQ ID Nr. 159 haben,
- den Peptiden, die zumindest die Sequenz SEQ ID Nr. 139 und höchstens die Sequenz SEQ ID Nr. 160 haben,
- den Peptiden, die zumindest die Sequenz SEQ ID Nr. 140 und höchstens die Sequenz SEQ ID Nr. 161 haben,
- den Peptiden, die zumindest die Sequenz SEQ ID Nr. 141 und höchstens die Sequenz SEQ ID Nr. 163 haben,
- den Peptiden, die zumindest die Sequenz SEQ ID Nr. 142 und höchstens die Sequenz SEQ ID Nr. 164 haben,
- den Peptiden, die zumindest die Sequenz SEQ ID Nr. 143 und höchstens die Sequenz SEQ ID Nr. 165 haben,
- den Peptiden, die zumindest die Sequenz SEQ ID Nr. 144 und höchstens die Sequenz SEQ ID Nr. 166 haben,
- den Peptiden, die zumindest die Sequenz SEQ ID Nr. 145 und höchstens die Sequenz SEQ ID Nr. 172 haben, und
- den Peptiden, die zumindest die Sequenz SEQ ID Nr. 146 und höchstens die Sequenz SEQ ID Nr. 173 haben,

10. Peptidzusammensetzung nach Anspruch 9, **dadurch gekennzeichnet, dass** das Peptid C ausgewählt ist unter den Peptiden der Sequenzen SEQ ID Nr. 129 bis 146.

11. Peptidzusammensetzung nach Anspruch 10, **dadurch gekennzeichnet, dass** das Peptid C die Sequenz ID Nr. 129 hat.

12. Peptidzusammensetzung nach einem der Ansprüche 1 bis 11, **dadurch gekennzeichnet, dass** das Peptid D ausgewählt aus:
- den Peptiden, die zumindest die Sequenz SEQ ID Nr. 176 und höchstens eine Sequenz haben, die ausgewählt ist aus:
i) der Sequenz SEQ ID Nr. 188,
ii) der Sequenz SEQ ID Nr. 192,
iii) der Sequenz SEQ ID Nr. 193,
iv) der Sequenz SEQ ID Nr. 194,
v) der Sequenz SEQ ID Nr. 201,
vi) der Sequenz SEQ ID Nr. 202,
vii) der Sequenz SEQ ID Nr. 203,
viii)der Sequenz SEQ ID Nr. 204,
ix) der Sequenz SEQ ID Nr. 205 und
x) der Sequenz SEQ ID Nr. 210,
- den Peptiden, die zumindest die Sequenz SEQ ID Nr. 177 und höchstens eine Sequenz haben, die ausgewählt ist aus:
i) der Sequenz SEQ ID Nr. 189 und
ii) der Sequenz SEQ ID Nr. 190,
- den Peptiden, die zumindest die Sequenz SEQ ID Nr. 178 und höchstens die Sequenz SEQ ID Nr. 191 haben,
- den Peptiden, die zumindest die Sequenz SEQ ID Nr. 179 und höchstens die Sequenz SEQ ID Nr. 195 haben,
- den Peptiden, die zumindest die Sequenz SEQ ID Nr. 180 und höchstens eine Sequenz haben, die ausgewählt ist aus:
i) der Sequenz SEQ ID Nr. 196 und
ii) der Sequenz SEQ ID Nr. 206,
- den Peptiden, die zumindest die Sequenz SEQ ID Nr. 181 und höchstens die Sequenz SEQ ID Nr. 197 haben,
- den Peptiden, die zumindest die Sequenz SEQ ID Nr. 182 und höchstens eine Sequenz haben, die ausgewählt ist aus:
i) der Sequenz SEQ ID Nr. 198 und
ii) der Sequenz SEQ ID Nr. 209,
- den Peptiden, die zumindest die Sequenz SEQ ID Nr. 183 und höchstens eine Sequenz haben, die ausgewählt ist aus:
i) der Sequenz SEQ ID Nr. 199 und
ii) der Sequenz SEQ ID Nr. 200,
- den Peptiden, die zumindest die Sequenz SEQ ID Nr. 184 und höchstens die Sequenz SEQ ID Nr. 207 haben,
- den Peptiden, die zumindest die Sequenz SEQ ID Nr. 185 und höchstens die Sequenz SEQ ID Nr. 208 haben,
- den Peptiden, die zumindest die Sequenz SEQ ID Nr. 186 und höchstens die Sequenz SEQ ID Nr. 211 haben,
- den Peptiden, die zumindest die Sequenz SEQ ID Nr. 187 und höchstens die Sequenz SEQ ID Nr. 212 haben.

13. Peptidzusammensetzung nach Anspruch 12, **dadurch gekennzeichnet, dass** das Peptid D ausgewählt ist unter den Peptiden der Sequenzen SEQ ID Nr. 176 bis 187.

14. Peptidzusammensetzung nach Anspruch 13, **dadurch gekennzeichnet, dass** das Peptid D die Sequenz SEQ ID Nr. 176 hat.

15. Peptidzusammensetzung nach einem der Ansprüche 1 bis 14, **dadurch gekennzeichnet, dass** sie die folgenden zwei Peptide enthält:
i) das Peptid A, wie es in einem der Ansprüche 1 und 3 bis 5 definiert ist, und ein anderes Peptid, das ausgewählt ist unter den Peptiden B bis D, wie sie in einem der Ansprüche 1 und 6 bis 14 definiert sind, oder
ii) das Peptid B, wie es in einem der Ansprüche 1 und 6 bis 8 definiert ist, und ein anderes Peptid, das ausgewählt ist unter den Peptiden A, C oder D, wie sie in einem der Ansprüche 1 und 3 bis 5 und 9 bis 14 definiert sind, oder
iii) das Peptid C, wie es in einem der Ansprüche 1 und 9 bis 11 definiert ist, und ein anderes Peptid, das ausgewählt ist unter den Peptiden A, B oder D, wie sie in einem der Ansprüche 1 und 3 bis 8 und 12 bis 14 definiert sind, oder
iv) das Peptid D, wie es in einem der Ansprüche 1 und 12 bis 14 definiert ist, und ein anderes Peptid, das ausgewählt ist unter den Peptiden A, B oder C, wie sie in einem der Ansprüche 1 und 3 bis 11 definiert sind.

16. Peptidzusammensetzung nach einem der Ansprüche 1 bis 14, **dadurch gekennzeichnet, dass** sie die drei folgenden Peptide umfasst:
i) das Peptid A, wie es in einem der Ansprüche 1 und 3 bis 5 definiert ist, und zwei andere Peptide, die ausgewählt sind unter den Peptiden B bis D, wie sie in einem der Ansprüche 1 und 6 bis 14 definiert sind, oder
ii) das Peptid B, wie es in einem der Ansprüche 1 und 6 bis 8 definiert ist, und zwei andere Peptide, die ausgewählt sind unter den Peptiden A, C oder D, wie sie in einem der Ansprüche 1 und 3 bis 5 und 9 bis 14 definiert sind, oder
iii) das Peptid C, wie es in einem der Ansprüche 1 und 9 bis 11 definiert ist, und zwei andere Peptide, die ausgewählt sind unter den Peptiden A, B oder D, wie sie in einem der Ansprüche 1 und 3 bis 8 und 12 bis 14 definiert sind, oder
iv) das Peptid D, wie es in einem der Ansprüche 1 und 12 bis 14 definiert ist, und zwei andere Peptide, die ausgewählt sind unter den Peptiden A, B oder C, wie sie in einem der Ansprüche 1 und 3 bis 11 definiert sind.

17. Peptidzusammensetzung nach einem der Ansprüche 1 bis 14, **dadurch gekennzeichnet, dass** sie die vier Peptide A, B, C, D umfasst, wie sie in einem der Ansprüche 1 und 3 bis 14 definiert sind.

18. Peptid A, das mindestens die Aminosäuresequenz SEQ ID Nr. 1 und höchstens die Sequenz SEQ ID Nr. 2 hat.

19. Peptid A nach Anspruch 18, **dadurch gekennzeichnet, dass** es ausgewählt ist aus:
- den Peptiden, die zumindest die Sequenz SEQ ID Nr. 3 und höchstens die Sequenz SEQ ID Nr. 19 haben,
- den Peptiden, die zumindest die Sequenz SEQ ID Nr. 4 und höchstens eine Sequenz haben, die ausgewählt ist aus den Sequenzen SEQ ID Nr. 20, SEQ ID Nr. 24, SEQ ID Nr. 32 und SEQ ID Nr. 34,
- den Peptiden, die zumindest die Sequenz SEQ ID Nr. 5 und höchstens eine Sequenz haben, die ausgewählt ist aus den Sequenzen SEQ ID Nr. 21, SEQ ID Nr. 28, und SEQ ID Nr. 36,
- den Peptiden, die zumindest die Sequenz SEQ ID Nr. 6 und höchstens eine Sequenz haben, die ausgewählt ist aus den Sequenzen SEQ ID Nr. 22, SEQ ID Nr. 27 und SEQ ID Nr. 41,
- den Peptide, die zumindest die Sequenz SEQ ID Nr. 7 und höchstens eine Sequenz haben, die ausgewählt ist aus den Sequenzen SEQ ID Nr. 23 und SEQ ID Nr. 37,
- den Peptiden, die zumindest die Sequenz SEQ ID Nr. 8 und höchstens die Sequenz SEQ ID Nr. 25 haben,
- den Peptiden, die zumindest die Sequenz SEQ ID Nr. 9 und höchstens die Sequenz SEQ ID Nr. 26 haben,
- den Peptiden, die zumindest die Sequenz SEQ ID Nr. 10 und höchstens die Sequenz SEQ ID Nr. 29 haben,
- den Peptiden, die zumindest die Sequenz SEQ ID Nr. 11 und höchstens die Sequenz SEQ ID Nr. 30 haben,
- den Peptiden, die zumindest die Sequenz SEQ ID Nr. 12 und höchstens die Sequenz SEQ ID Nr. 31 haben,
- den Peptiden, die zumindest die Sequenz SEQ ID Nr. 13 und höchstens die Sequenz SEQ ID Nr. 33 haben,
- den Peptiden, die zumindest die Sequenz SEQ ID Nr. 14 und höchstens eine Sequenz haben, die ausgewählt ist aus den Sequenzen SEQ ID Nr. 35 und SEQ ID Nr. 39,
- den Peptiden, die zumindest die Sequenz SEQ ID Nr. 15 und höchstens die Sequenz SEQ ID Nr. 38 haben,
- den Peptiden, die zumindest die Sequenz SEQ ID Nr. 16 und höchstens eine Sequenz haben, die ausgewählt ist aus den Sequenzen SEQ ID Nr. 40 und SEQ ID Nr. 42,
- den Peptiden, die zumindest die Sequenz SEQ ID Nr. 17 und höchstens die Sequenz SEQ ID Nr. 43 haben, und
- den Peptiden, die zumindest die Sequenz SEQ ID Nr. 18 und höchstens die Sequenz SEQ ID Nr. 44 haben.

20. Peptid A nach Anspruch 19, **dadurch gekennzeichnet, dass** es ausgewählt ist unter den Peptiden der Sequenzen SEQ ID Nr. 3 bis 18.

21. Peptid A nach Anspruch 20, **dadurch gekennzeichnet, dass** es die Sequenz SEQ ID Nr. 3 hat.

22. Peptid B, das mindestens die Aminosäuresequenz SEQ ID Nr. 45 und höchstens die Sequenz SEQ ID Nr. 46 hat.

23. Peptid B nach Anspruch 22, **dadurch gekennzeichnet, dass** es ausgewählt ist aus:
- den Peptiden, die zumindest die Sequenz SEQ ID Nr. 47 und höchstens die Sequenz SEQ ID Nr. 81 haben,
- den Peptiden, die zumindest die Sequenz SEQ ID Nr. 48 und höchstens die Sequenz SEQ ID Nr. 82 haben,
- den Peptiden, die zumindest die Sequenz SEQ ID Nr. 49 und höchstens die Sequenz SEQ ID Nr. 83 haben,
- den Peptiden, die zumindest die Sequenz SEQ ID Nr. 50 und höchstens eine Sequenz haben, die ausgewählt ist aus den Sequenzen SEQ ID Nr. 84, SEQ ID Nr. 90, SEQ ID Nr. 105, SEQ ID Nr. 107 und SEQ ID Nr. 116,
- den Peptiden, die zumindest die Sequenz SEQ ID Nr. 51 und höchstens eine Sequenz haben, die ausgewählt ist aus den Sequenzen SEQ ID Nr. 85 und SEQ ID Nr. 124,
- den Peptiden, die zumindest die Sequenz SEQ ID Nr. 52 und höchstens eine Sequenz haben, die ausgewählt ist aus den Sequenzen SEQ ID Nr. 86 und SEQ ID Nr. 120,
- den Peptiden, die zumindest die Sequenz SEQ ID Nr. 53 und höchstens die Sequenz SEQ ID Nr. 87 haben,
- den Peptiden, die zumindest die Sequenz SEQ ID Nr. 54 und höchstens eine Sequenz haben, die ausgewählt ist aus den Sequenzen SEQ ID Nr. 88 und SEQ ID Nr. 117,
- den Peptiden, die zumindest die Sequenz SEQ ID Nr. 55 und höchstens die Sequenz SEQ ID Nr. 89 haben,
- den Peptiden, die zumindest die Sequenz SEQ ID Nr. 56 und höchstens eine Sequenz haben, die ausgewählt ist aus den Sequenzen SEQ ID Nr. 91 und SEQ ID Nr. 92,
- den Peptiden, die zumindest die Sequenz SEQ ID Nr. 57 und höchstens die Sequenz SEQ ID Nr. 93 haben,
- den Peptiden, die zumindest die Sequenz SEQ ID Nr. 58 und höchstens eine Sequenz haben, die ausgewählt ist aus den Sequenzen SEQ ID Nr. 94 und SEQ ID Nr. 110,
- den Peptiden, die zumindest die Sequenz SEQ ID Nr. 59 und höchstens die Sequenz SEQ ID Nr. 95 haben,
- den Peptiden, die zumindest die Sequenz SEQ ID Nr. 60 und höchstens eine Sequenz haben, die ausgewählt ist aus den Sequenzen SEQ ID Nr. 96 und SEQ ID Nr. 115,
- den Peptiden, die zumindest die Sequenz SEQ ID Nr. 61 und höchstens die Sequenz SEQ ID Nr. 97 haben,
- den Peptiden, die zumindest die Sequenz SEQ ID Nr. 62 und höchstens eine Sequenz haben, die ausgewählt ist aus den Sequenzen SEQ ID Nr. 98 und SEQ ID Nr. 109,
- den Peptiden, die zumindest die Sequenz SEQ ID Nr. 63 und höchstens die Sequenz SEQ ID Nr. 99 haben,
- den Peptiden, die zumindest die Sequenz SEQ ID Nr. 64 und höchstens die Sequenz SEQ ID Nr. 100 haben,
- den Peptiden, die zumindest die Sequenz SEQ ID Nr. 65 und höchstens die Sequenz SEQ ID Nr. 101 haben,
- den Peptiden, die zumindest die Sequenz SEQ ID Nr. 66 und höchstens die Sequenz SEQ ID Nr. 102 haben,
- den Peptiden, die zumindest die Sequenz SEQ ID Nr. 67 und höchstens die Sequenz SEQ ID Nr. 103 haben,
- den Peptiden, die zumindest die Sequenz SEQ ID Nr. 68 und höchstens die Sequenz SEQ ID Nr. 104 haben,
- den Peptiden, die zumindest die Sequenz SEQ ID Nr. 69 und höchstens die Sequenz SEQ ID Nr. 106 haben,
- den Peptiden, die zumindest die Sequenz SEQ ID Nr. 70 und höchstens die Sequenz SEQ ID Nr. 108 haben,
- den Peptiden, die zumindest die Sequenz SEQ ID Nr. 71 und höchstens die Sequenz SEQ ID Nr. 111 haben,
- den Peptiden, die zumindest die Sequenz SEQ ID Nr. 72 und höchstens die Sequenz SEQ ID Nr. 112 haben,
- den Peptiden, die zumindest die Sequenz SEQ ID Nr. 73 und höchstens die Sequenz SEQ ID Nr. 113 haben,
- den Peptiden, die zumindest die Sequenz SEQ ID Nr. 74 und höchstens die Sequenz SEQ ID Nr. 114 haben,
- den Peptiden, die zumindest die Sequenz SEQ ID Nr. 75 und höchstens eine Sequenz haben, die ausgewählt ist aus den Sequenzen SEQ ID Nr. 118 und SEQ ID Nr. 119,
- den Peptiden, die zumindest die Sequenz SEQ ID Nr. 76 und höchstens die Sequenz SEQ ID Nr. 121 haben,
- den Peptiden, die zumindest die Sequenz SEQ ID Nr. 77 und höchstens die Sequenz SEQ ID Nr. 122 haben,
- den Peptiden, die zumindest die Sequenz SEQ ID Nr. 78 und höchstens die Sequenz SEQ ID Nr. 123 haben,
- den Peptiden, die zumindest die Sequenz SEQ ID Nr. 79 und höchstens die Sequenz SEQ ID Nr. 125 haben, und
- den Peptiden, die zumindest die Sequenz SEQ ID Nr. 80 und höchstens die Sequenz SEQ ID Nr. 126 haben.

24. Peptid B nach Anspruch 23, **dadurch gekennzeichnet, dass** es ausgewählt ist unter den Peptiden der Sequenzen SEQ ID Nr. 47 bis 80.

25. Peptid B nach Anspruch 24, **dadurch gekennzeichnet, dass** es die Sequenz SEQ ID Nr. 47 hat.

26. Peptid C, das mindestens die Sequenz SEQ ID Nr. 127 und höchstens die Sequenz SEQ ID Nr. 128 hat.

27. Peptid C nach Anspruch 26, **dadurch gekennzeichnet, dass** es ausgewählt ist aus:
- den Peptiden, die zumindest die Sequenz SEQ ID Nr. 129 und höchstens eine Sequenz haben, die ausgewählt ist aus den Sequenzen SEQ ID Nr. 147, SEQ ID Nr. 153, SEQ ID Nr. 162 und SEQ ID Nr. 167,
- den Peptiden, die zumindest die Sequenz SEQ ID Nr. 130 und höchstens eine Sequenz haben, die ausgewählt ist aus den Sequenzen SEQ ID Nr. 148 und SEQ ID Nr. 150,
- den Peptiden, die zumindest die Sequenz SEQ ID Nr. 131 und höchstens die Sequenz SEQ ID Nr. 149 haben,
- den Peptiden, die zumindest die Sequenz SEQ ID Nr. 132 und höchstens eine Sequenz haben, die ausgewählt ist aus den Sequenzen SEQ ID Nr. 151, SEQ ID Nr. 155, SEQ ID Nr. 168 und SEQ ID Nr. 171,
- den Peptiden, die zumindest die Sequenz SEQ ID Nr. 133 und höchstens die Sequenz SEQ ID Nr. 152 haben,
- den Peptiden, die zumindest die Sequenz SEQ ID Nr. 134 und höchstens eine Sequenz haben, die ausgewählt ist aus den Sequenzen SEQ ID Nr. 154 und SEQ ID Nr. 169,
- den Peptiden, die zumindest die Sequenz SEQ ID Nr. 135 und höchstens die Sequenz SEQ ID Nr. 156 haben,
- den Peptiden, die zumindest die Sequenz SEQ ID Nr. 136 und höchstens eine Sequenz haben, die ausgewählt ist aus den Sequenzen SEQ ID Nr. 157 und SEQ ID Nr. 170,
- den Peptiden, die zumindest die Sequenz SEQ ID Nr. 137 und höchstens die Sequenz SEQ ID Nr. 158 haben,
- den Peptiden, die zumindest die Sequenz SEQ ID Nr. 138 und höchstens die Sequenz SEQ ID Nr. 159 haben,
- den Peptiden, die zumindest die Sequenz SEQ ID Nr. 139 und höchstens die Sequenz SEQ ID Nr. 160 haben,
- den Peptiden, die zumindest die Sequenz SEQ ID Nr. 140 und höchstens die Sequenz SEQ ID Nr. 161 haben,
- den Peptiden, die zumindest die Sequenz SEQ ID Nr. 141 und höchstens die Sequenz SEQ ID Nr. 163 haben,
- den Peptiden, die zumindest die Sequenz SEQ ID Nr. 142 und höchstens die Sequenz SEQ ID Nr. 164 haben,
- den Peptiden, die zumindest die Sequenz SEQ ID Nr. 143 und höchstens die Sequenz SEQ ID Nr. 165 haben,
- den Peptiden, die zumindest die Sequenz SEQ ID Nr. 144 und höchstens die Sequenz SEQ ID Nr. 166 haben,
- den Peptiden, die zumindest die Sequenz SEQ ID Nr. 145 und höchstens die Sequenz SEQ ID Nr. 172 haben, und
- den Peptiden, die zumindest die Sequenz SEQ ID Nr. 146 und höchstens die Sequenz SEQ ID Nr. 173 haben.

28. Peptid C nach Anspruch 27, **dadurch gekennzeichnet, dass** es ausgewählt ist unter den Sequenzen SEQ ID Nr. 129 bis 146.

29. Peptid C nach Anspruch 28, **dadurch gekennzeichnet, dass** es die Sequenz SEQ ID Nr. 129 hat.

30. Peptid D, das mindestens die Sequenz SEQ ID Nr. 174 und höchstens die Sequenz SEQ ID Nr. 176 hat.

31. Peptid D nach Anspruch 30, **dadurch gekennzeichnet, dass** es ausgewählt ist aus:
- den Peptiden, die zumindest die Sequenz SEQ ID Nr. 176 und höchstens eine Sequenz haben, die ausgewählt ist aus den Sequenzen SEQ ID Nr. 188, SEQ ID Nr. 192, SEQ ID Nr. 193, SEQ ID Nr. 194, SEQ ID Nr. 201, SEQ ID Nr. 202, SEQ ID Nr. 203, SEQ ID Nr. 204, SEQ ID Nr. 205 und SEQ ID Nr. 210,
- den Peptiden, die zumindest die Sequenz SEQ ID Nr. 177 und höchstens eine Sequenz haben, die ausgewählt ist aus den Sequenzen SEQ ID Nr. 189 und SEQ ID Nr. 190,
- den Peptiden, die zumindest die Sequenz SEQ ID Nr. 178 und höchstens die Sequenz SEQ ID Nr. 191 haben,
- den Peptiden, die zumindest die Sequenz SEQ ID Nr. 179 und höchstens die Sequenz SEQ ID Nr. 195 haben,
- den Peptiden, die zumindest die Sequenz SEQ ID Nr. 180 und höchstens eine Sequenz haben, die ausgewählt ist aus den Sequenzen SEQ ID Nr. 196 und SEQ ID Nr. 206,
- den Peptiden, die zumindest die Sequenz SEQ ID Nr. 181 und höchstens die Sequenz SEQ ID Nr. 197 haben,
- den Peptiden, die zumindest die Sequenz SEQ ID Nr. 182 und höchstens eine Sequenz haben, die ausgewählt ist aus den Sequenzen SEQ ID Nr. 198 und SEQ ID Nr. 209,
- den Peptiden, die zumindest die Sequenz SEQ ID Nr. 183 und höchstens eine Sequenz haben, die ausgewählt ist aus den Sequenzen SEQ ID Nr. 199 und SEQ ID Nr. 200,
- den Peptiden, die zumindest die Sequenz SEQ ID Nr. 184 und höchstens die Sequenz SEQ ID Nr. 207 haben,
- den Peptiden, die zumindest die Sequenz SEQ ID Nr. 185 und höchstens die Sequenz SEQ ID Nr. 208 haben,
- den Peptiden, die zumindest die Sequenz SEQ ID Nr. 186 und höchstens die Sequenz SEQ ID Nr. 211 haben,
- den Peptiden, die zumindest die Sequenz SEQ ID Nr. 187 und höchstens die Sequenz SEQ ID Nr. 212 haben.

32. Peptid D nach Anspruch 31, **dadurch gekennzeichnet, dass** es ausgewählt ist unter den Peptiden der Sequenzen SEQ ID Nr. 176 bis 187.

33. Peptid D, nach Anspruch 32, **dadurch gekennzeichnet, dass** es die Sequenz SEQ ID Nr. 176 hat.

34. Epitop B', **dadurch gekennzeichnet, dass** es ausgewählt ist aus den Epitopen der Sequenzen SEQ ID Nr. 215 bis SEQ ID Nr. 220.

35. Epitop C', **dadurch gekennzeichnet, dass** es ausgewählt ist aus den Epitopen der Sequenzen SEQ ID Nr. 223 bis SEQ ID Nr. 232.

36. Nukleotidsequenzen, die für eines der Peptide A bis D kodieren, wie sie in einem der Ansprüche 18 bis 33 definiert sind, oder für eines der Epitope B' und C' kodieren, wie sie in den Ansprüchen 34 und 35 definiert sind.

37. Expressionsvektor, **dadurch gekennzeichnet, dass** er eine Nukleotidsequenz nach Anspruch 36 enthält, sowie für seine Expression notwendige Mittel.

38. Expressionsvektor, **dadurch gekennzeichnet, dass** er mindestens zwei Nukleotidsequenzen enthält, die für die Peptide A bis D, wie sie in den Ansprüchen 18 bis 33 definiert sind, kodieren, sowie die notwendigen Mittel für ihre Expression, wobei jede Nukleotidsequenz für ein Verschiedenes Peptid kodiert, wobei die mindestens zwei Nukleotidsequenzen direkt untereinander oder auch über Spacer, die durch Aminosäuren mit einer neutralen Ladung unter physiologischen Bedingungen und mit einer Größe von 3 bis 6 Resten gebildet werden, verbunden sind.

39. Expressionsvektor nach Anspruch 38, **dadurch gekennzeichnet, dass** die beiden Nukleotidsequenzen für die Peptide A und B, A und C, A und D, B und C, B und D oder C und D kodieren.

40. Expressionsvektor nach Anspruch 38, **dadurch gekennzeichnet, dass** er drei Nukleotidsequenzen enthält, die für die Peptide A, B und C, A, B und D, A, C und D oder B, C und D kodieren.

41. Expressionsvektor nach Anspruch 38, **dadurch gekennzeichnet, dass** er vier Nukleotidsequenzen enthält, die für die Peptide A bis D kodieren.

42. Expressionsvektor nach Anspruch 37, **dadurch gekennzeichnet, dass** er mindestens eine Nukleotidsequenz enthält, die für ein Epitop B' oder C', wie sie in einem der Ansprüche 34 und 35 definiert sind, kodiert.

43. Expressionsvektor nach Anspruch 37, **dadurch gekennzeichnet, dass** er zwei bis vier Nukleotidsequenzen enthält, die aus jenen ausgewählt sind, die für die Peptide A, B, C, D und die Epitope B'und C' kodieren, wie sie in den Ansprüchen 18 bis 35 definiert sind, wobei:
- die Nukleotidsequenz, die für das Peptid B kodiert, und die Nukleotidsequenz, die für das Epitop B' kodiert, nicht gleichzeitig vorhanden sind, und
- die Nukleotidsequenz, die für das Peptid C kodiert, und die Nukleotidsequenz, die für das Epitop C' kodiert, nicht gleichzeitig vorhanden sind,
wobei die zwei bis vier Nukleotidsequenzen direkt untereinander verbunden sind, oder über Spacer, die durch Aminosäuren, die eine neutrale Ladung unter physiologischen Bedingungen und eine Größe von 3 bis 6 Resten haben, gebildet werden.

44. Mikroorganismus oder Wirtszelle, der bzw. die durch einen Expressionsvektor, wie er bzw. sie in den Ansprüchen 37 bis 43 definiert ist, transformiert ist.

45. Mikroorganismus oder Wirtszelle, der bzw. die durch mindestens zwei Expressionsvektoren, wie sie in Anspruch 37 definiert sind, kotransformiert ist, wobei jeder Expressionsvektor für ein unterschiedliches Peptid kodiert.

46. Mikroorganismus oder Wirtszelle nach Anspruch 45, **dadurch gekennzeichnet, dass** er bzw. sie mit zwei Expressionsvektoren, die jeweils für die Peptide A und B, A und C, A und D, B und C, B und D oder C und D kodieren, kotransformiert ist.

47. Mikroorganismus oder Wirtszelle nach Anspruch 45, **dadurch gekennzeichnet, dass** er bzw. sie mit drei Expressionsvektoren, die jeweils für die Peptide A, B und C, A B und D, A, C und D oder B, C und D kodieren, kotransformiert ist.

48. Mikroorganismus oder Wirtszelle nach Anspruch 45, **dadurch gekennzeichnet, dass** er bzw. sie mit vier Vektoren, die jeweils für die Peptide A, B, C und D kodieren, kotransformiert ist.

49. Antikörper, die gegen mindestens eines der Peptide A bis D, wie sie in den Ansprüchen 18 bis 33 definiert sind, oder gegen eine der Zusammensetzungen, wie sie in einem der Ansprüche 1 bis 17 definiert sind oder auch gegen mindestens eines der Epitope B' und C', wie sie in einem der Ansprüche 34 und 35 definiert sind, gerichtet sind.

50. Verwendung einer Zusammensetzung nach einem der Ansprüche 1 bis 17 für die Herstellung eines Medikaments für die Inhibierung, die Präventation oder die Behandlung einer Infektion, die durch das Hepatitis C-Virus hervorgerufen wurde, bei einem Tier, vorzugsweise dem Menschen.

51. Verwendung von zumindest einem der Epitope B' oder C', wie sie in den Ansprüchen 34 und 35 definiert wurden, für die Herstellung eines Medikaments für die Inhibierung, die Prävention oder die Behandlung einer Infektion, die durch das Hepatitis C-Virus hervorgerufen wurde, bei einem Tier, vorzugsweise dem Menschen.

52. Pharmazeutische Zusammensetzung, insbesondere Impfstoff, der als aktiven Wirkstoff mindestens eine Peptidzusammensetzung nach einem der Ansprüche 1 bis 16, oder auch mindestens zwei Nukleotidsequenzen nach Anspruch 36, die für verschiedene Peptide ausgewählt unter den Peptiden A bis D kodieren, hat, die unter die Kontrolle von notwendigen Elementen einer konstitutiven und/oder induzierbaren Expression der genannten Peptide A bis D gestellt werden, oder auch mindestens einen der Antikörper nach Anspruch 49, oder auch noch mindestens eines der Epitope B' und C', wie sie in Anspruch 34 und 35 definiert sind, oder auch mindestens einer ihrer Nukleotidsequenzen, wie sie in Anspruch 36 definiert ist, in Verbindung mit einem pharmazeutisch akzeptablen Träger enthält.

53. Diagnostische Zusammensetzung für das Auffinden und/oder die Quantifizierung des Hepatitis C-Virus, die zumindest eine Peptidzusammensetzung nach einem der Ansprüche 1 bis 16, oder mindestens einen Antikörper nach Anspruch 49 oder auch mindestens ein Epitop B' oder C' nach einem der Ansprüche 34 und 35 enthält.

54. Verfahren zur Auffindung und/oder Quantifizierung des Hepatitis C-Virus in einer biologischen Probe, die von einem Individuum abgenommen wurde, das verdächtigt wird, mit dem Virus infiziert zu sein, wie Plasma, Serum oder Gewebe, **dadurch gekennzeichnet, dass** es die Schritte enthält, die bestehen aus:
- in Kontakt bringen der biologischen Probe mit den Antikörpern nach dem Anspruch 49, unter Bedingungen, die die Ausbildung eines Komplexes zwischen dem Virus und dem Antikörper erlauben,
- Auffinden und/oder Quantifizieren der Ausbildung dieses Komplexes durch alle geeigneten Mittel.

55. Verwendung der Zusammensetzung nach Anspruch 53 für die *in vitro* Diagnostik des Hepatitis C-Virus in einer biologischen Probe oder Abnahme.


### SEQUENCE LISTING

<110> BIOMERIEUX
   INSERM
<120> Composition peptidique et son utilisation dans la vaccination et le diagnostic du VHC
<130> EpitoVHC
<150> FR02/06111 <151> 2002-05-17
<160> 232
<170> PatentIn version 3.1
<210> 1
   <211> 31
   <212> PRT
   <213> séquence artificielle
<220>
   <221> MISC_FEATURE
   <222> (1) .. (1)
   <223> Y our H
<220>
   <221> MISC_FEATURE
   <222> (3)..(3).
   <223> A, G or T
<220>
   <221> MISC_FEATURE
   <222> (9)..(9)
   <223> R ou L
<220>
   <221> MISC_FEATURE
   <222> (16)..(16)
   <223> A ou T
<220>
   <221> MISC_FEATORE
   <222> (20)..(20)
   <223> G ou S
<220>
   <221> MISC_FEATURE
   <222> (23)..(23)
   <223> A, T ou V
<220>
   <221> MISC_FEATURE
   <222> (29)..(29)
   <223> H ou Y
<220>
   <221> MISC_FEATURE
   <222> (31)..(31)
   <223> I, T, M ou V
<400> 1
   Xaa Ala Xaa Gln Gly Tyr Lys Val Xaa Val Leu Asn Pro Ser Val Xaa
   1               5                   10                  15
   Ala Thr Leu Xaa Phe Gly Xaa Tyr Met Ser Lys Ala Xaa Gly Xaa
               20                  25                  30
<210> 2
   <211> 46
   <212> PRT
   <213> séquence artificielle
<220>
   <221> MISC_FEATURE
   <222> (2)..(2)
   <223> T ou N
<220>
   <221> MISC_FEATURE
   <222> (3) **..** (3)
   <223> K ou R
<220>
   <221> MISC_FEATURE
   <222> (6)..(6)
   <223> A ou V
<220>
   <221> MISC_FEATURE
   <222> (7)..(7)
   <223> A ou E
<220>
   <221> MISC_FEATURE
   <222> (8) .. (8)
   <223> Y ou H
<220>
   <221> MISC_FEATURE
   <222> (10) .. (10)
   <223> Y ou H
<220>
   <221> MISC_FEATURE
   <222> (10)..(10)
   <223> A, G ou T
<220>
   <221> MISC_FEATURE
   <222> (16) .. (16)
   <223> R ou L
<220>
   <221> MISC_FEATURE
   <222> (23).. (23)
   <223> A ou T
<220>
   <221> MISC_FEATURE
   <222> (27)..(27)
   <223> G ou S
<220>
   <221> MISC_FEATURE
   <222> (30).. (30)
   <223> A, T ou V
<220>
   <221> MISC-FEATURE
   <222> (36)..(36)
   <223> H ou Y
<220>
   <221> MISC_FEATURE
   <222> (38)..(38)
   <223> I, T, M ou V
<220>
   <221> MISC_FEATURE
   <222> (39).. (39)
   <223> E ou D
<220>
   <221> MISC_PEATURE
   <222> (41)..(41)
   <223> N ou S
<220>
   <221> MISC_FEATURE
   <222> (42) .. (42)
   <223> I, L ou V
<220>
   <221> MISC_FEATURE
   <222> (43)..(43)
   <223> R ou S
<220>
   <221> MISC_FEATURE
   <222> (44)..(44)
   <223> T ou S
<400> 2
   Ser Xaa Xaa Val Pro Xaa Xaa Xaa Ala Xaa Gln Gly Tyr Lys Val Xaa
   1               5                   10                  15
   Val Leu Asn Pro Ser Val Xaa Ala Thr Leu Xaa Phe Gly Xaa Tyr Met
               20                  25                  30
   Ser Lys Ala Xaa Gly Xaa Xaa Pro Xaa Xaa Xaa Xaa Gly Val
           35                  40                  45
<210> 3
   <211> 31
   <212> PRT
   <213> séquence artificielle
<400> 3
   Tyr Ala Ala Gln Gly Tyr Lys Val Arg Val Leu Asn Pro Ser Val Ala
   1 5 10 15
   Ala Thr Leu Gly Phe Gly Ala Tyr Met Ser Lys Ala His Gly Ile
   20 25 30
<210> 4
   <211> 31
   <212> PRT
   <213> séquence artificielle
<400> 4
   Tyr Ala Ala Gln Gly Tyr Lys Val Leu Val Leu Asn Pro Ser Val Ala
   1 5 10 15
   Ala Thr Leu Gly Phe Gly Ala Tyr Met Ser Lys Ala His Gly Ile
   20 25 30
<210> 5
   <211> 31
   <212> PRT
   <213> séquence artificielle
<400> 5
   Tyr Ala Ala Gln Gly Tyr Lys Val Leu Val Leu Asn Pro Ser Val Ala
   1 5 10 15
   Ala Thr Leu Gly Phe Gly Ala Tyr Met Ser Lys Ala His Gly Val
   20 25 30
<210> 6
   <211> 31
   <212> PRT
   <213> séquence artificielle
<400> 6
   Tyr Ala Ala Gln Gly Tyr Lys Val Leu Val Leu Asn Pro Ser Val Ala
   1 5 10 15
   Ala Thr Leu Gly Phe Gly Ala Tyr Met Ser Lys Ala His Gly Thr
   20 25 30
<210> 7
   <211> 31
   <212> PRT
   <213> séquence artificielle
<400> 7
   Tyr Ala Ala Gln Gly Tyr Lys Val Leu Val Leu Asn Pro Ser Val Ala
   1 5 10 15
   Ala Thr Leu Gly Phe Gly Ala Tyr Met Ser Lys Ala Tyr Gly Thr
   20 25 30
<210> 8
   <211> 31
   <212> PRT
   <213> séquence artificielle
<400> 8
   Tyr Ala Ala Gln Gly Tyr Lys Val Leu Val Leu Asn Pro Ser Val Ala
   1 5 10 15
   Ala Thr Leu Ser Phe Gly Ala Tyr Met Ser Lys Ala His Gly Thr
   20 25 30
<210> 9
   <211> 31
   <212> PRT
   <213> séquence artificielle
<400> 9
   Tyr Ala Ala Gln Gly Tyr Lys Val Leu Val Leu Asn Pro Ser Val Ala
   1 5 10 15
   Ala Thr Leu Gly Phe Gly Ala Tyr Met Ser Lys Ala Tyr Gly Ile
   20 25 30

<210> 10
   <211> 31
   <212> PRT
   <213> séquence artificielle
<400> 10
   Tyr Ala Ala Gln Gly Tyr Lys Val Leu Val Leu Asn Pro Ser Val Thr
   1 5 10 15
   Ala Thr Leu Gly Phe Gly Ala Tyr Met Ser Lys Ala His Gly Val
   20 25 30
<210> 11
   <211> 31
   <212> PRT
   <213> séquence artificielle
<400> 11
   Tyr Ala Gly Gln Gly Tyr Lys Val Leu Val Leu Asn Pro Ser Val Ala
   1 5 10 15
   Ala Thr Leu Gly Phe Gly Ala Tyr Met Ser Lys Ala His Gly Ile
   20 25 30
<210> 12
   <211> 31
   <212> PRT
   <213> séquence artificielle
<400> 12
   Tyr Ala Ala Gln Gly Tyr Lys Val Leu Val Leu Asn Pro Ser Val Ala
   1 5 10 15
   Ala Thr Leu Ser Phe Gly Ala Tyr Met Ser Lys Ala His Gly Ile
   20 25 30
<210> 13
   <211> 31
   <212> PRT
   <213> séquence artificielle
<400> 13
   Tyr Ala Thr Gln Gly Tyr Lys Val Leu Val Leu Asn Pro Ser Val Ala
   1               5                   10                  15
   Ala Thr Leu Gly Phe Gly Ala Tyr Met Ser Lys Ala His Gly Thr
               20                  25                  30
<210> 14
   <211> 31
   <212> PRT
   <213> séquence artificielle
<400> 14
   Tyr Ala Ala Gln Gly Tyr Lys Val Leu Val Leu Asn Pro Ser Val Ala
   1               5                   10                  15
   Ala Thr Leu Ser Phe Gly Ala Tyr Met Ser Lys Ala His Gly Val
               20                  25                  30
<210> 15
   <211> 31
   <212> PRT
   <213> séquence artificielle
<400> 15
   Tyr Ala Thr Gln Gly Tyr Lys Val Leu Val Leu Asn Pro Ser Val Ala
   1               5                   10                  15
   Ala Thr Leu Ser Phe Gly Ala Tyr Met Ser Lys Ala Tyr Gly Met
               20                  25                  30
<210> 16
   <211> 31
   <212> PRT
   <213> séquence artificielle
<400> 16
   Tyr Ala Ala Gln Gly Tyr Lys Val Leu Val Leu Asn Pro Ser Val Ala
   1 5 10 15
   Ala Thr Leu Ser Phe Gly Ala Tyr Met Ser Lys Ala Tyr Gly Val
   20 25 30
<210> 17
   <211> 31
   <212> PRT
   <213> séquence artificielle
<400> 17
   Tyr Ala Ala Gln Gly Tyr Lys Val Leu Val Leu Asn Pro Ser Val Ala
   1 5 10 15
   Ala Thr Leu Gly Phe Gly Thr Tyr Met Ser Lys Ala Tyr Gly Thr
   20 25 30
<210> 18
   <211> 31
   <212> PRT
   <213> séquence artificielle
<400> 18
   His Ala Ala Gln Gly Tyr Lys Val Leu Val Leu Asn Pro Ser Val Ala
   1 5 10 15
   Ala Thr Leu Gly Phe Gly Val Tyr Met Ser Lys Ala Tyr Gly Ile
   20 25 30
<210> 19
   <211> 46
   <212> PRT
   <213> séquence artificielle
<400> 19
   Ser Thr Lys Val Pro Ala Ala Tyr Ala Ala Gln Gly Tyr Lys Val Arg
   1 5 10 15
   Val Leu Asn Pro Ser Val Ala Ala Thr Leu Gly Phe Gly Ala Tyr Met
   20 25 30
   Ser Lys Ala His Gly Ile Glu Pro Asn Ile Arg Thr Gly Val
   35 40 45
<210> 20
   <211> 46
   <212> PRT
   <213> séquence artificielle
<400> 20
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Ile Asp Pro Asn Ile Arg Thr Gly Val
   35 40 45
<210> 21
   <211> 46
   <212> PRT
   <213> séquence artificielle
<400> 21
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Val Asp Pro Asn Ile Arg Thr Gly Val
   35 40 45
<210> 22
   <211> 46
   <212> PRT
   <213> séquence artificielle
<400> 22
   Ser Thr Lys Val Pro Ala Ala Tyr Ala Ala Gln Gly Tyr Lys Val Leu
   1               5                   10                  15
   Val Leu Asn Pro Ser Val Ala Ala Thr Leu Gly Phe Gly Ala Tyr Met
               20                  25                  30
   Ser Lys Ala His Gly Thr Asp Pro Asn Ile Arg Thr Gly Val
           35                  40                  45
<210> 23
   <211> 46
   <212> PRT
   <213> séquence artificielle
<400> 23
   Ser Thr Lys Val Pro Ala Ala Tyr Ala Ala Gln Gly Tyr Lys Val Leu
   1               5                   10                  15
   Val Leu Asn Pro Ser Val Ala Ala Thr Leu Gly Phe Gly Ala Tyr Met
               20                  25                  30
   Ser Lys Ala Tyr Gly Thr Asp Pro Asn Ile Arg Thr Gly Val
           35                  40                  45
<210> 24
   <211> 46
   <212> PRT
   <213> séquence aartificielle
<400> 24
   Ser Thr Lys Val Pro Ala Ala Tyr Ala Ala Gln Gly Tyr Lys Val Leu
   1               5                   10                  15
   Val Leu Asn Pro Ser Val Ala Ala Thr Leu Gly Phe Gly Ala Tyr Met
               20                  25                  30
   Ser Lys Ala His Gly Ile Glu Pro Asn Ile Arg Thr Gly Val
           35                  40                  45
<210> 25
   <211> 46
   <212> PRT
   <213> séquence artificielle
<400> 25
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Ser Phe Gly Ala Tyr Met
   20 25 30
   Ser Lys Ala His Gly Thr Asp Pro Asn Ile Arg Thr Gly Val
   35 40 45
<210> 26
   <211> 46
   <212> PRT
   <213> séquence artificielle
<400> 26
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala Tyr Gly Ile Asp Pro Asn Val Arg Thr Gly Val
   35 40 45

<210> 27
   <211> 46
   <212> PRT
   <213> séquence artificielle
<400> 27
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Thr Glu Pro Asn Ile Arg Thr Gly Val
   35 40 45
<210> 28
   <211> 46
   <212> PRT
   <213> séquence artificielle
<400> 28
   Ser Asn Lys Val Pro Val Glu Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Val Asp Pro Asn Ile Arg Thr Gly Val
   35 40 45
<210> 29
   <211> 46
   <212> PRT
   <213> séquence artificielle
<400> 29
   Ser Thr Lys Val Pro Ala Ala Tyr Ala Ala Gln Gly Tyr Lys Val Leu
   1 5 10 15
   Val Leu Asn Pro Ser Val Thr Ala Thr Leu Gly Phe Gly Ala Tyr Met
   20 25 30
   Ser Lys Ala His Gly Val Asp Pro Asn Ile Arg Thr Gly Val
   35 40 45
<210> 30
   <211> 46
   <212> PRT
   <213> séquence artificielle
<400> 30
   Ser Thr Lys Val Pro Ala Ala Tyr Ala Gly Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Ile Asp Pro Asn Ile Arg Thr Gly Val
   35 40 45
<210> 31
   <211> 46
   <212> PRT
   <213> séquence artificielle
<400> 31
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Ser Phe Gly Ala Tyr Met
   20 25 30
   Ser Lys Ala His Gly Ile Asp Pro Asn Ile Arg Thr Gly Val
   35 40 45
<210> 32
   <211> 46
   <212> PRT
   <213> séquence artificielle
<400> 32
   Ser Thr Arg Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Ile Asp Pro Asn Leu Arg Thr Gly Val
   35 40 45
<210> 33
   <211> 46
   <212> PRT
   <213> séquence artificielle
<400> 33
   Ser Thr Lys Val Pro Ala Ala Tyr Ala Thr Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Thr Asp Pro Asn Ile Arg Thr Gly Val
   35 40 45
<210> 34
   <211> 46
   <212> PRT
   <213> séquence artificielle
<400> 34
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Ile Asp Pro Asn Val Arg Thr Gly Val
   35 40 45
<210> 35
   <211> 46
   <212> PRT
   <213> séquence artificielle
<400> 35
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Ser Phe Gly Ala Tyr Met
   20 25 30
   Ser Lys Ala His Gly Val Asp Pro Ser Ile Arg Thr Gly Val
   35 40 45
<210> 36
   <211> 46
   <212> PRT
   <213> séquence artificielle
<400> 36
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala His Gly Val Asp Pro Asn Ile Ser Thr Gly Val
   35 40 45
<210> 37
   <211> 46
   <212> PRT
   <213> séquence artificielle
<400> 37
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Gly Phe Gly Ala Tyr Met
   20 25 30
   Ser Lys Ala Tyr Gly Thr Asp Pro Asn Val Arg Thr Gly Val
   35 40 45
<210> 38
   <211> 46
   <212> PRT
   <213> séquence artificielle
<400> 38
   Ser Thr Lys Val Pro Ala Ala Tyr Ala Thr Gln Gly Tyr Lys Val Leu
   1 5 10 15
   Val Leu Asn Pro Ser Val Ala Ala Thr Leu Ser Phe Gly Ala Tyr Met
   20 25 30
   Ser Lys Ala Tyr Gly Met Asp Pro Asn Leu Arg Thr Gly Val
   35 40 45
<210> 39
   <211> 46
   <212> PRT
   <213> séqence artificielle
<400> 39
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Ser Phe Gly Ala Tyr Met
   20 25 30
   Ser Lys Ala His Gly Val Asp Pro Asn Ile Arg Thr Gly Val
   35 40 45
<210> 40
   <211> 46
   <212> PRT
   <213> séquence artificielle
<400> 40
   Ser Thr Lys Val Pro Ala Ala Tyr Ala Ala Gln Gly Tyr Lys Val Leu
   1 5 10 15
   Val Leu Asn Pro Ser Val Ala Ala Thr Leu Ser Phe Gly Ala Tyr Met
   20 25 30
   Ser Lys Ala Tyr Gly Val Asp Pro Asn Ile Arg Thr Gly Val
   35 40 45
<210> 41
   <211> 46
   <212> PRT
   <213> séquence artificielle
<400> 41
   Ser Thr Arg Val Pro Ala Ala Tyr Ala Ala Gln Gly Tyr Lys Val Leu
   1               5                   10                  15
   Val Leu Asn Pro Ser Val Ala Ala Thr Leu Gly Phe Gly Ala Tyr Met
               20                  25                  30
   Ser Lys Ala His Gly Thr Asp Pro Asn Ile Arg Thr Gly Val
           35                  40                  45
<210> 42
   <211> 46
   <212> PRT
   <213> séquence artificielle
<400> 42
   Ser Thr Arg Val Pro Ala Ala Tyr Ala Ala Gln Gly Tyr Lys Val Leu
   1               5                   10                  15
   Val Leu Asn Pro Ser Val Ala Ala Thr Leu Ser Phe Gly Ala Tyr Met
               20                  25                  30
   Ser Lys Ala Tyr Gly Val Asp Pro Asn Ile Arg Thr Gly Val
           35                  40                  45
<210> 43
   <211> 46
   <212> PRT
   <213> séquence artificielle
<400> 43
   Ser Thr Arg Val Pro Ala Ala Tyr Ala Ala Gln Gly Tyr Lys Val Leu
   1               5                   10                  15
   Val Leu Asn Pro Ser Val Ala Ala Thr Leu Gly Phe Gly Thr Tyr Met
               20                  25                  30
   Ser Lys Ala Tyr Gly Thr Asp Pro Asn Ile Arg Thr Gly Val
           35                  40                  45

<210> 44
   <211> 46
   <212> PRT
   <213> séquence artificielle
<400> 44
   Ser Thr Lys Val Pro Ala Ala His Ala Ala Gln Gly Tyr Lys Val Leu
   1 5 10 15
   Val Leu Asn Pro Ser Val Ala Ala Thr Leu Gly Phe Gly Val Tyr Met
   20 25 30
   Ser Lys Ala Tyr Gly Ile Asp Pro Asn Ile Arg Ser Gly Val
   35 40 45
<210> 45
   <211> 45
   <212> PRT
   <213> séquence artificielle
<220>
   <221> MISC_FEATURE
   <222> (2) ..(2)
   <223> L ou V
<220>
   <221> MISC_FEATURE
   <222> (3)..(3)
   <223> L ou F
<220>
   <221> MISC_FEATURE
   <222> (4)..(4)
   <223> G ou S
<220>
   <221> MISC_FEATURE
   <222> (5)..(5)
   <223> C ou T
<220>
   <221> MISC_FEATURE
   <222> (6).. (6)
   <223> I ou V
<220>
   <221> MISC_FEATURE
   <222> (7)..(7)
   <223> I ou V
<220>
   <221> MISC_FEATURE
   <222> (15)..(15)
   <223> K, R ou T
<220>
   <221> MISC_FEATURE
   <222> (17) .. (17)
   <223> Q ou B
<220>
   <221> MISC_FEATURE
   <222> (18) .. (18)
   <223> V ou N
<220>
   <221> MISC-FEATURE
   <222> (19)..(19)
   <223> D, B ou C
<220>
   <221> MISC_FEATURE
   <222> (22)..(22)
   <223> V ou A
<220>
   <221> MISC_FEATURE
   <222> (24).. (24)
   <223> V, I, E ou M
<220>
   <221> MISC_FEATURE
   <222> (25)..(25)
   <223> L ou V
<220>
   <221> MISC_FEATURE
   <222> (29)..(29)
   <223> T, K ou A
<220>
   <221> MISC_FEATURE
   <222> (30) .. (30)
   <223> Q ou H
<220>
   <221> MISC_FEATURE
   <222> (31) .. (31)
   <223> S ou T
<220>
   <221> MISC_FEATURE
   <222> (34)..(34)
   <223> A ou G
<220>
   <221> MISC_FEATURE
   <222> (35)..(35)
   <223> T ou S
<220>
   <221> MISC_FEATURE
   <222> (36)..(36)
   <223> C ou A
<220>
   <221> MISC_FEATURE
   <222> (37)..(37)
   <223> V, I ou T
<220>
   <221> MISC_FEATURE
   <222> (40)..(40)
   <223> V ou A
<220>
   <221> MISC_FEATURE
   <222> (41)..(41)
   <223> C ou M
<220>
   <221> MISC_FEATURE
   <222> (45) .. (45)
   <223> Y ou F
<400> 45
   Gly Xaa Xaa Xaa Xaa Xaa Xaa Thr Ser Leu Thr Gly Arg Asp Xaa Asn
   1               5                   10                  15
   Xaa Xaa Xaa Gly Glu Xaa Gln Xaa Xaa Ser Thr Ala Xaa Xaa Xaa Phe
               20                  25                  30
   Leu Xaa Xaa Xaa Xaa Asn Gly Xaa Xaa Trp Thr Val Xaa
           35                  40                  45
<210> 46
   <211> 63
   <212> PRT
   <213> séquence artificielle
<220>
   <221> MISC_FEATURE
   <222> (2) .. (2)
   <223> P, 8 ou H
<220>
   <221> MISC_FEATURE
   <222> (5)..(5)
   <223> A ou T
<220>
   <221> MISC_FEATURE
   <222> (7)..(7)
   <223> S, A, T ou C
<220>
   <221> MISC_FEATURE
   <222> (8) .. (8)
   <223> Q ou R
<220>
   <221> MISC_FEATURE
   <222> (13)..(13)
   <223> L ou V
<220>
   <221> MISC_FEATURE
   <222> (14)..(14)
   <223> L ou F
<220>
   <221> MISC_FEATURE
   <222> (15)..(15)
   <223> G ou S
<220>
   <221> MISC_FEATURE
   <222> (16) .. (16)
   <223> C ou T
<220>
   <221> MISC_FEATURE
   <222> (17)..(17)
   <223> I ou V
<220>
   <221> MISC_FEATURE
   <222> (18)..(18)
   <223> I ou V
<220>
   <221> MISC_FEATURE
   <222> (26)..(26)
   <223> K, R ou T
<220>
   <221> MISC_FEATURE
   <222> (28).. (28)
   <223> Q ou E
<220>
   <221> MISC_FEATURE
   <222> (29)..(29)
   <223> V ou N
<220>
   <221> MISC_FEATURE
   <222> (30).. (30)
   <223> D, E ou C
<220>
   <221> MISC_FEATURE
   <222> (33)..(33)
   <223> V ou A
<220>
   <221> MISC_FEATURE
   <222> (35).. (35)
   <223> V, I, B ou M
<220>
   <221> MISC_FEATURE
   <222> (36) .. (36)
   <223> L ou V
<220>
   <221> MISC_FEATURE
   <222> (40)..(40)
   <223> T, K ou A
<220>
   <221> MISC_FEATURE
   <222> (41)..(41)
   <223> Q ou H
<220>
   <221> MISC_FEATURE
   <222> (42)..(42)
   <223> S ou T
<220>
   <221> MISC_FEATURE
   <222> (45)..(45)
   <223> A ou G
<220>
   <221> MISC_FEATURE
   <222> (46)..(46)
   <223> T ou S
<220>
   <221> MISC_FEATURE
   <222> (47) .. (47)
   <223> C ou A
<220>
   <221> MISC_FEATURE
   <222> (48) .. (48)
   <223> V, I ou T
<220>
   <221> MISC_FEATURE
   <222> (51).. (51)
   <223> V ou A
<220>
   <221> MISC_FEATURE
   <222> (52)..(52)
   <223> C ou M

<220>
   <221> MISC_FEATURE
   <222> (56) .. (56)
   <223> Y ou F
<220>
   <221> MISC_FEATURB
   <222> (61).. (61)
   <223> S, T ou A
<220>
   <221> MISC_FEATURE
   <222> (62) .. (62)
   <223> K ou R
<220>
   <221> MISC_FEATURE
   <222> (63)..(63)
   <223> T ou I
<400> 46
   Ala Xaa Ile Thr Xaa Tyr Xaa Xaa Gln Thr Arg Gly Xaa Xaa Xaa Xaa
   1               5                   10                  15
   Xaa Xaa Thr Ser Leu Thr Gly Arg Asp Xaa Asn Xaa Xaa Xaa Gly Glu
               20                  25                  30
   Xaa Gln Xaa Xaa Ser Thr Ala Xaa Xaa Xaa Phe Leu Xaa Xaa Xaa Xaa
           35                  40                  45
   Asn Gly Xaa Xaa Trp Thr Val Xaa His Gly Ala Gly Xaa Xaa Xaa
       50                  55                  60
<210> 47
   <211> 45
   <212> PRT
   <213> séquence artificielle
<400> 47
   Gly Leu Leu Gly Cys Ile Ile Thr Ser Leu Thr Gly Arg Asp Lys Asn
   1               5                   10                  15
   Gln Val Asp Gly Glu Val Gln Val Leu Ser Thr Ala Thr Gln Ser Phe
               20                  25                  30
   Leu Ala Thr Cys Val Asn Gly Val Cys Trp Thr Val Tyr
           35                  40                  45
<210> 48
   <211> 45
   <212> PRT
   <213> séquence artificielle
<400> 48
   Gly Leu Leu Gly Cys Ile Ile Thr Ser Leu Thr Gly Arg Asp Lys Asn
   1               5                   10                  15
   Gln Val Glu Gly Glu Val Gln Ile Val Ser Thr Ala Ala Gln Thr Phe
               20                  25                  30
   Leu Ala Thr Cys Ile Asn Gly Val Cys Trp Thr Val Tyr
           35                  40                  45
<210> 49
   <211> 45
   <212> PRT
   <213> séquence artificielle
<400> 49
   Gly Leu Leu Gly Cys Ile Ile Thr Ser Leu Thr Gly Arg Asp Lys Asn
   1               5                   10                  15
   Gln Val Glu Gly Glu Val Gln Ile Val Ser Thr Ala Thr Gln Thr Phe
               20                  25                  30
   Leu Ala Thr Cys Ile Asn Gly Val Cys Trp Thr Val Tyr
           35                  40                  45
<210> 50
   <211> 45
   <212> PRT
   <213> séquence artificielle
<400> 50
   Gly Leu Leu Gly Cys Ile Ile Thr Ser Leu Thr Gly Arg Asp Lys Asn
   1               5                   10                  15
   Gln Val Glu Gly Glu Val Gln Val Val Ser Thr Ala Thr Gln Ser Phe
               20                  25                  30
   Leu Ala Thr Cys Val Asn Gly Val Cys Trp Thr Val Tyr
           35                  40                  45
<210> -51
   <211> 45
   <212> PRT
   <213> séquence artificielle
<400> 51
<210> 52
   <211> 45
   <212> PRT
   <213> séquence artificielle
<400> 52
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Ile Asn Gly Val Cys Trp Thr Val Tyr
   35 40 45
<210> 53
   <211> 45
   <212> PRT
   <213> séquence artificielle
<400> 53
   Gly Leu Phe Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Val Asn Gly Val Cys Trp Thr Val Tyr
   35 40 45
<210> 54
   <211> 45
   <212> PRT
   <213> séquence artificielle
<400> 54
   Gly Val Leu Gly Cys Val Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Ile Asn Gly Val Cys Trp Thr Val Tyr
   35 40 45
<210> 55
   <211> 45
   <212> PRT
   <213> séquence artificielle
<400> 55
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Val Asn Gly Ala Cys Trp Thr Val Phe
   35 40 45
<210> 56
   <211> 45
   <212> PRT
   <213> séquence artificielle
<400> 56
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Ile Asn Gly Val Cys Trp Thr Val Phe
   35 40 45
<210> 57
   <211> 45
   <212> PRT
   <213> séquence artificielle
<400> 57
   Gly Val Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Ile Asn Gly Val Cys Trp Thr Val Tyr
   35 40 45
<210> 58
   <211> 45
   <212> PRT
   <213> séquence artificielle
<400> 58
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Val Asn Gly Val Cys Trp Thr Val Phe
   35 40 45

<210> 59
   <211> 45
   <212> PRT
   <213> séquence artificielle
<400> 59
   Gly Leu Leu Gly Cys Ile Val Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Val Asn Gly Ala Cys Trp Thr Val Phe
   35 40 45
<210> 60
   <211> 45
   <212> PRT
   <213> séquence artificielle
<400> 60
   Gly Val Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Val Asn Gly Val Cys Trp Thr Val Phe
   35 40 45
<210> 61
   <211> 45
   <212> PRT
   <213> séquence artificielle
<400> 61
   Gly Leu Leu Gly Cys Ile Ile Thr Ser Leu Thr Gly Arg Asp Lys Asn
   1 5 10 15
   Gln Val Asp Gly Glu Val Gln Val Leu Ser Thr Ala Thr Gln Ser Phe
   20 25 30
   Leu Ala Thr Cys Ile Asn Gly Val Cys Trp Thr Val Tyr
   35 40 45
<210> 62
   <211> 45
   <212> PRT
   <213> séquence artificielle
<400> 62
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Val Asn Gly Ala Cys Trp Thr Val Tyr
   35 40 45
<210> 63
   <211> 45
   <212> PRT
   <213> séquence artificielle
<400> 63
   Gly Val Leu Gly Cys Ile Ile Thr Ser Leu Thr Gly Arg Asp Lys Asn
   1 5 10 15
   Gln Val Glu Gly Glu Val Gln Val Val Ser Thr Ala Thr His Ser Phe
   20 25 30
   Leu Ala Thr Cys Ile Asn Gly Val Cys Trp Thr Val Tyr
   35 40 45
<210> 64
   <211> 45
   <212> PRT
   <213> séquence artificielle
<400> 64
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Ile Asn Gly Ala Cys Trp Thr Val Tyr
   35 40 45
<210> 65
   <211> 45
   <212> PRT
   <213> séquence artificielle
<400> 65
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Ser Cys Val Asn Gly Val Cys Trp Thr Val Tyr
   35 40 45
<210> 66
   <211> 45
   <212> PRT
   <213> séquence artificielle
<400> 66
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Thr Asn Gly Val Cys Trp Thr Val Tyr
   35 40 45
<210> 67
   <211> 45
   <212> PRT
   <213> séquence artificielle
<400> 67
   Gly Val Leu Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Val Asn Gly Val Cys Trp Thr Val Tyr
   35 40 45
<210> 68
   <211> 45
   <212> PRT
   <213> séquence artificielle
<400> 68
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Lys Gln Ser Phe
   20 25 30
   Leu Ala Thr Cys Val Asn Gly Val Cys Trp Thr Val Tyr
   35 40 45
<210> 69
   <211> 45
   <212> PRT
   <213> séquence artificielle
<400> 69
   Gly Leu Leu Gly Cys Ile Ile Thr Ser Leu Thr Gly Arg Asp Lys Asn
   1 5 10 15
   Gln Val Glu Gly Glu Val Gln Val Val Ser Thr Ala Lys Gln Ser Phe
   20 25 30
   Leu Ala Thr Cys Val Asn Gly Ala Cys Trp Thr Val Tyr
   35 40 45
<210> 70
   <211> 45
   <212> PRT
   <213> séquence artificielle
<400> 70
   Gly Leu Leu Gly Cys Ile Ile Thr Ser Leu Thr Gly Arg Asp Lys Asn
   1               5                   10                  15
   Gln Val Glu Gly Glu Val Gln Val Val Ser Thr Ala Thr Gln Thr Phe
               20                  25                  30
   Leu Ala Thr Cys Val Asn Gly Val Cys Trp Thr Val Phe
           35                  40                  45
<210> 71
   <211> 45
   <212> PRT
   <213> séquence artificielle
<400> 71
   Gly Leu Leu Gly Cys Ile Ile Thr Ser Leu Thr Gly Arg Asp Lys Asn
   1               5                   10                  15
   Gln Val Glu Gly Glu Val Gln Glu Val Ser Thr Ala Thr Gln Ser Phe
               20                  25                  30
   Leu Ala Thr Cys Val Asn Gly Val Cys Trp Thr Val Tyr
           35                  40                  45
<210> 72
   <211> 45
   <212> PRT
   <213> séquence artificielle
<400> 72
   Gly Leu Phe Gly Cys Ile Val Thr Ser Leu Thr Gly Arg Asp Lys Asn
   1 5 10 15
   Gln Val Glu Gly Glu Ala Gln Val Val Ser Thr Ala Thr Gln Ser Phe
   20 25 30
   Leu Ala Thr Cys Val Asn Gly Val Cys Trp Thr Val Tyr
   35 40 45

<210> 73
   <211> 45
   <212> PRT
   <213> séquence artificielle
<400> 73
   Gly Leu Phe Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Ile Asn Gly Val Cys Trp Thr Val Tyr
   35 40 45
<210> 74
   <211> 45
   <212> PRT
   <213> séquence artificielle
<400> 74
   Gly Leu Phe Gly Cys Ile Val Thr Ser Leu Thr Gly Arg Asp Lys Asn
   1 5 10 15
   Gln Val Glu Gly Glu Ala Gln Val Val Ser Thr Ala Thr Gln Ser Phe
   20 25 30
   Leu Ala Thr Cys Val Asn Gly Ala Cys Trp Thr Val Tyr
   35 40 45
<210> 75
   <211> 45
   <212> PRT
   <213> séquence artificielle
<400> 75
   Gly Leu Leu Gly Cys Ile Val Thr Ser Leu Thr Gly Arg Asp Lys Asn
   1               5                   10                  15
   Gln Val Glu Gly Glu Val Gln Val Val Ser Thr Ala Thr Gln Ser Phe
               20                  25                  30
   Leu Ala Thr Cys Val Asn Gly Val Cys Trp Thr Val Phe
           35                  40                  45
<210> 76
   <211> 45
   <212> PRT
   <213> séquence artificielle
<400> 76
   Gly Leu Phe Gly Cys Ile Ile Thr Ser Leu Thr Gly Arg Asp Arg Asn
   1               5                   10                  15
   Gln Val Glu Gly Glu Val Gln Val Val Ser Thr Ala Thr Gln Ser Phe
               20                  25                  30
   Leu Ala Thr Cys Ile Asn Gly Val Cys Trp Thr Val Tyr
           35                  40                  45
<210> 77
   <211> 45
   <212> PRT
   <213> séquence artificielle
<400> 77
   Gly Leu Phe Gly Cys Ile Ile Thr Ser Leu Thr Gly Arg Asp Lys Asn
   1 5 10 15
   Gln Val Glu Gly Glu Val Gln Val Val Ser Thr Ala Thr Gln Ser Phe
   20 25 30
   Leu Ala Thr Cys Ile Asn Gly Val Cys Trp Thr Val Tyr
   35 40 45
<210> 78
   <211> 45
   <212> PRT
   <213> séquence artificielle
<400> 78
   Gly Leu Leu Gly Cys Ile Ile Thr Ser Leu Thr Gly Arg Asp Arg Asn
   1 5 10 15
   Gln Val Glu Gly Glu Val Gln Val Val Ser Thr Ala Thr Gln Ser Phe
   20 25 30
   Leu Ala Thr Cys Ile Asn Gly Val Cys Trp Thr Val Phe
   35 40 45
<210> 79
   <211> 45
   <212> PRT
   <213> séquence artificielle
<400> 79
   Gly Leu Leu Gly Cys Ile Ile Thr Ser Leu Thr Gly Arg Asp Arg Asn
   1 5 10 15
   Gln Val Glu Gly Glu Val Gln Met Val Ser Thr Ala Thr Gln Ser Phe
   20 25 30
   Leu Ala Thr Cys Val Asn Gly Val Cys Trp Thr Val Tyr
   35 40 45
<210> 80
   <211> 45
   <212> PRT
   <213> séquence artificielle
<400> 80
   Gly Leu Phe Ser Thr Ile Ile Thr Ser Leu Thr Gly Arg Asp Thr Asn
   1 5 10 15
   Glu Asn Cys Gly Glu Val Gln Val Leu Ser Thr Ala Thr Gln Ser Phe
   20 25 30
   Leu Gly Thr Ala Val Asn Gly Val Met Trp Thr Val Tyr
   35 40 45
<210> 81
   <211> 63
   <212> PRT
   <213> séquence artificielle
<400> 81
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Asp Gly Glu
   20 25 30
   Val Gln Val Leu Ser Thr Ala Thr Gln Ser Phe Leu Ala Thr Cys Val
   35 40 45
   Asn Gly Val Cys Trp Thr Val Tyr His Gly Ala Gly Ser Lys Thr
   50 55 60
<210> 82
   <211> 63
   <212> PRT
   <213> séquence artificielle
<400> 82
   Ala Pro Ile Thr Ala Tyr Ala Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Ile Val Ser Thr Ala Ala Gln Thr Phe Leu Ala Thr Cys Ile
   35 40 45
   Asn Gly Val Cys Trp Thr Val Tyr His Gly Ala Gly Thr Arg Thr
   50 55 60
<210> 83
   <211> 63
   <212> PRT
   <213> séquence artificielle
<400> 83
   Ala Pro Ile Thr Ala Tyr Thr Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Ile Val Ser Thr Ala Thr Gln Thr Phe Leu Ala Thr Cys Ile
   35 40 45
   Asn Gly Val Cys Trp Thr Val Tyr His Gly Ala Gly Thr Arg Thr
   50 55 60
<210> 84
   <211> 63
   <212> PRT
   <213> séquence artificielle
<400> 84
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Tyr His Gly Ala Gly Ser Lys Thr
   50 55 60
<210> 85
   <211> 63
   <212> PRT
   <213> séquence artificielle
<400> 85
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Arg Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Tyr His Gly Ala Gly Ser Lys Thr
   50 55 60

<210> 86
   <211> 63
   <212> PRT
   <213> séquence artificielle
<400> 86
   Ala Pro Ile Thr Ala Tyr Ser Gln Gln Thr Arg Gly Leu Leu Gly Cys
   1               5                   10                  15
   Ile Ile Thr Ser Leu Thr Gly Arg Asp Lys Asn Gln Val Glu Gly Glu
               20                  25                  30
   Val Gln Val Val Ser Thr Ala Thr Gln Ser Phe Leu Ala Thr Cys Ile
           35                  40                  45
   Asn Gly Val Cys Trp Thr Val Tyr His Gly Ala Gly Ser Lys Thr
       50                  55                  60
<210> 87
   <211> 63
   <212> PRT
   <213> séquence artificielle
<400> 87
   Ala Pro Ile Thr Ala Tyr Ser Gln Gln Thr Arg Gly Leu Phe Gly Cys
   1               5                   10                  15
   Ile Ile Thr Ser Leu Thr Gly Arg Asp Lys Asn Gln Val Glu Gly Glu
               20                  25                  30
   Val Gln Val Val Ser Thr Ala Thr Gln Ser Phe Leu Ala Thr Cys Val
           35                  40                  45
   Asn Gly Val Cys Trp Thr Val Tyr His Gly Ala Gly Ser Lys Thr
       50                  55                  60
<210> 88
   <211> 63
   <212> PRT
   <213> séquence artificielle
<400> 88
   Ala Pro Ile Thr Ala Tyr Ser Gln Gln Thr Arg Gly Val Leu Gly Cys
   1 5 10 15
   Val Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Tyr His Gly Ala Gly Ser Lys Thr
   50 55 60
<210> 89
   <211> 63
   <212> PRT
   <213> séquence artificielle
<400> 89
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Ala Cys Trp Thr Val Phe His Gly Ala Gly Ser Lys Thr
   50 55 60
<210> 90
   <211> 63
   <212> PRT
   <213> séquence artificielle
<400> 90
   Ala Pro Ile Thr Ala Tyr Ser Arg Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Tyr His Gly Ala Gly Ser Lys Thr
   50 55 60
<210> 91
   <211> 63
   <212> PRT
   <213> séquence artificielle
<400> 91
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Phe His Gly Ala Gly Ala Lys Thr
   50 55 60
<210> 92
   <211> 63
   <212> PRT
   <213> séquence artificielle
<400> 92
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Phe His Gly Ala Gly Ser Lys Thr
   50 55 60
<210> 93
   <211> 63
   <212> PRT
   <213> séquence artificielle
<400> 93
   Ala Pro Ile Thr Ala Tyr Ser Gln Gln Thr Arg Gly Val Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Tyr His Gly Ala Gly Ser Lys Thr
   50 55 60
<210> 94
   <211> 63
   <212> PRT
   <213> séquence artificielle
<400> 94
   Ala Pro Ile Thr Ala Tyr Ala Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Phe His Gly Ala Gly Ser Lys Thr
   50 55 60
<210> 95
   <211> 63
   <212> PRT
   <213> séquence artificielle
<400> 95
   Ala Pro Ile Thr Ala Tyr Ser Gln Gln Thr Arg Gly Leu Leu Gly Cys
   1 5 10 15
   Ile Val Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Ala Cys Trp Thr Val Phe His Gly Ala Gly Ser Lys Thr
   50 55 60

<210> 96
   <211> 63
   <212> PRT
   <213> séquence artificielle
<400> 96
   Ala Pro Ile Thr Ala Tyr Ala Gln Gln Thr Arg Gly Val Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Phe His Gly Ala Gly Ser Lys Thr
   50 55 60
<210> 97
   <211> 63
   <212> PRT
   <213> séquence artificielle
<400> 97
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Asp Gly Glu
   20 25 30
   Val Gln Val Leu Ser Thr Ala Thr Gln Ser Phe Leu Ala Thr Cys Ile
   35 40 45
   Asn Gly Val Cys Trp Thr Val Tyr His Gly Ala Gly Ser Lys Thr
   50 55 60
<210> 98
   <211> 63
   <212> PRT
   <213> séquence artificielle
<400> 98
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Ala Cys Trp Thr Val Tyr His Gly Ala Gly Ser Lys Thr
   50 55 60
<210> 99
   <211> 63
   <212> PRT
   <213> séquene artificielle
<400> 99
   Ala Pro Ile Thr Ala Tyr Ser Gln Gln Thr Arg Gly Val Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr His Ser Phe Leu Ala Thr Cys Ile
   35 40 45
   Asn Gly Val Cys Trp Thr Val Tyr His Gly Ala Gly Ser Lys Thr
   50 55 60
<210> 100
   <211> 63
   <212> PRT
   <213> séquence artificielle
<400> 100
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Ala Cys Trp Thr Val Tyr His Gly Ala Gly Ser Lys Thr
   50 55 60
<210> 101
   <211> 63
   <212> PRT
   <213> séquence artificielle
<400> 101
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Ser Cys Val
   35 40 45
   Asn Gly Val Cys Trp Thr Val Tyr His Gly Ala Gly Ser Lys Thr
   50 55 60
<210> 102
   <211> 63
   <212> PRT
   <213> séquence artificielle
<400> 102
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Thr
   35 40 45
   Asn Gly Val Cys Trp Thr Val Tyr His Gly Ala Gly Ser Lys Thr
   50 55 60
<210> 103
   <211> 63
   <212> PRT
   <213> séquence artificielle
<400> 103
   Ala Pro Ile Thr Ala Tyr Ser Gln Gln Thr Arg Gly Val Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Tyr His Gly Ala Gly Ser Lys Thr
   50 55 60
<210> 104
   <211> 63
   <212> PRT
   <213> séquence artificielle
<400> 104
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Lys Gln Ser Phe Leu Ala Thr Cys Val
   35 40 45
   Asn Gly Val Cys Trp Thr Val Tyr His Gly Ala Gly Ser Lys Thr
   50 55 60
<210> 105
   <211> 63
   <212> PRT
   <213> séquence artificielle
<400> 105
   Ala Pro Ile Thr Ala Tyr Cys Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Tyr His Gly Ala Gly Ser Lys Thr
   50 55 60
<210> 106
   <211> 63
   <212> PRT
   <213> séquence artificielle
<400> 106
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Lys Gln Ser Phe Leu Ala Thr Cys Val
   35 40 45
   Asn Gly Ala Cys Trp Thr Val Tyr His Gly Ala Gly Ser Lys Thr
   50 55 60
<210> 107
   <211> 63
   <212> PRT
   <213> séquence artificielle
<400> 107
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Tyr His Gly Ala Gly Thr Lys Thr
   50 55 60
<210> 108
   <211> 63
   <212> PRT
   <213> séquence artificielle
<400> 108
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Thr Phe Leu Ala Thr Cys Val
   35 40 45
   Asn Gly Val Cys Trp Thr Val Phe His Gly Ala Gly Ser Lys Thr
   50 55 60

<210> 109
   <211> 63
   <212> PRT
   <213> séquence artificielle
<400> 109
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Ala Cys Trp Thr Val Tyr His Gly Ala Gly Thr Lys Thr
   50 55 60
<210> 110
   <211> 63
   <212> PRT
   <213> séquence artificielle
<400> 110
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Phe His Gly Ala Gly Thr Lys Thr
   50 55 60
<210> 111
   <211> 63
   <212> PRT
   <213> séquence artificielle
<400> 111
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Glu Val Ser Thr Ala Thr Gln Ser Phe Leu Ala Thr Cys Val
   35 40 45
   Asn Gly Val Cys Trp Thr Val Tyr His Gly Ala Gly Ser Lys Ile
   50 55 60
<210> 112
   <211> 63
   <212> PRT
   <213> séquence artificielle
<400> 112
   Ala Pro Ile Thr Ala Tyr Ser Gln Gln Thr Arg Gly Leu Phe Gly Cys
   1 5 10 15
   Ile Val Thr Ser Leu Thr Gly Arg Asp Lys Asn Gln Val Glu Gly Glu
   20 25 30
   Ala Gln Val Val Ser Thr Ala Thr Gln Ser Phe Leu Ala Thr Cys Val
   35 40 45
   Asn Gly Val Cys Trp Thr Val Tyr His Gly Ala Gly Ser Lys Thr
   50 55 60
<210> 113
   <211> 63
   <212> PRT
   <213> séquence artificielle
<400> 113
   Ala Pro Ile Thr Ala Tyr Ser Gln Gln Thr Arg Gly Leu Phe Gly Cys
   1               5                   10                  15
   Ile Ile Thr Ser Leu Thr Gly Arg Asp Lys Asn Gln Val Glu Gly Glu
               20                  25                  30
   Val Gln Val Val Ser Thr Ala Thr Gln Ser Phe Leu Ala Thr Cys Ile
           35                  40                  45
   Asn Gly Val Cys Trp Thr Val Tyr His Gly Ala Gly Ser Lys Thr
       50                  55                  60
<210> 114
   <211> 63
   <212> PRT
   <213> séquence artificielle
<400> 114
   Ala Pro Ile Thr Ala Tyr Ser Gln Gln Thr Arg Gly Leu Ala Gly Cys
   1               5                   10                  15
   Ile Val Thr Ser Leu Thr Gly Arg Asp Lys Asn Gln Val Glu Gly Glu
               20                  25                  30
   Ala Gln Val Val Ser Thr Ala Thr Gln Ser Phe Leu Ala Thr Cys Val
           35                  40                  45
   Asn Gly Ala Cys Trp Thr Val Tyr His Gly Ala Gly Ser Lys Thr
       50                  55                  60
<210> 115
   <211> 63
   <212> PRT
   <213> séquence artificielle
<400> 115
   Ala Pro Ile Thr Ala Tyr Ser Gln Gln Thr Arg Gly Val Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Phe His Gly Ala Gly Ser Lys Thr
   50 55 60
<210> 116
   <211> 63
   <212> PRT
   <213> séquence artificielle
<400> 116
   Ala Pro Ile Thr Thr Tyr Ser Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Tyr His Gly Ala Gly Thr Lys Thr
   50 55 60
<210> 117
   <211> 63
   <212> PRT
   <213> séquence artificielle
<400> 117
   Ala Ser Ile Thr Ala Tyr Ser Gln Gln Thr Arg Gly Val Leu Gly Cys
   1 5 10 15
   Val Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Tyr His Gly Ala Gly Ser Lys Thr
   50 55 60

<210> 118
   <211> 63
   <212> PRT
   <213> séquene artificielle
<400> 118
   Ala Ser Ile Thr Ala Tyr Ser Gln Gln Thr Arg Gly Leu Leu Gly Cys
   1 5 10 15
   Ile Val Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Phe His Gly Ala Gly Ser Lys Thr
   50 55 60
<210> 119
   <211> 63
   <212> PRT
   <213> séquene artificielle
<400> 119
   Ala Pro Ile Thr Ala Tyr Ala Gln Gln Thr Arg Gly Leu Leu Gly Cys
   1 5 10 15
   Ile Val Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Val
   35 40 45
   Asn Gly Val Cys Trp Thr Val Phe His Gly Ala Gly Ser Lys Thr
   50 55 60
<210> 120
   <211> 63
   <212> PRT
   <213> séquence artificielle
<400> 120
   Ala Pro Ile Thr Ala Tyr Ala Gln Gln Thr Arg Gly Leu Leu Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Tyr His Gly Ala Gly Ser Lys Thr
   50 55 60
<210> 121
   <211> 63
   <212> PRT
   <213> séquence artificielle
<400> 121
   Ala Pro Ile Thr Ala Tyr Ala Gln Gln Thr Arg Gly Leu Phe Gly Cys
   1 5 10 15
   Ile Ile Thr Ser Leu Thr Gly Arg Asp Arg Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Tyr His Gly Ala Gly Ser Lys Thr
   50 55 60
<210> 122
   <211> 63
   <212> PRT
   <213> séquence artificielle
<400> 122
   Ala Pro Ile Thr Ala Tyr Ala Gln Gln Thr Arg Gly Leu Phe Gly Cys
   1 5 10 15
   Ile Ile Thr Ser Leu Thr Gly Arg Asp Lys Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Tyr His Gly Ala Gly Ser Lys Thr
   50 55 60
<210> 123
   <211> 63
   <212> PRT
   <213> séquence artificielle
<400> 123
   Ala Pro Ile Thr Ala Tyr Ser Gln Gln Thr Arg Gly Leu Leu Gly Cys
   1 5 10 15
   Ile Ile Thr Ser Leu Thr Gly Arg Asp Arg Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Phe His Gly Ala Gly Ser Lys Thr
   50 55 60
<210> 124
   <211> 63
   <212> PRT
   <213> séquence artificielle
<400> 124
   Ala Pro Ile Thr Ala Tyr Ser Gln Gln Thr Arg Gly Leu Leu Gly Cys
   1               5                   10                  15
   Ile Ile Thr Ser Leu Thr Gly Arg Asp Arg Asn Gln Val Glu Gly Glu
               20                  25                  30
   Val Gln Val Val Ser Thr Ala Thr Gln Ser Phe Leu Ala Thr Cys Val
           35                  40                  45
   Asn Gly Val Cys Trp Thr Val Tyr His Gly Ala Gly Ala Lys Thr
       50                  55                  60
<210> 125
   <211> 63
   <212> PRT
   <213> séquence artificielle
<400> 125
   Ala His Ile Thr Ala Tyr Ser Gln Gln Thr Arg Gly Leu Leu Gly Cys
   1               5                   10                  15
   Ile Ile Thr Ser Leu Thr Gly Arg Asp Arg Asn Gln Val Glu Gly Glu
               20                  25                  30
   Val Gln Met Val Ser Thr Ala Thr Gln Ser Phe Leu Ala Thr Cys Val
           35                  40                  45
   Asn Gly Val Cys Trp Thr Val Tyr His Gly Ala Gly Ser Lys Thr
       50                  55                  60
<210> 126
   <211> 63
   <212> PRT
   <213> séquence artificielle
<400> 126
   Ala Pro Ile Thr Ala Tyr Ser Gln Gln Thr Arg Gly Leu Phe Ser Thr
   1               5                   10                  15
   Ile Ile Thr Ser Leu Thr Gly Arg Asp Thr Asn Glu Asn Cys Gly Glu
               20                  25                  30
   Val Gln Val Leu Ser Thr Ala Thr Gln Ser Phe Leu Gly Thr Cys Val
           35                  40                  45
   Asn Gly Val Met Trp Thr Val Tyr His Gly Ala Gly Ala Lys Thr
       50                  55                  60
<210> 127
   <211> 32
   <212> PRT
   <213> séquence artificielle
<220>
   <221> MISC_FEATURE
   <222> (2) .. (2)
   <223> L ou P
<220>
   <221> MISC_FEATURE
   <222> (4)..(4)
   <223> A ou S
<220>
   <221> MISC_FEATURE
   <222> (7)..(7)
   <223> A ou S
<220>
   <221> MISC_FEATURE
   <222> (8)..(8)
   <223> A ou B
<220>
   <221> MISC_FEATURE
   <222> (9)..(9)
   <223> I ou V
<220>
   <221> MISC_FEATURE
   <222> (14)..(14)
   <223> T, S ou A
<220>
   <221> MISC_FEATURE
   <222> (15)..(15)
   <223> T ou I
<220>
   <221> MISC_FEATURE
   <222> (16)..(16)
   <223> Q, S ou G

<220>
   <221> MISC_FEATURE
   <222> (17)..(17)
   <223> N, Q, H, S, Y ou T
<220>
   <221> MISC_FEATURE
   <222> (20)..(20)
   <223> L ou M
<220>
   <221> MISC_FEATURE
   <222> (24)..(24)
   <223> L ou W
<220>
   <221> MISC_FEATURE
   <222> (30)..(30)
   <223> A ou S
<220>
   <221> MISC_FEATURE
   <222> (32)..(32)
   <223> L, P ou I
<400> 127
   Ser Xaa Met Xaa Phe Thr Xaa Xaa Xaa Thr Ser Pro Leu Xaa Xaa Xaa
   1               5                   10                  15
   Xaa Thr Leu Xaa Phe Asn Ile Xaa Gly Gly Trp Val Ala Xaa Gln Xaa
               20                  25                  30
<210> 128
   <211> 57
   <212> PRT
   <213> séquence artificielle
<220>
   <221> MISC_FEATURE
   <222> (4)..(4)
   <223> T ou S
<220>
   <221> MISC_FEATURE
   <222> (6) .. (6)
   <223> I, T ou V
<220>
   <221> MISC_FEATURE
   <222> (11)..(11)
   <223> G ou A
<220>
   <221> MISC_FEATURE
   <222> (19)..(19)
   <223> P ou L
<220>
   <221> MISC_FEATURE
   <222> (21)..(21)
   <223> I ou M
<220>
   <221> MISC_FEATURE
   <222> (22)..(22)
   <223> A, V ou R
<220>
   <221> MISC_FEATURE
   <222> (24)..(24)
   <223> L ou P
<220>
   <221> MISC_FEATURE
   <222> (26) .. (26)
   <223> A ou 8
<220>
   <221> MISC_FEATURE
   <222> (29)..(29)
   <223> A ou S
<220>
   <221> MISC_FEATURE
   <222> (30) .. (30)
   <223> A ou 8
<220>
   <221> MISC_FEATURE
   <222> (31) .. (31)
   <223> I ou V
<220>
   <221> MISC_FEATURE
   <222> (36).. (36)
   <223> T, S ou A
<220>
   <221> MISC_FEATURE
   <222> (37) .. (37)
   <223> T ou I
<220>
   <221> MISC_FEATURE
   <222> (38) .. (38)
   <223> Q, S ou G
<220>
   <221> MISC_FEATURE
   <222> (39) .. (39)
   <223> N, Q, H, S, Y ou T
<220>
   <221> MISC_FEATURE
   <222> (42) .. (42)
   <223> L ou M
<220>
   <221> MISC_FEATURE
   <222> (46) .. (46)
   <223> L ou W
<220>
   <221> MISC_FEATURE
   <222> (52) ..(52)
   <223> A ou S
<220>
   <221> MISC_FEATURE
   <222> (54) .. (54)
   <223> L, P ou I
<220>
   <221> MISC_FEATURE
   <222> (55) .. (55)
   <223> A ou R
<220>
   <221> MISC_FEATURE
   <222> (56) .. (56)
   <223> P, A ou D
<220>
   <221> MISC_FEATURE
   <222> (57)..(57)
   <223> P, A ou S
<400> 128
   Asn Phe Ile Xaa Gly Xaa Gln Tyr Leu Ala Xaa Leu Ser Thr Leu Pro
   1               5                   10                  15
   Gly Asn Xaa Ala Xaa Xaa Ser Xaa Met Xaa Phe Thr Xaa Xaa Xaa Thr
               20                  25                  30
   Ser Pro Leu Xaa Xaa Xaa Xaa Thr Leu Xaa Phe Asn Ile Xaa Gly Gly
           35                  40                  45
   Trp Val Ala Xaa Gln Xaa Xaa Xaa Xaa
<210> 129
   <211> 32
   <212> PRT
   <213> séquence artificielle
<400> 129
   Ser Leu Met Ala Phe Thr Ala Ser Ile Thr Ser Pro Leu Thr Thr Gln
   1               5                   10                  15
   Asn Thr Leu Leu Phe Asn Ile Leu Gly Gly Trp Val Ala Ala Gln Leu
               20                  25                  30
<210> 130
   <211> 32
   <212> PRT
   <213> séquence artificielle
<400> 130
   Ser Leu Met Ala Phe Thr Ala Ala Val Thr Ser Pro Leu Thr Thr Ser
   1               5                   10                  15
   Gln Thr Leu Leu Phe Asn Ile Leu Gly Gly Trp Val Ala Ala Gln Leu
               20                  25                  30
<210> 131
   <211> 32
   <212> PRT
   <213> séquence artificielle
<400> 131
   Ser Leu Met Ala Phe Thr Ala Ala Val Thr Ser Pro Leu Thr Thr Gly
   1               5                   10                  15
   Gln Thr Leu Leu Phe Asn Ile Leu Gly Gly Trp Val Ala Ala Gln Leu
               20                  25                  30
<210> 132
   <211> 32
   <212> PRT
   <213> séquence artificielle
<400> 132
   Ser Leu Met Ala Phe Thr Ala Ser Ile Thr Ser Pro Leu Thr Thr Gln
   1 5 10 15
   His Thr Leu Leu Phe Asn Ile Leu Gly Gly Trp Val Ala Ala Gln Leu
   20 25 30
<210> 133
   <211> 32
   <212> PRT
   <213> séquence artificielle
<400> 133
   Ser Leu Met Ala Phe Thr Ala Ser Ile Thr Ser Pro Leu Thr Thr Gln
   1 5 10 15
   Ser Thr Leu Leu Phe Asn Ile Leu Gly Gly Trp Val Ala Ala Gln Leu
   20 25 30
<210> 134
   <211> 32
   <212> PRT
   <213> séquence artificielle
<400> 134
   Ser Leu Met Ala Phe Thr Ala Ser Ile Thr Ser Pro Leu Thr Thr Gln
   1 5 10 15
   Tyr Thr Leu Leu Phe Asn Ile Leu Gly Gly Trp Val Ala Ala Gln Leu
   20 25 30
<210> 135
   <211> 32
   <212> PRT
   <213> séquence artificielle
<400> 135
   Ser Leu Met Ala Phe Thr Ala Ser Val Thr Ser Pro Leu Thr Thr Gln
   1 5 10 15
   Asn Thr Leu Leu Phe Asn Ile Leu Gly Gly Trp Val Ala Ala Gln Leu
   20 25 30
<210> 136
   <211> 32
   <212> PRT
   <213> séquence artificielle

<400> 136
   Ser Leu Met Ala Phe Thr Ala Ser Val Thr Ser Pro Leu Thr Thr Gln
   1 5 10 15
   Ser Thr Leu Leu Phe Asn Ile Leu Gly Gly Trp Val Ala Ala Gln Leu
   20 25 30
<210> 137
   <211> 32
   <212> PRT
   <213> séquence artificielle
<400> 137
   Ser Leu Met Ala Phe Thr Ala Ser Ile Thr Ser Pro Leu Thr Thr Gln
   1 5 10 15
   Thr Thr Leu Met Phe Asn Ile Leu Gly Gly Trp Val Ala Ala Gln Leu
   20 25 30
<210> 138
   <211> 32
   <212> PRT
   <213> séquence artificielle
<400> 138
   Ser Leu Met Ala Phe Thr Ala Ser Val Thr Ser Pro Leu Thr Thr Gln
   1 5 10 15
   Tyr Thr Leu Leu Phe Asn Ile Leu Gly Gly Trp Val Ala Ala Gln Ile
   20 25 30
<210> 139
   <211> 32
   <212> PRT
   <213> séquence artificielle
<400> 139
   Ser Pro Met Ala Phe Thr Ala Ser Ile Thr Ser Pro Leu Thr Thr Gln
   1               5                   10                  15
   His Thr Leu Leu Phe Asn Ile Leu Gly Gly Trp Val Ala Ala Gln Leu
               20                  25                  30
<210> 140
   <211> 32
   <212> PRT
   <213> séquence artificielle
<400> 140
   Ser Leu Met Ala Phe Thr Ala Ser Ile Thr Ser Pro Leu Thr Ile Gln
   1               5                   10                  15
   His Thr Leu Leu Phe Asn Ile Leu Gly Gly Trp Val Ala Ala Gln Pro
               20                  25                  30
<210> 141
   <211> 32
   <212> PRT
   <213> séquence artificielle
<400> 141
   Ser Leu Met Ala Phe Thr Ser Ser Ile Thr Ser Pro Leu Thr Thr Gln
   1               5                   10                  15
   Ser Thr Leu Leu Phe Asn Ile Leu Gly Gly Trp Val Ala Ala Gln Leu
               20                  25                  30
<210> 142
   <211> 32
   <212> PRT
   <213> séquence artificielle
<400> 142
   Ser Leu Met Ala Phe Thr Ala Ser Ile Thr Ser Pro Leu Ser Thr Gln
   1 5 10 15
   Asn Thr Leu Leu Phe Asn Ile Trp Gly Gly Trp Val Ala Ala Gln Leu
   20 25 30
<210> 143
   <211> 32
   <212> PRT
   <213> séquence artificielle
<400> 143
   Ser Leu Met Ala Phe Thr Ala Ser Ile Thr Ser Pro Leu Thr Thr Gln
   1 5 10 15
   Asn Thr Leu Met Phe Asn Ile Leu Gly Gly Trp Val Ala Ala Gln Leu
   20 25 30
<210> 144
   <211> 32
   <212> PRT
   <213> séquence artificielle
<400> 144
   Ser Leu Met Ala Phe Thr Ala Ser Ile Thr Ser Pro Leu Thr Thr Gln
   1 5 10 15
   His Thr Leu Met Phe Asn Ile Leu Gly Gly Trp Val Ala Ala Gln Leu
   20 25 30
<210> 145
   <211> 32
   <212> PRT
   <213> séquence artificielle
<400> 145
   Ser Leu Met Ala Phe Thr Ala Ser Ile Thr Ser Pro Leu Ala Thr Gln
   1 5 10 15
   Tyr Thr Leu Leu Phe Asn Ile Leu Gly Gly Trp Val Ala Ala Gln Leu
   20 25 30
<210> 146
   <211> 32
   <212> PRT
   <213> séquence artificielle
<400> 146
   Ser Leu Met Ser Phe Thr Ala Ala Val Thr Ser Pro Leu Thr Thr Gln
   1 5 10 15
   Gln Thr Leu Leu Phe Asn Ile Leu Gly Gly Trp Val Ala Ser Gln Ile
   20 25 30
<210> 147
   <211> 57
   <212> PRT
   <213> séquence artificielle
<400> 147
   Asn Phe Ile Thr Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln Asn Thr Leu Leu Phe Asn Ile Leu Gly Gly
   35 40 45
   Trp Val Ala Ala Gln Leu Ala Pro Pro
   50 55
<210> 148
   <211> 57
   <212> PRT
   <213> séquence artificielle
<400> 148
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ala Val Thr
   20 25 30
   Ser Pro Leu Thr Thr Ser Gln Thr Leu Leu Phe Asn Ile Leu Gly Gly
   35 40 45
   Trp Val Ala Ala Gln Leu Ala Ala Pro
   50 55
<210> 149
   <211> 57
   <212> PRT
   <213> séquence artificielle
<400> 149
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ala Val Thr
   20 25 30
   Ser Pro Leu Thr Thr Gly Gln Thr Leu Leu Phe Asn Ile Leu Gly Gly
   35 40 45
   Trp Val Ala Ala Gln Leu Ala Ala Pro
   50 55
<210> 150
   <211> 57
   <212> PRT
   <213> séquence artificielle
<400> 150
   Asn Phe Ile Ser Gly Thr Gln Tyr Leu Ala Gly Leu Ser Thr Leu Pro
   1               5                   10                  15
   Gly Asn Pro Ala Ile Ala Ser Leu Met Ala Phe Thr Ala Ala Val Thr
               20                  25                  30
   Ser Pro Leu Thr Thr Ser Gln Thr Leu Leu Phe Asn Ile Leu Gly Gly
           35                  40                  45
   Trp Val Ala Ala Gln Leu Ala Ala Pro
       50                  55
<210> 151
   <211> 57
   <212> PRT
   <213> séquence artificielle
<400> 151
   Asn Phe Ile Ser Gly Ile Gln Tyr Leu Ala Gly Leu Ser Thr Leu Pro
   1               5                   10                  15
   Gly Asn Pro Ala Ile Ala Ser Leu Met Ala Phe Thr Ala Ser Ile Thr
               20                  25                  30
   Ser Pro Leu Thr Thr Gln His Thr Leu Leu Phe Asn Ile Leu Gly Gly
           35                  40                  45
   Trp Val Ala Ala Gln Leu Ala Pro Pro
       50                  55

<210> 152
   <211> 57
   <212> PRT
   <213> séquence artificielle
<400> 152
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln Ser Thr Leu Leu Phe Asn Ile Leu Gly Gly
   35 40 45
   Trp Val Ala Ala Gln Leu Ala Pro Pro
   50 55
<210> 153
   <211> 57
   <212> PRT
   <213> séquence artificielle
<400> 153
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln Asn Thr Leu Leu Phe Asn Ile Leu Gly Gly
   35 40 45
   Trp Val Ala Ala Gln Leu Ala Pro Pro
   50 55
<210> 154
   <211> 57
   <212> PRT
   <213> séquence artificielle
<400> 154
   Asn Phe Ile Ser Gly Ile Gln Tyr Leu Ala Gly Leu Ser Thr Leu Pro
   1               5                   10                  15
   Gly Asn Pro Ala Ile Ala Ser Leu Met Ala Phe Thr Ala Ser Ile Thr
               20                  25                  30
   Ser Pro Leu Thr Thr Gln Tyr Thr Leu Leu Phe Asn Ile Leu Gly Gly
           35                  40                  45
   Trp Val Ala Ala Gln Leu Ala Pro Pro
       50                  55
<210> 155
   <211> 57
   <212> PRT
   <213> séquence artificielle
<400> 155
   Asn Phe Ile Ser Gly Ile Gln Tyr Leu Ala Gly Leu Ser Thr Leu Pro
   1               5                   10                  15
   Gly Asn Pro Ala Ile Val Ser Leu Met Ala Phe Thr Ala Ser Ile Thr
               20                  25                  30
   Ser Pro Leu Thr Thr Gln His Thr Leu Leu Phe Asn Ile Leu Gly Gly
           35                  40                  45
   Trp Val Ala Ala Gln Leu Ala Pro Pro
       50                  55
<210> 156
   <211> 57
   <212> PRT
   <213> séquence artificielle
<400> 156
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Val Thr
   20 25 30
   Ser Pro Leu Thr Thr Gln Asn Thr Leu Leu Phe Asn Ile Leu Gly Gly
   35 40 45
   Trp Val Ala Ala Gln Leu Ala Pro Pro
   50 55
<210> 157
   <211> 57
   <212> PRT
   <213> séquence artificielle
<400> 157
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Val Thr
   20 25 30
   Ser Pro Leu Thr Thr Gln Ser Thr Leu Leu Phe Asn Ile Leu Gly Gly
   35 40 45
   Trp Val Ala Ala Gln Leu Ala Pro Pro
   50 55
<210> 158
   <211> 57
   <212> PRT
   <213> séquence artificielle
<400> 158
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln Thr Thr Leu Met Phe Asn Ile Leu Gly Gly
   35 40 45
   Trp Val Ala Ala Gln Leu Ala Pro Pro
   50 55
<210> 159
   <211> 57
   <212> PRT
   <213> séquence artificielle
<400> 159
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Val Thr
   20 25 30
   Ser Pro Leu Thr Thr Gln Tyr Thr Leu Leu Phe Asn Ile Leu Gly Gly
   35 40 45
   Trp Val Ala Ala Gln Ile Ala Pro Pro
   50 55
<210> 160
   <211> 57
   <212> PRT
   <213> séquence artificielle
<400> 160
   Asn Phe Ile Ser Gly Ile Gln Tyr Leu Ala Gly Leu Ser Thr Leu Pro
   1               5                   10                  15
   Gly Asn Pro Ala Ile Arg Ser Pro Met Ala Phe Thr Ala Ser Ile Thr
               20                  25                  30
   Ser Pro Leu Thr Thr Gln His Thr Leu Leu Phe Asn Ile Leu Gly Gly
           35                  40                  45
   Trp Val Ala Ala Gln Leu Ala Pro Pro
       50                  55
<210> 161
   <211> 57
   <212> PRT
   <213> séquence artificielle
<400> 161
   Asn Phe Ile Ser Gly Ile Gln Tyr Leu Ala Gly Leu Ser Thr Leu Pro
   1               5                   10                  15
   Gly Asn Pro Ala Ile Ala Ser Leu Met Ala Phe Thr Ala Ser Ile Thr
               20                  25                  30
   Ser Pro Leu Thr Ile Gln His Thr Leu Leu Phe Asn Ile Leu Gly Gly
           35                  40                  45
   Trp Val Ala Ala Gln Pro Ala Pro Pro
       50                  55
<210> 162
   <211> 57
   <212> PRT
   <213> séquence artificielle
<400> 162
   Asn Phe Ile Ser Gly Ile Gln Tyr Leu Ala Ala Leu Ser Thr Leu Pro
   1               5                   10                  15
   Gly Asn Pro Ala Ile Ala Ser Leu Met Ala Phe Thr Ala Ser Ile Thr
               20                  25                  30
   Ser Pro Leu Thr Thr Gln Asn Thr Leu Leu Phe Asn Ile Leu Gly Gly
           35                  40                  45
   Trp Val Ala Ala Gln Leu Ala Pro Ala
       50                  55

<210> 163
   <211> 57
   <212> PRT
   <213> séquence artificielle
<400> 163
   Asn Phe Ile Ser Gly Ile Gln Tyr Leu Ala Gly Leu Ser Thr Leu Pro
   1               5                   10                  15
   Gly Asn Pro Ala Ile Ala Ser Leu Met Ala Phe Thr Ser Ser Ile Thr
               20                  25                  30
   Ser Pro Leu Thr Thr Gln Ser Thr Leu Leu Phe Asn Ile Leu Gly Gly
           35                  40                  45
   Trp Val Ala Ala Gln Leu Ala Pro Pro
       50                  55
<210> 164
   <211> 57
   <212> PRT
   <213> séquence artificielle
<400> 164
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Ser Thr Gln Asn Thr Leu Leu Phe Asn Ile Trp Gly Gly
   35 40 45
   Trp Val Ala Ala Gln Leu Ala Pro Pro
   50 55
<210> 165
   <211> 57
   <212> PRT
   <213> séquence artificielle
<400> 165
   Asn Phe Ile Ser Gly Ile Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln Asn Thr Leu Met Phe Asn Ile Leu Gly Gly
   35 40 45
   Trp Val Ala Ala Gln Leu Ala Pro Pro
   50 55
<210> 166
   <211> 57
   <212> PRT
   <213> séquence artificielle
<400> 166
   Asn Phe Ile Ser Gly Ile Gln Tyr Leu Ala Gly Leu Ser Thr Leu Pro
   1 5 10 15
   Gly Asn Pro Ala Met Ala Ser Leu Met Ala Phe Thr Ala Ser Ile Thr
   20 25 30
   Ser Pro Leu Thr Thr Gln His Thr Leu Met Phe Asn Ile Leu Gly Gly
   35 40 45
   Trp Val Ala Ala Gln Leu Ala Pro Pro
   50 55
<210> 167
   <211> 57
   <212> PRT
   <213> séquence artificielle
<400> 167
   Asn Phe Ile Ser Gly Val Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln Asn Thr Leu Leu Phe Asn Ile Leu Gly Gly
   35 40 45
   Trp Val Ala Ala Gln Leu Ala Pro Pro
   50 55
<210> 168
   <211> 57
   <212> PRT
   <213> séquence artificielle
<400> 168
   Asn Phe Ile Ser Gly Val Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln His Thr Leu Leu Phe Asn Ile Leu Gly Gly
   35 40 45
   Trp Val Ala Ala Gln Leu Ala Pro Pro
   50 55
<210> 169
   <211> 57
   <212> PRT
   <213> séquence artificielle
<400> 169
   Asn Phe Ile Ser Gly Val Gln Tyr Leu Ala Gly Leu Ser Thr Leu Pro
   1 5 10 15
   Gly Asn Pro Ala Ile Ala Ser Leu Met Ala Phe Thr Ala Ser Ile Thr
   20 25 30
   Ser Pro Leu Thr Thr Gln Tyr Thr Leu Leu Phe Asn Ile Leu Gly Gly
   35 40 45
   Trp Val Ala Ala Gln Leu Ala Pro Pro
   50 55
<210> 170
   <211> 57
   <212> PRT
   <213> séquence artificielle
<400> 170
   Asn Phe Ile Ser Gly Val Gln Tyr Leu Ala Gly Leu Ser Thr Leu Pro
   1 5 10 15
   Gly Asn Pro Ala Ile Ala Ser Leu Met Ala Phe Thr Ala Ser Val Thr
   20 25 30
   Ser Pro Leu Thr Thr Gln Ser Thr Leu Leu Phe Asn Ile Leu Gly Gly
   35 40 45
   Trp Val Ala Ala Gln Leu Ala Pro Pro
   50 55
<210> 171
   <211> 57
   <212> PRT
   <213> séquence artificielle
<400> 171
   Asn Phe Ile Ser Gly Ile Gln Tyr Leu Ala Gly Leu Ser Thr Leu Pro
   1               5                   10                  15
   Gly Asn Leu Ala Ile Ala Ser Leu Met Ala Phe Thr Ala Ser Ile Thr
               20                  25                  30
   Ser Pro Leu Thr Thr Gln His Thr Leu Leu Phe Asn Ile Leu Gly Gly
           35                  40                  45
   Trp Val Ala Ala Gln Leu Ala Pro Pro
       50                  55
<210> 172
   <211> 57
   <212> PRT
   <213> séquence artificielle
<400> 172
   Asn Phe Ile Ser Gly Ile Gln Tyr Leu Ala Gly Leu Ser Thr Leu Pro
   1               5                   10                  15
   Gly Asn Pro Ala Ile Ala Ser Leu Met Ala Phe Thr Ala Ser Ile Thr
               20                  25                  30
   Ser Pro Leu Ala Thr Gln Tyr Thr Leu Leu Phe Asn Ile Leu Gly Gly
           35                  40                  45
   Trp Val Ala Ala Gln Leu Ala Pro Pro
       50                  55
<210> 173
   <211> 57
   <212> PRT
   <213> séquence artificielle
<400> 173
   Asn Phe Ile Ser Gly Ile Gln Tyr Leu Ala Gly Leu Ser Thr Leu Pro
   1               5                   10                  15
   Gly Asn Pro Ala Ile Ala Ser Leu Met Ser Phe Thr Ala Ala Val Thr
               20                  25                  30
   Ser Pro Leu Thr Thr Gln Gln Thr Leu Leu Phe Asn Ile Leu Gly Gly
           35                  40                  45
   Trp Val Ala Ser Gln Ile Arg Asp Ser
       50                  55

<210> 174
   <211> 29
   <212> PRT
   <213> séquence artificielle
<220>
   <221> MISC_FEATURE
   <222> (1)..(1)
   <223> R ou Q
<220>
   <221> MISC_FEATURE
   <222> (3) ..(3)
   <223> P ou A
<220>
   <221> MISC_FEATURE
   <222> (6) .. (6)
   <223> L ou F
<220>
   <221> MISC_FEATURE
   <222> (9)..(9)
   <223> P ou Y
<220>
   <221> MISC_FEATURE
   <222> (11) ..(11)
   <223> D ou E
<220>
   <221> MISC_FEATURE
   <222> (14)..(14)
   <223> V ou S
<220>
   <221> MISC_FEATURE
   <222> (20)..(20)
   <223> M ou R
<220>
   <221> MISC_FEATURE
   <222> (23)..(23)
   <223> Y ou H
<220>
   <221> MISC_FEATURE
   <222> (24) .. (24)
   <223> D ou N
<220>
   <221> MISC_FEATURE
   <222> (26) .. (26)
   <223> V ou I
<220>
   <221> MISC_FEATURE
   <222> (27)..(27)
   <223> S, T ou K
<220>
   <221> MISC_FEATURE
   <222> (28)..(28)
   <223> T, K, I ou N
<220>
   <221> MISC_FEATURE
   <222> (29)..(29)
   <223> L ou T
<400> 174
   Xaa Lys Xaa Ala Arg Xaa Ile Val Xaa Pro Xaa Leu Gly Xaa Arg Val
   1               5                   10                  15
   Cys Glu Lys Xaa Ala Leu Xaa Xaa Val Xaa Xaa Xaa Xaa
               20                  25
<210> 175
   <211> 44
   <212> PRT
   <213> séquence artificielle
<220>
   <221> MISC_FEATURE
   <222> (4)..(4)
   <223> R ou Q
<220>
   <221> MISC_FEATURE
   <222> (6) .. (6)
   <223> P ou A
<220>
   <221> MISC_FEATURE
   <222> (9) .. (9)
   <223> L ou F
<220>
   <221> MISC_FEATURE
   <222> (12)..(12)
   <223> F ou Y
<220>
   <221> MISC_FEATURE
   <222> (14)..(14)
   <223> D ou E.
<220>
   <221> MISC_FEATURE
   <222> (17)..(17)
   <223> V ou S
<220>
   <221> MISC_FEATURE
   <222> (23).. (23)
   <223> M ou R
<220>
   <221> MISC_FEATURE
   <222> (26) .. (26)
   <223> Y ou H
<220>
   <221> MISC_FEATURE
   <222> (27)..(27)
   <223> D ou N
<220>
   <221> MISC_FEATURE
   <222> (29)..(29)
   <223> V ou I
<220>
   <221> MISC_FEATURE
   <222> (30) .. (30)
   <223> S, T ou K
<220>
   <221> MISC_FEATURE
   <222> (31).. (31)
   <223> T, K, I ou N
<220>
   <221> MISC_FEATURE
   <222> (32) .. (32)
   <223> L ou T
<220>
   <221> MISC_FEATURE
   <222> (33).. (33)
   <223> P ou A
<220>
   <221> MISC_FEATURE
   <222> (34) .. (34)
   <223> Q, L, H, R, K ou P
<220>
   <221> MISC_FEATURE
   <222> (35)..(35)
   <223> A, T, V ou P
<220>
   <221> MISC_FEATURE
   <222> (39) .. (39)
   <223> P, S ou A
<220>
   <221> MISC_FEATURE
   <222> (40).. (40)
   <223> S ou A
<220>
   <221> MISC_FEATURE
   <222> (42)..(42)
   <223> G ou R
<220>
   <221> MISC_FEATURE
   <222> (43)..(43)
   <223> F ou C
<400> 175
   Lys Gly Gly Xaa Lys Xaa Ala Arg Xaa Ile Val Xaa Pro Xaa Leu Gly
   1               5                   10                  15
   Xaa Arg Val Cys Glu Lys Xaa Ala Leu Xaa Xaa Val Xaa Xaa Xaa Xaa
               20                  25                  30
   Xaa Xaa Xaa Val Met Gly Xaa Xaa Tyr Xaa Xaa Gln
           35                  40
<210> 176
   <211> 29
   <212> PRT
   <213> séquence artificielle
<400> 176
   Arg Lys Pro Ala Arg Leu Ile Val Phe Pro Asp Leu Gly Val Arg Val
   1               5                   10                  15
   Cys Glu Lys Met Ala Leu Tyr Asp Val Val Ser Thr Leu
               20                  25
<210> 177
   <211> 29
   <212> PRT
   <213> séquence artificielle
<400> 177
   Arg Lys Pro Ala Arg Leu Ile Val Phe Pro Asp Leu Gly Val Arg Val
   1 5 10 15
   Cys Glu Lys Met Ala Leu Tyr Asp Val Val Ser Lys Leu
   20 25
<210> 178
   <211> 29
   <212> PRT
   <213> séquence artificielle
<400> 178
   Arg Lys Pro Ala Arg Leu Ile Val Phe Pro Asp Leu Gly Val Arg Val
   1 5 10 15
   Cys Glu Lys Met Ala Leu Tyr Asp Val Val Thr Lys Leu
   20 25
<210> 179
   <211> 29
   <212> PRT
   <213> séquence artificielle
<400> 179
   Gln Lys Pro Ala Arg Leu Ile Val Phe Pro Asp Leu Gly Val Arg Val
   1 5 10 15
   Cys Glu Lys Met Ala Leu Tyr Asp Val Val Ser Thr Leu
   20 25
<210> 180
   <211> 29
   <212> PRT
   <213> séquence artificielle
<400> 180
   Arg Lys Ala Ala Arg Leu Ile Val Phe Pro Asp Leu Gly Val Arg Val
   1               5                   10                  15
   Cys Glu Lys Met Ala Leu Tyr Asp Val Val Ser Thr Leu
               20                  25

<210> 181
   <211> 29
   <212> PRT
   <213> séquence artificielle
<400> 181
   Arg Lys Pro Ala Arg Leu Ile Val Phe Pro Asp Leu Gly Val Arg Val
   1               5                   10                  15
   Cys Glu Lys Met Ala Leu Tyr Asp Val Val Ser Ile Leu
               20                  25
<210> 182
   <211> 29
   <212> PRT
   <213> séquence artificielle
<400> 182
   Arg Lys Pro Ala Arg Leu Ile Val Phe Pro Glu Leu Gly Val Arg Val
   1               5                   10                  15
   Cys Glu Lys Met Ala Leu Tyr Asp Val Val Ser Thr Leu
               20                  25
<210> 183
   <211> 29
   <212> PRT
   <213> séquence artificielle
<400> 183
   Arg Lys Pro Ala Arg Phe Ile Val Phe Pro Asp Leu Gly Val Arg Val
   1               5                   10                  15
   Cys Glu Lys Met Ala Leu Tyr Asp Val Val Ser Thr Leu
               20                  25
<210> 184
   <211> 29
   <212> PRT
   <213> séquence artificielle
<400> 184
   Arg Lys Ala Ala Arg Leu Ile Val Phe Pro Asp Leu Gly Val Arg Val
   1               5                   10                  15
   Cys Glu Lys Met Ala Leu Tyr Asn Val Val Ser Thr Leu
               20                  25
<210> 185
   <211> 29
   <212> PRT
   <213> séquence artificielle
<400> 185
   Arg Lys Pro Ala Arg Leu Ile Val Phe Pro Asp Leu Gly Val Arg Val
   1               5                   10                  15
   Cys Glu Lys Met Ala Leu Tyr Asp Val Val Ser Asn Leu
               20                  25
<210> 186
   <211> 29
   <212> PRT
   <213> séquence artificielle
<400> 186
   Arg Lys Pro Ala Arg Leu Ile Val Tyr Pro Asp Leu Gly Val Arg Val
   1               5                   10                  15
   Cys Glu Lys Met Ala Leu Tyr Asp Val Val Ser Thr Leu
               20                  25
<210> 187
   <211> 29
   <212> PRT
   <213> séquence artificielle
<400> 187
   Arg Lys Pro Ala Arg Leu Ile Val Tyr Pro Asp Leu Gly Ser Arg Val
   1               5                   10                  15
   Cys Glu Lys Arg Ala Leu His Asp Val Ile Lys Lys Thr
               20                  25
<210> 188
   <211> 44
   <212> PRT
   <213> séquence artificielle
<400> 188
   Lys Gly Gly Arg Lys Pro Ala Arg Leu Ile Val Phe Pro Asp Leu Gly
   1               5                   10                  15
   Val Arg Val Cys Glu Lys Met Ala Leu Tyr Asp Val Val Ser Thr Leu
               20                  25                  30
   Pro Gln Ala Val Met Gly Pro Ser Tyr Gly Phe Gln
   35                  40
<210> 189
   <211> 44
   <212> PRT
   <213> séquence artificielle
<400> 189
   Lys Gly Gly Arg Lys Pro Ala Arg Leu Ile Val Phe Pro Asp Leu Gly
   1               5                   10                  15
   Val Arg Val Cys Glu Lys Met Ala Leu Tyr Asp Val Val Ser Lys Leu
               20                  25                  30
   Pro Leu Ala Val Met Gly Ser Ser Tyr Gly Phe Gln
           35                  40
<210> 190
   <211> 44
   <212> PRT
   <213> séquence artificielle
<400> 190
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Lys Leu
   20 25 30
   Pro Pro Ala Val Met Gly Ser Ser Tyr Gly Phe Gln
   35 40
<210> 191
   <211> 44
   <212> PRT
   <213> séquence artificielle
<400> 191
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Thr Lys Leu
   20 25 30
   Pro Leu Ala Val Met Gly Ser Ser Tyr Gly Phe Gln
   35 40
<210> 192
   <211> 44
   <212> PRT
   <213> séquence artificielle
<400> 192
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Ser Ser Tyr Gly Phe Gln
   35 40
<210> 193
   <211> 44
   <212> PRT
   <213> séquence artificielle
<400> 193
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Ala Ser Tyr Gly Phe Gln
   35 40
<210> 194
   <211> 44
   <212> PRT
   <213> séquence artificielle
<400> 194
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Val Val Met Gly Ser Ser Tyr Gly Phe Gln
   35 40
<210> 195
   <211> 44
   <212> PRT
   <213> séquence artificielle
<400> 195
   Lys Gly Gly Gln Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Ser Ser Tyr Gly Phe Gln
   35 40
<210> 196
   <211> 44
   <212> PRT
   <213> séquence artificielle
<400> 196
   Lys Gly Gly Arg Lys Ala Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Ser Ser Tyr Gly Phe Gln
   35 40

<210> 197
   <211> 44
   <212> PRT
   <213> séquence artificielle
<400> 197
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Ile Leu
   20 25 30
   Pro Gln Ala Val Met Gly Ser Ser Tyr Gly Phe Gln
   35 40
<210> 198
   <211> 44
   <212> PRT
   <213> séquence artificielle
<400> 198
   Lys Gly Gly Arg Lys Pro Ala Arg Leu Ile Val Phe Pro Glu Leu Gly
   1               5                   10                  15
   Val Arg Val Cys Glu Lys Met Ala Leu Tyr Asp Val Val Ser Thr Leu
               20                  25                  30
   Pro Gln Ala Val Met Gly Ser Ser Tyr Gly Phe Gln
           35                  40
<210> 199
   <211> 44
   <212> PRT
   <213> séquence artificielle
<400> 199
   Lys Gly Gly Arg Lys Pro Ala Arg Phe Ile Val Phe Pro Asp Leu Gly
   1               5                   10                  15
   Val Arg Val Cys Glu Lys Met Ala Leu Tyr Asp Val Val Ser Thr Leu
               20                  25                  30
   Pro Lys Ala Val Met Gly Ser Ser Tyr Gly Phe Gln
           35                  40
<210> 200
   <211> 44
   <212> PRT
   <213> séquence artificielle
<400> 200
   Lys Gly Gly Arg Lys Pro Ala Arg Phe Ile Val Phe Pro Asp Leu Gly
   1               5                   10                  15
   Val Arg Val Cys Glu Lys Met Ala Leu Tyr Asp Val Val Ser Thr Leu
               20                  25                  30
   Pro Gln Ala Val Met Gly Ser Ser Tyr Gly Phe Gln
           35                  40
<210> 201
   <211> 44
   <212> PRT
   <213> séquence artificielle
<400> 201
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Arg Ala Val Met Gly Ser Ser Tyr Gly Cys Gln
   35 40
<210> 202
   <211> 44
   <212> PRT
   <213> séquence artificielle
<400> 202
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Pro Val Met Gly Ser Ser Tyr Gly Phe Gln
   35 40
<210> 203
   <211> 44
   <212> PRT
   <213> séquence artificielle
<400> 203
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro His Ala Val Met Gly Ser Ser Tyr Gly Phe Gln
   35 40
<210> 204
   <211> 44
   <212> PRT
   <213> séquence artificielle
<400> 204
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Ser Ala Tyr Gly Phe Gln
   35 40
<210> 205
   <211> 44
   <212> PRT
   <213> séquence artificielle
<400> 205
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Ser Ser Tyr Arg Phe Gln
   35 40
<210> 206
   <211> 44
   <212> PRT
   <213> séquence artificielle
<400> 206
   Lys Gly Gly Arg Lys Ala Ala Arg Leu Ile Val Phe Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Pro Ser Tyr Gly Phe Gln
   35 40
<210> 207
   <211> 44
   <212> PRT
   <213> séquence artificielle
<400> 207
   Lys Gly Gly Arg Lys Ala Ala Arg Leu Ile Val Phe Pro Asp Leu Gly
   1 5 10 15
   Val Arg Val Cys Glu Lys Met Ala Leu Tyr Asn Val Val Ser Thr Leu
   20 25 30
   Pro Gln Ala Val Met Gly Ser Ser Tyr Gly Phe Gln
   35 40
<210> 208
   <211> 44
   <212> PRT
   <213> séquence artificielle
<400> 208
   Lys Gly Gly Arg Lys Pro Ala Arg Leu Ile Val Phe Pro Asp Leu Gly
   1 5 10 15
   Val Arg Val Cys Glu Lys Met Ala Leu Tyr Asp Val Val Ser Asn Leu
   20 25 30
   Pro Gln Ala Val Met Gly Ser Ser Tyr Gly Phe Gln
   35 40
<210> 209
   <211> 44
   <212>. PRT
   <213> séquence artificielle
<400> 209
   Lys Gly Gly Arg Lys Pro Ala Arg Leu Ile Val Phe Pro Glu Leu Gly
   1               5                   10                  15
   Val Arg Val Cys Glu Lys Met Ala Leu Tyr Asp Val Val Ser Thr Leu
               20                  25                  30
   Pro Gln Ala Val Met Gly Pro Ser Tyr Gly Phe Gln
           35                  40
<210> 210
   <211> 44
   <212> PRT
   <213> séquence artificielle
<400> 210
   Lys Gly Gly Arg Lys Pro Ala Arg Leu Ile Val Phe Pro Asp Leu Gly
   1               5                   10                  15
   Val Arg Val Cys Glu Lys Met Ala Leu Tyr Asp Val Val Ser Thr Leu
               20                  25                  30
   Pro His Thr Val Met Gly Ser Ser Tyr Gly Phe Gln
           35                  40
<210> 211
   <211> 44
   <212> PRT
   <213> séquence artificielle
<400> 211
   Lys Gly Gly Arg Lys Pro Ala Arg Leu Ile Val Tyr Pro Asp Leu Gly
   1 5 10 15
   Val Arg Val Cys Glu Lys Met Ala Leu Tyr Asp Val Val Ser Thr Leu
   20 25 30
   Pro Gln Ala Val Met Gly Ser Ser Tyr Gly Phe Gln
   35 40

<210> 212
   <211> 44
   <212> PRT
   <213> séquence artificielle
<400> 212
   Lys Gly Gly Arg Lys Pro Ala Arg Leu Ile Val Tyr Pro Asp Leu Gly
   1 5 10 15
   Ser Arg Val Cys Glu Lys Arg Ala Leu His Asp Val Ile Lys Lys Thr
   20 25 30
   Ala Leu Ala Val Met Gly Ala Ala Tyr Gly Phe Gln
   35 40
<210> 213
   <211> 10
   <212> PRT
   <213> séquence artificielle
<220>
   <221> MISC_FEATURE
   <222> (2)..(2)
   <223> L ou V
<220>
   <221> MISC_FEATURE
   <222> (3)..(3)
   <223> L ou F
<220>
   <221> MISC_FEATURE
   <222> (4) ..(4)
   <223> G ou S
<220>
   <221> MISC_FEATURE
   <222> (5) .. (5)
   <223> C ou T
<220>
   <221> MISC_FEATURE
   <222> (6) .. (6)
   <223> I ou V
<220>
   <221> MISC_FEATURE
   <222> (7)..(7)
   <223> I ou V
<400> 213
   Gly Xaa Xaa Xaa Xaa Xaa Xaa Thr Ser Leu
   1 5 10
<210> 214
   <211> 10
   <212> PRT
   <213> séquence artificielle
<400> 214
   Gly Leu Leu Gly Cys Ile Ile Thr Ser Leu
   1 5 10
<210> 215
   <211> 10
   <212> PRT
   <213> séquence artificielle
<400> 215
   Gly Leu Phe Gly Cys Ile Ile Thr Ser Leu
   1 5 10
<210> 216
   <211> 10
   <212> PRT
   <213> séquence artificielle
<400> 216
   Gly Val Leu Gly Cys Val Ile Thr Ser Leu
   1 5 10
<210> 217
   <211> 10
   <212> PRT
   <213> séquence artificielle
<400> 217
   Gly Val Leu Gly Cys Ile Ile Thr Ser Leu
   1 5 10
<210> 218
   <211> 10
   <212> PRT
   <213> séquence artificielle
<400> 218
   Gly Leu Leu Gly Cys Ile Val Thr Ser Leu
   1 5 10
<210> 219
   <211> 10
   <212> PRT
   <213> séquence artificielle
<400> 219
   Gly Leu Phe Gly Cys Ile Val Thr Ser Leu
   1 5 10
<210> 220
   <211> 10
   <212> PRT
   <213> séquence artificielle
<400> 220
   Gly Leu Phe Ser Thr Ile Ile Thr Ser Leu
   1 5 10
<210> 221
   <211> 9
   <212> PRT
   <213> séquence artificielle
<220>
   <221> MISC_FEATURE
   <222> (4) .. (4)
   <223> T, S ou A
<220>
   <221> MISC_FEATURE
   <222> (5) .. (5)
   <223> T ou I
<220>
   <221> MISC_FEATURE
   <222> (6)..(6)
   <223> Q, S ou G
<220>
   <221> MISC_FEATURE
   <222> (7) .. (7)
   <223> N, Q, H, S, Y ou T
<400> 221
   Ser Pro Leu Xaa Xaa Xaa Xaa Thr Leu
   1 5
<210> 222
   <211> 9
   <212> PRT
   <213> séquence artificielle
<400> 222
   Ser Pro Leu Thr Thr Gln Asn Thr Leu
   1 5
<210> 223
   <211> 9
   <212> PRT
   <213> séquence artificielle
<400> 223
   Ser Pro Leu Thr Thr Ser Gln Thr Leu
   1 5
<210> 224
   <211> 9
   <212> PRT
   <213> séquence artificielle
<400> 224
   Ser Pro Leu Thr Thr Gly Gln Thr Leu
   1 5
<210> 225
   <211> 9
   <212> PRT
   <213> séquence artificielle
<400> 225
   Ser Pro Leu Thr Thr Gln His Thr Leu
   1 5
<210> 226
   <211> 9
   <212> PRT
   <213> séquence artificielle
<400> 226
   Ser Pro Leu Thr Thr Gln Ser Thr Leu
   1 5
<210> 227
   <211> 9
   <212> PRT
   <213> séquence artificielle
<400> 227
   Ser Pro Leu Thr Thr Gln Tyr Thr Leu
   1 5
<210> 228
   <211> 9
   <212> PRT
   <213> séquence artificielle
<400> 228
   Ser Pro Leu Thr Thr Gln Thr Thr Leu
   1 5
<210> 229
   <211> 9
   <212> PRT
   <213> séquence artificielle
<400> 229
   Ser Pro Leu Thr Ile Gln His Thr Leu
   1 5

<210> 230
   <211> 9
   <212> PRT
   <213> séquence artificielle
<400> 230
   Ser Pro Leu Ser Thr Gln Asn Thr Leu
   1 5
<210> 231
   <211> 9
   <212> PRT
   <213> séquence artificielle
<400> 231
   Ser Pro Leu Ala Thr Gln Tyr Thr Leu
   1 5
<210> 232
   <211> 9
   <212> PRT
   <213> séquence artificielle
<400> 232
   Ser Pro Leu Thr Thr Gln Gln Thr Leu
   1 5